# EUROPEAN PATENT SPECIFICATION

(11) **EP 0 701 444 B1**
(45) Date of publication and mention of the grant of the patent: **07.04.2010**
(21) Application number: 94918659.7
(22) Date of filing: 23.05.1994
(51) Int. Cl.: A61K 31/56, A61K 31/57, A61K 31/575, A61K 31/58, A61P 25/20

(54) **METHODS AND COMPOSITIONS FOR INDUCING SLEEP**
VERFAHREN UND ZUSAMMENSETZUNGEN ZUM HERVORRUFEN VON SCHLAF
PROCEDES ET COMPOSITIONS INDUISANT LE SOMMEIL

(30) Priority: 24.05.1993 US 68378; 14.02.1994 US 196919; 14.02.1994 US 196972; 19.05.1994 US 246275
(43) Date of publication of application: 20.03.1996
(73) Proprietor: Purdue Pharma Ltd., Irvine, CA 92718 (US)
(72) Inventor: GEE, Kelvin W., Irvine, CA 92715 (US); LAN, Nancy Tsai-Yun, South Pasadena, CA 91030 (US); UPASANI, Ravindra, B., 63 Montecilo, CA 92610 (US); HOGENKAMP, Derk, J., Carlsbad, CA 92008 (US); PURDY, Robert, San Diego, CA 92122 (US); BOLGER, Michael, B., Los Alamitos, CA 90720 (US); TAHIR, Hasan, New Delhi 110 044 (IN)
(74) Representative: Chapman, Paul William
(86) International application number: PCT/US1994/005820
(87) International publication number: WO 1994/027608

(56) References cited:
- WO-A-89/02272
- WO-A-91/16897
- WO-A-93/03732
- WO-A-93/05786
- DE-A1- 2 162 555
- BIOCHEM.PHARMACOL., vol.21, no.5, 1972 pages 657 - 671 L.F.SOYKA ET AL. 'DISTRIBUTION AND HEPATIC METABOLISM OF PREGNANOLONE IN THE RAT'
- BRAIN RES. BULL., vol.29, no.1, 1992 pages 57 - 68 B.J.BOWERS ET AL. 'Biochemical and Behavioral Effects of Steroids on GABA-A Receptor Function in Long- and Short-Sleep Mice'
- PSYCHOPHARMACOLOGY, vol.93, no.2, 1987 pages 226 - 229 W.B.MENDELSON ET AL. 'Sleep induction by an adrenal steroid in the rat'
- PHARMACOL.BIOCHEM.BEHAV., vol.42, no.4, August 1992 pages 605 - 611 C.L.MELCHIOR ET AL. 'Interaction of Pregnanolone and Pregnenolone Sulfate With Ethanol and Pentobarbital'
- PROC.SOC.EXP.BIOL.MED., vol.125, 1967 pages 1058 - 1062 L. GYERMEK 'Pregnanolone: A Highly Potent, Naturally Occurring Hypnotic-Anesthetic Agent.' cited in the application
- AM.J.OBSTET.GYNECOL., vol.159, November 1988 pages 1203 - 1209 E.S.ARAFAT ET AL. 'Sedative and hypnotic effects of oral administration of micronized progesterone may be mediated through its metabolites' cited in the application
- ANESTHESIOLOGY, vol.28, 1967 pages 173 - 183 G.HEUSER 'Induction of Anesthesia, Seizures and Sleep by Steroid Hormones' cited in the application
- SCIENCE, vol.232, no.4753, 23 May 1986 pages 1004 - 1007 M.D.MAJEWSKA ET AL. 'Steroid Hormone Metabolites Are Barbiturate-Like Modulators of the GABA Receptor' cited in the application
- HOGENKAMP ET AL: "Synthesis and in vitro Activity of 3.beta.-Substituted-3.alpha.- hydroxypregnan-20-ones: Allosteric Modulators of the GABAA Receptor" JOURNAL OF MEDICINAL CHEMISTRY, AMERICAN CHEMICAL SOCIETY, WASHINGTON, US, vol. 40, no. 1, 1 January 1997 (1997-01-01), pages 61-72, XP002341275 ISSN: 0022-2623
- ZORUMSKI C F ET AL: "Potential clinical uses of neuroactive steroids." September 2000 (2000-09), IDRUGS : THE INVESTIGATIONAL DRUGS JOURNAL SEP 2000, VOL. 3, NR. 9, PAGE(S) 1053 - 1063 ISSN: 1369-7056

## Description

### Background

The present invention is directed to the use of compounds as defined in the claims for the manufacture of a medicament for the treatment of insomnia wherein the compounds interact with the gamma-aminobutyric acid receptor ionophore complex ("GABA_{A} receptor complex").

The relationship between steroids, the GABA_{A} receptor complex, and brain excitability has previously been described in U.S. Patents Nos. 5,120,723 and 5,232,917.

The inventors have found that certain steroids, called neuroactive steroids, acting at the steroid site on this receptor complex can be used to induce sleep and in particular, treat insomnia. For example, the inventors have found that 3α-hydroxy-5β-pregnan-20-one ("pregnanolone"), can induce sleep when administered orally. Arafat and co-workers had established a correlation between the progesterone metabolites pregnanolone and 3α-hydroxy-5α-pregnan-20-one and sleep following administration of progesterone in humans. Arafat, E.S., Hargrove, J.T., Maxson, W.G., Desiderio, D.M., Wentz, A.C., and Anderson, R.N. "Sedative and Hypnotic Effects of Oral Administration of Micronized Progesterone May Be Mediated Through Its Metabolites" Am. J. Obstet. Gynecol. 159:1203-1209 (1988). The oral activity of pregnanolone is surprising since Laszlo Gyermek found that it was almost inactive orally in "Pregnanolone: a Highly Potent, Naturally Occurring Hypnotic-Anesthetic Agent" Proc. soc. Exp. Biol. Med. 1967, 125:1058-1062.

No incentive existed, however, to make an orally active formulation of pregnanolone because earlier investigations indicated that the neuroactive steroids acted like the barbiturates, which exhibit the undesirable side effect of anesthesia when used to induce sleep, a low therapeutic index, abuse potential, and adverse side effects such as rebound insomnia. Majewska, M.D.; Harrison, N.L.; Schwartz, R.D.; Barker, J.L.; and Paul, S.M. "Steroid Hormone Metabolites Are Barbiturate-Like Modulators of the GABA Receptor" Science 232:1004-1007, 1986.

It has been known since 1967 that progesterone, an anesthetic steroid, when administered at subanesthetic doses could induce sleep in the cat. Heuser, G. "Induction of Anesthesia, Seizures and Sleep by Steroid Hormones" Anesthesiology 28:173-183 (1967). Heuser concluded that progesterone produced a "natural" sleep in the cat because REM sleep was not suppressed during administration of the drug, nor was REM sleep increased upon withdrawal of the drug. Significantly, Heuser found that not all anesthetic steroids were capable of producing this "natural" sleep.

The German patent application DE 2162555 discloses 3α-oxygen-containing steroids of the pregnane and androstane series, including the compound 3α-hydroxy-3β-methyl-5α-pregnan-20-one, as anaesthetics and for the induction of sleep.

The National Commission on Sleep Disorders Research estimates that 40 million Americans suffer from chronic sleep disorders such as insomnia, narcolepsy, and sleep apnea. It is estimated that 20-30 million Americans experience intermittent sleep-related problems. "Wake Up America: A National Sleep Alert" Vol. 1, p. vi; Draft Report of the National Commission on Sleep Disorders Research 1992. The large incidence of insomnia and other sleep problems is not surprising when one considers that normal sleep-wake patterns can be interrupted by emotional distress, drugs, jet lag, shift work, illness, and strong sensory stimuli such as pain or hunger.

The social costs of this high incidence of sleep disorders and sleepiness are significant. These costs include reduced productivity, lowered cognitive performance, increased likelihood of accidents, higher morbidity and mortality risk, and decreased quality of life. Id. The Commission estimated that in 1990 alone, sleep disorders and sleepiness cost the United States at least $15.9 billion in direct costs alone, which does not include indirect costs from sleep related tragedies such as the grounding of the Exxon Valdez. Id. Society desperately needs safe and effective treatments for sleep disorders.

Currently, insomnia is treated with a variety of drugs, all of which have certain drawbacks. A common treatment is the use of the drug Triazolam, known by the tradename Halcion. Its drawbacks include rebound insomnia and limited efficacy in increasing sleep. Rebound insomnia is a return of abnormal sleep patterns as the drug wears off or as a result of either tolerance to the drug or withdrawal symptoms when the treatment is stopped. Zolpidem, a new insomnia drug, also tends to cause rebound insomnia.

The compounds of the present invention have superior characteristics to currently available drugs. The present invention provides medicaments for an insomnia treatment with rapid onset of sleep, high potency and efficacy, and minimal rebound insomnia.

To aid in fully understanding the present invention, a brief outline of sleep and how it is studied follows. Sleep is often defined using two sets of criteria: 1) electrophysiological; and 2) behavioral. The electrophysiological criteria can include electroencephalograms (EEGs), electro-oculograms (EOGs), and electromyograms (EMGs). The most common criterion used for studying sleep is the EEG which is a recording of the electrical activity of the brain. By studying cortical EEGs of sleeping individuals, scientists have found that there are two major stages of sleep manifested by the production of different electrical wave patterns. These two stages are called non-REM (NREM) and REM. NREM sleep is conventionally divided into four stages in which the EEG pattern is commonly described as synchronous, with characteristic waveforms such as sleep spindles, K complexes and high-voltage slow waves. Kryger, M.H.; Roth, T.; and Dement, W.C. Principles and Practice of Sleep Medicine, p.3, 1989 (hereinafter "PPSM"). REM sleep generally shows greater EEG activation, muscle atonia, and episodic periods of rapid eye movements. Id.

When an individual closes his or her eyes in preparation for sleep, the cortical electrical rhythms synchronize to a rhythm of 8-13 Hz. This is known as an α-rhythm. An EEG taken in the first stage of sleep shows a slower rhythm (4-6 Hz) and a lower amplitude. Bowman, W.C., and Rand, M.J. Textbook of Pharmacology, p 6.24, 2nd ed. 1980 (hereinafter Bowman and Rand). In the second stage of sleep, more irregular and slower EEG waves of 1-5 Hz with a larger amplitude appear. These irregular, slow waves are interspersed with bursts of faster waves called sleep-spindles. In this second stage of sleep there are also K-complexes which consist of a small sharp wave, followed by one or two larger waves and then by faster waves of about 12 Hz. Id. Stage 2 accounts for 45-55 % of sleep time. PPSM at p. 9.

Stage 3 sleep is characterized by high voltage, slow EEG wave activity accounting for 20-50 % of the EEG activity in this stage. PPSM p. 7. In stage 4, high voltage, slow wave activity accounts for >50% of the EEG, and K-complexes cannot be evoked by stimuli such as calling the subject's name. PPSM at p. 7; Bowman and Rand p. 6.24.

After about an hour of sleep the EEG pattern speeds up to a 4-10 Hz rhythm of low amplitude waves that are desynchronized. This is REM sleep and is often referred to as paradoxical sleep because the EEG resembles that of wakefulness, yet it is very deep sleep. Bowman and Rand p. 6.24. The biological significance of REM sleep is not yet known. However, alcohol's strong inhibition of REM sleep has been associated with sleep disruption. Triazolam and other insomnia drugs reduce REM sleep, whereas compounds of the present invention have the further advantage of only minimally disrupting REM sleep.

### Summary Of The Invention

The present invention is directed to methods for inducing sleep in human beings. More particularly, the invention relates to the use of certain 3α-hydroxylated-5-reduced steroid derivatives as defined in the claims which act at a recently identified site on the GABA_{A} receptor complex to treat insomnia.

Experiments by the inventors have established that the compounds used when practicing the invention give a rapid onset of sleep and have high potency and efficacy. They also cause less rebound insomnia and fewer effects on body temperature than known hypnotic drugs used to treat insomnia.

### Brief Description Of The Drawings

These and other features, aspects, and advantages of the present invention will become better understood from the following description, the appended claims, and the accompanying drawings, where:
FIGS. 1A and 1B are plots of the percentage of NREM sleep per hour in rats vs. clock time for 30mg/kg and 3 & 10mg/kg of pregnanolone, respectively, administered at circadian time 18 hours (CT-18);
FIG. 1C is a plot of the percentage of NREM sleep per hour in rats vs. clock time for 10mg/kg of pregnanolone, administered at CT-5.
FIGS. 2A and 2B are plots of the minutes spent sleeping vs. clock time when 30mg/kg and 3 & 10mg/kg of pregnanolone, respectively, administered at CT-18;
FIG. 2C is a plot of the minutes spent sleeping vs. clock time for 10mg/kg of pregnanolone administered at CT-5.
FIGS. 3A and 3B are plots of the mean deviation in body temperature vs. clock time for 30mg/kg and 3 & 10mg/kg of pregnanolone, respectively, administered at CT-18;
FIG. 3C is a plot of the mean deviation in body temperature vs. clock time for 10mg/kg of pregnanolone administered at CT-5.
FIGS. 4A and 4B are plots of counts of movement per hour (locomotor activity) vs. clock time for 30mg/kg and 3 & 10mg/kg of pregnanolone, respectively, administered at CT-18;
FIG. 4C is a plot of counts of movement per hour (locomotor activity) vs. clock time for 10mg/kg of pregnanolone, administered at CT-5.
FIGS. 5A and 5B are plots of the percentage of REM sleep per hour vs. clock time for 30mg/kg and 3 & 10mg/kg of pregnanolone, respectively, administered at CT-18;
FIG. 5C is a plot of the percentage of REM sleep per hour vs. clock time for 10mg/kg of pregnanolone, administered at CT-5.
FIGS. 6A, 6B, and 6C are plots if the percentage of NREM sleep per hour vs. clock time for 0.4 mg/kg of Triazolam administered at CT-5, for 0.4 mg/kg administered at CT-18, and for 0.4 mg/kg and 1.6 mg/kg of Triazolam administered at CT-18, respectively.
FIG. 6D is a plot of the minutes spent sleeping vs. clock time for 0.4 mg/kg and 1.6 mg/kg Triazolam administered at CT-18.
FIG. 6E is a plot of the mean deviation in body temperature vs. clock time for 0.4 mg/kg and 1.6 mg/kg Triazolam administered at CT-18.
FIG. 6F is a plot of the counts of body movement per hour (locomotor activity) vs. clock time for 0.4 mg/kg and 1.6 mg/kg Triazolam administered at CT-18.
FIG. 6G is a plot of the percentage of REM sleep per hour vs. clock time for 0.4 mg/kg and 1.6 mg/kg of Triazolam administered at CT-18.
FIGS. 7A and 7B show the EEG pattern during NREM sleep for 10 mg/kg of pregnanolone. FIG. 7B is an expanded view of part of FIG. 7A.
FIGS. 7C and 7D show the EEG pattern during NREM sleep for 30 mg/kg Zolpidem. FIG. 7D is an expanded view of part of FIG. 7C.
FIGS. 8A, 8B, and 8C are plots of the per- centage of NREM sleep per hour vs. clock time for 10 mg/kg of Zolpidem administered at CT-5 and CT-18, respectively, and for 30 mg/kg of Zolpidem administered at CT-18.
FIG. 8C is a plot of the minutes spent sleeping vs. clock time for 10 mg/kg and 30 mg/kg Zolpidem administered at CT-18.
FIG. 8D is a plot of the mean deviation in body temperature vs. clock time for 10 mg/kg and 30 mg/kg of Zolpidem administered at CT-18.
FIG. 8E is a plot of the counts of body movement per hour (locomotor activity) vs. clock time for 10 mg/kg and 30 mg/kg Zolpidem administered at CT-18.
FIG. 8F is a plot of the percent REM sleep per hour vs. clock time for 10 mg/kg and 30 mg/kg Zolpidem administered at CT-18.

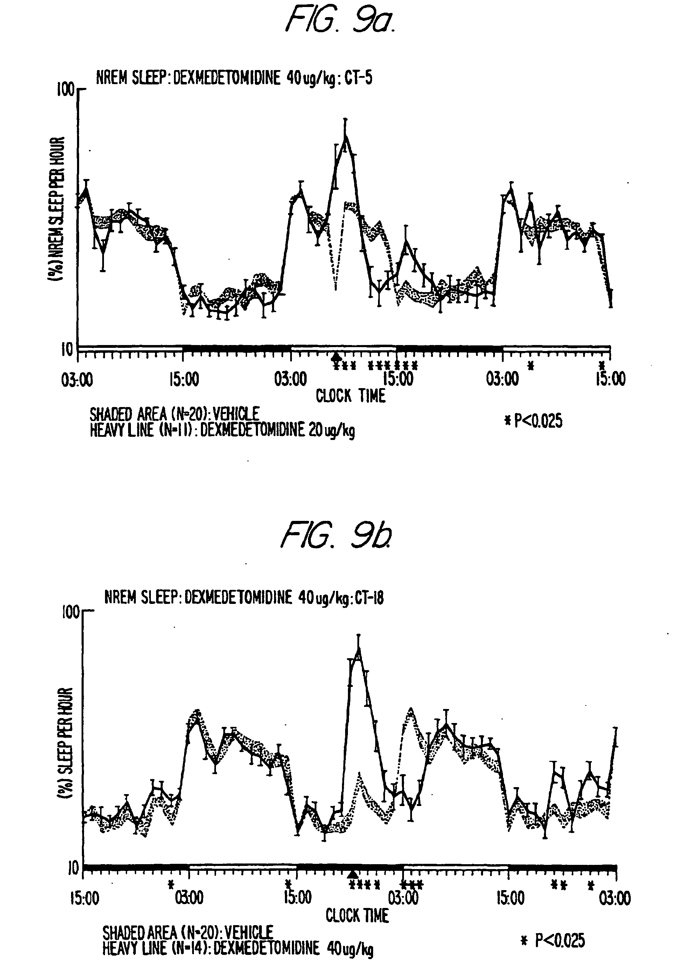

FIGS. 9A and 9B are plots of the percentage of NREM sleep per hour vs. clock time for 40 ug/kg of dexmedetomidine administered at CT-5 and CT-18, respectively.
FIGS. 10A, 10B, and 10C are plots of the percentage of total power (activity during NREM sleep) vs. minutes after treatment for pregnanolone, Zolpidem, and Triazolam, respectively, administered at CT-5.
FIGS. 11A, 11B, and 11C are plots of the percent of total power (activity during NREM sleep) vs. frequency for pregnanolone, Zolpidem, and Triazolam, respectively, administered at CT-5.
FIG. 12 is a bar graph showing the change from baseline in locomotor activity for the first hour after treatment with various drugs. Locomotor activity is measured in counts per hour.
FIG. 13 is a bar graph depicting the change in REM sleep 2-3 hours after treatment with various drugs.
FIGS. 14A and 14B show plots of counts of movement per 5 minutes vs. minutes after treatment for pregnanolone and Zolpidem administered at CT-18, respectively.
FIGS. 15A-15H are plots of human plasma concentration of pregnanolone over time for Formulation 1, which is 5% pregnanolone, 94% polyvinylpyrrolidone, and 1% sodium laurel sulfate. Arrows indicate time points when sleep was observed.
FIGS. 16A-16H are plots of human plasma concentrations of pregnanolone over time for Formulation 2, which is an inclusion complex of 12.3% pregnanolone and 87.7% β-cyclodextrin. Arrows indicate time points when sleep was observed.
FIGS. 17A-17H are plots of human plasma concentration of pregnanolone over time for Formulation 3, which is 99% pregnanolone nanosized by supercritical fluid nucleation with 1% sodium laurel sulfate. Arrows indicate time points when sleep was observed.
FIGS. 18A-18H are plots of human plasma concentration of pregnanolone over time for Formulation 4, which is 99% micronized pregnanolone from AKZO with 1% sodium laurel sulfate. Arrows indicate time points when sleep was observed.
FIG. 19 is a plot of serum concentration in human subjects of pregnanolone versus time for all four formulations.
FIG. 20 is a table showing human mean plasma concentrations of pregnanolone from two formulations at half-hour intervals.
FIG. 21 is a table showing the mean time awake in male human subjects during the 5 minute polygraphic recording sessions at half-hour intervals from two formulations of pregnanolone.
FIG. 22A is a graph plotting the number of sleep attempts by male human subjects verses plasma concentration of pregnanolone.
FIG. 22B is a table showing that the number of sleep attempts by male human subjects during a study day is related to the plasma concentration of pregnanolone.

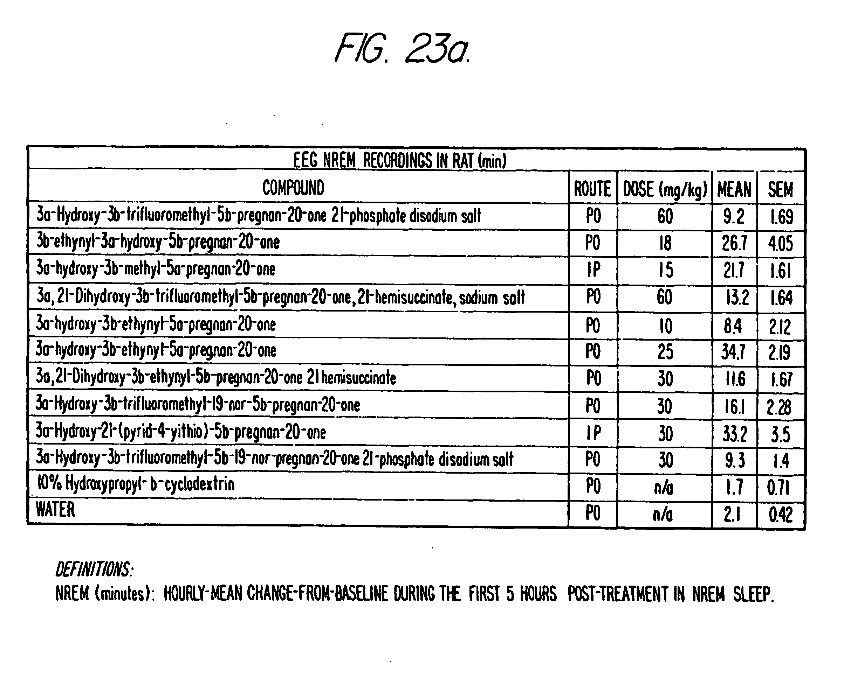

FIGS. 23A-C are tables showing the mean change in NREM sleep, the mean change in REM rebound, and the mean change in REM sleep, respectively in rats receiving 3α-hydroxy-3β-trifluoromethyl-5β-pregnan-20-one 21-phosphate disodium salt, 3β-ethynyl-3α-hydroxy-5β-pregnan-20-one, 3α-hydroxy-3β-methyl-5α-pregnan-20-one, 3α,21-dihydroxy-3β-trifluoromethyl-5β-pregnan-20-one 21-hemisuccinate sodium salt, 3α-hydroxy-3β-ethynyl-5α-pregnan-20-one, 3α,21-dihyrdoxy-3β-ethynyl-5β-pregnan-20-one 21-hemisuccinate, 3α-hydroxy-3β-trifluoromethyl-19-nor-5β-pregnan-20-one, 3α-hydroxy-21-(pyrid-4-ylthio)-5β-pregnan-20-one, 3α-hydroxy-3β-trifluoromethyl-5β-19-nor-pregnan-20-one 21-phosphate disodium salt, or controls.
FIGS. 24A and 24B are plots of the percentage of NREM sleep and REM sleep, respectively, per hour in rats vs. clock time for 18 mg/kg of 3β-ethynyl-3α-hydroxy-5β-pregnan-20-one (CCD-3093), administered at circadian time 18 hours (CT-18).
FIG. 25 is a plot of the minutes spent sleeping vs. clock time for 18 mg/kg of 3β-ethynyl-3α-hydroxy-5β-pregnan-20-one administered at CT-18.
FIGS. 26A and 26B are plots of the mean deviation in body temperature vs. clock time and of counts of movement per hour (locomotor activity) vs. clock time, respectively, for 18 mg/kg of 3β-ethynyl 1-3α-hydroxy-5β-pregnan-20-one administered at CT-18.

Figures referring to unclaimed compounds are for reference only.

### Description

The compounds used in this invention are 3α-hydroxylated-5-reduced steroids as defined in the claims which interact with the GABA_{A} receptor complex.

A preferred group of Compounds includes those in which R is not hydrogen, the compound has an IC₅₀ value of ≤ 300nM as an allosteric modulator of [³⁵S] TBPS binding, and the compound is orally active.

Another preferred group of compounds which can be used according to this invention are 3α-hydroxy-3β-R-5α or 5β-19-R¹-21-R²-pregnan-20-ones having the structural formula II: 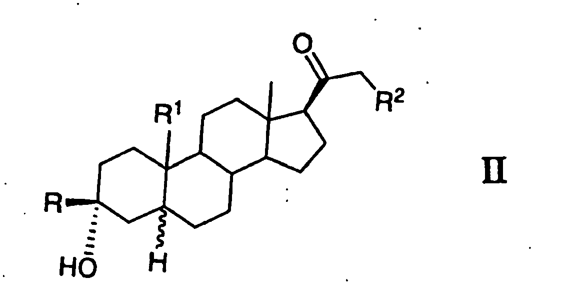 wherein:
R is a C₁₋₈ trihaloalkyl group, e.g., trifluoromethyl;
R¹ is hydrogen or a C₁₋₈ alkyl group;
R² is hydrogen, hydroxy, a pyrid-4-ylthio group, a hemisuccinoyloxy group, or a phosphoryloxy group and the sodium salts thereof.

A preferred group of compounds of Formula II is where R² is a hemisuccinoyloxy group or a phosphoryloxy group, or sodium salts thereof.

The preferred neuroactive steroids of the present invention include 3α-hydroxy-3β-trifluoromethyl-5β-pregnan-20-one 21-phosphate disodium salt; 3α,21-dihydroxy-3β-trifluoromethyl-5β-pregnan-20-one 21-hemisuccinate sodium salt; 3α-hydroxy-3β-trifluoromethyl-19-nor-5β-pregnan-20-one; and 3α-hydroxy-3β-trifluoromethyl-5β-19-norpregnan-20-one 21-phosphate disodium salt.

### Definitions

In accordance with the,present invention and as used herein, the following terms are defined with the following meanings, unless explicitly stated otherwise.

The term "alkyl" refers to saturated aliphatic groups including straight chain, branched chain.

The term "trihaloalkyl" refers to alkyl groups substituted with three halogen atoms, F, Br, Cl or I. Preferred trihaloalkyl groups include trifluoromethyl and 2',2',2'-trifluoroethyl.

The term "lower" referred to herein in connection with organic radicals or compounds defines such as up to and including 8, preferably up to and including 6, and advantageously one, two, three, or four carbon atoms.

The term "halo" refers to the halogen atoms F, Br, Cl, and I.

The following synthetic methods and examples are directed to the preparation of compound used in the present invention.

### Synthetic Methods

The compounds according to the invention may be prepared by any convenient method, e.g. using conventional techniques such as are described in "Steroid Reactions" Djerassi, published in 1963 by Holden-Day, Inc., San Francisco or "Organic Reactions in Steroid Chemistry", Fried and Edwards, published in 1972 by Van Nostrand-Reinhold Co., New York.

21-hemisuccinates were prepared from pregnan-20-one derivatives which were first brominated with molecular bromine to obtain the corresponding 21-bromo pregnanes. The bromo compounds were then reacted with succinic acid, in the presence of an amine to yield 21-hydroxy esters. The resulting esters from the dioic acids were then converted to their sodium salts by conventional means.

The esters may be formed using reactions well known in the art between the hydroxyl group on the compounds discussed above with an organic and inorganic acid, acid halide, acid anhydride, or ester.

### 3β-Substituents

### Trifluoromethyl

The-trifluoromethyl group may be prepared by reaction of a 3-ketosteroid with trimethyltrifluoromethylsilane catalyzed by fluoride ion.

### 21-Oxygenated compounds

### Lead tetraacetate oxidation of 21-methyl

Various compounds of this type may be prepared by a reaction sequence in which a pregnan-20-one is oxidized with lead tetraacetate to give a 21-acetoxy derivative, hydrolysis of the acetate to give a 21-alcohol. The 21-alcohol may then be acylated with an appropriate carboxylic acid derivative, such as an acid anhydride or acid chloride or other reagent capable of replacing the hydrogen of the hydroxyl group, such as methanesulfonyl chloride.

### Bromination of C-21

Alternatively, a pregnan-20-one may be treated with a brominating agent such as bromine in methanol to give a 21-bromopregnan-20-one. The bromide may then be displaced with an oxygen nucleophile, such as succinate anion, to give other 21-substituted pregnan-20-ones.

### EXAMPLE 1

### a. 3α-Hydroxy-5β-pregnan-20-one cyclic 20-(1,2-ethanediyl ketal)

A mixture of 3α-hydroxy-5β-pregnan-20-one (10.8 g, 34 mmol), ethylene glycol (45 mL), and triethyl orthoformate (30 mL) was stirred at r.t. for 5 min. p-Toluene sulfonic acid (pTSA) (200 mg) was then added and the stirring was continued at r.t. for 1.5 hr. The resulting thick paste was poured into a sat. NaHCO₃ solution (250 mL). The precipitated solid was collected by filtration, washed thoroughly with cold water and dried. This semi-dried product was dissolved in CH₂Cl₂ (350 mL) and dried over anhyd. K₂CO₃. The solution of the ketal was then filtered and used as such for the next step.

### b. 5β-Pregnan-3,20-dione cyclic 20-(1,2-ethanediyl ketal)

The above solution of 3α-hydroxy-5β-pregnan-20-one cyclic 20-(1,2-ethanediyl acetal) in CH₂Cl₂ was stirred with N-methylmorpholine-N-oxide (8.8 g, 75 mmol), and powdered 4Å molecular sieves (58 g) under N₂ for 15 min. Tetrapropylammonium perruthenate (400 mg) was then added and the stirring was continued at r.t. for 2 hr. The resulting dark green mixture was passed through a short column of Florisil and eluted with CH₂Cl₂. The fractions containing the product (by TLC) were combined and evaporated. The crude product was then crystallized from a mixture of EtOAc:Hex (1:1) to yield the title compound (10.3 g) as long rods.

An analogous method was used to prepare 5α-pregnan-3,20-dione cyclic 20-(1,2,ethanediyl ketal).

### c. 3α-hydroxy-3β-phenylethynyl-5β-pregnan-20-one

A solution of 5β-Pregnan-3,20-dione cyclic 20-(1,2-ethanediyl acetal) (180 mg, 0.5 mmol) in dry THF (15 mL) was treated with lithium phenylacetylide (1M in THF, 1.5 mmol, 1.5 mL) at -70°. After stirring the mixture at this temp. for 1 h and then at r.t. for 2 h, it was quenched with 2N HCl (1 mL). The solvent was removed and the residue was dissolved in acetone (20 mL). After adding 1N HCl (5 mL) the solution was stirred at r.t. for 15 h. The solvents were removed and the residue was extracted with CH₂Cl₂. The organic layer was washed with water, dil. NaHCO₃ soln., water, and brine. After drying over anhyd. MgSO₄ the solution was filtered and evaporated to yield the crude product (300 mg). This crude product was then dissolved in a small amount of CH₂Cl₂ and poured on a column of silica gel. On elution with toluene:acetone mixture (95:5) gave 3α-phenylethynyl-3β-hydroxy-5β-pregnan-20-one (70 mg) as a first fraction. Further elution with the same solvent mixture yielded 3β-phenylethynyl-3α-hydroxy-5β-pregnan-20-one (160 mg), m.p. 188-190°C.

### EXAMPLE 2

### a. 3α-hydroxy-3β-(2'-phenylethyl)-5β-pregnan-20-one

A solution of 3α-hydroxy-3β-phenylethynyl-5β-pregnan-20-one (44 mg) was dissolved in EtOAc (12 mL), Pd/C (5%, 12 mg) was added and the mixture was hydrogenated at 400 Kpa pressure overnight at r.t. Filtration of the catalyst followed by evaporation of the solvent yielded the crude product, which was purified by chromatography over silica gel to isolate the pure title compound (33 mg); mp 153-154°C; TLC Rf (hexane:acetone 7:3) = 0.4.

### EXAMPLE 3

### a. 3β-(3',4'-Dimethoxyphenylethynyl)-3α-hydroxy-5β-pregnan-20-one

A solution of 2,2-dibromo-1-(3',4'-dimethoxyphenyl) ethene (prepared by the Wittig reaction of 3,4-dimethoxybenzaldehyde with carbon tetrabromide in the presence of triphenyl phosphine) (966 mg, 3 mmol) in dry THF (15 mL) was treated under N₂ with n-BuLi (2.5M in THF, 6 mmol, 2.4 mL) at -78°. The mixture was stirred at this temp. for 2 hours and a solution of 5β-pregnan-3,20-dione cyclic 20-(1,2-ethanediyl acetal) (720 mg, 2 mmol) in dry THF (10 mL) was added dropwise over a period of 30 min. After stirring the resulting mixture at -78°C for 2 hr, the cooling bath was removed and the stirring was continued at r.t. for another hr. It was then quenched with 2N HCl solution (1 mL) at -10°C. The solvent was removed and the residue was then dissolved in acetone (25 mL). After adding 2N HCl (10 mL) the solution was stirred at r.t. for 2 h. A saturated NaHCO₃ soln, was added to neutralize the acid. The solvents were removed and the residue was extracted with EtOAc. The organic layer was washed with water, dil. NaHCO₃ soln., water, and brine. After drying over anhyd. MgSO₄ the solution was filtered and evaporated to yield the crude product (1.2 g). This crude product was then dissolved in a small amount of CH₂Cl₂ and poured on a column of silica gel. Elution with toluene:acetone mixture (96:4) gave a phenylacetylene compound, which was not characterized. Further elution with the same solvent yielded 3α-(3',4'-dimethoxyphenylethynyl)-3β-hydroxy-5β-pregnan-20-one (120 mg) as a first fraction, and 3β-(3',4'-dimethoxyphenylethynyl)-3α-hydroxy-5β-pregnan-20-one as a second fraction (430 mg); mp 82-88°C.

An analogous method was used to prepare: 3β-(4'-methoxyphenylethnyl)-3α-hydroxy-5β-pregnan-20-one; 3β-(4'-Chlorophenylethynyl)-3α-hydroxy-5β-pregnan-20-one; 3β-(2'-Methoxyphenylethynyl)-3α-hydroxy-5β-pregnan-20-one; 3β-(4'-biphenylethynyl)-3α-hydroxy-5β-pregnan-20-one; 3β-(4'-dimethylaminophenylethynyl)-3α-hydroxy-5β-pregnan-20-one; 3β-(4'-Cyanophenylethynyl)-3α-hydroxy-5β-pregnan-20-one

### EXAMPLE 4

### 3β-(3',4'-Dimethoxyphenylethyl)-3α-hydro-5β-pregnan-20-one

A mixture of Pd/C (5%, 28 mg) and EtOAc (12 mL) was presaturated with hydrogen by stirring it under hydrogen for 10 min. A solution of 3β-(3',4'-dimethoxyphenylethynyl)-3α-hydroxy-5β-pregnan-20-one (185 mg) in EtOAc (5 mL) was then added and the mixture was hydrogenated at 300 Kpa pressure overnight at r.t. Filtration of the catalyst followed by evaporation of the solvent yielded the crude product, which was purified by chromatography over silica gel (hexane:acetone 4:1) to isolate the pure title compound (135 mg); TLC Rf (hexane:acetone 4:1) = 0.14.

### EXAMPLE 5

### 3β-(4'-Nitrophenylethynyl)-3α-hydroxy-5β-pregnan-20-one

A solution of 2,2-dibromo-1-(4-nitrophenyl)ethene (prepared by the Wittig reaction of 4-nitrobenzaldehyde with carbon tetrabromide in the presence of triphenyl phosphine) (296 mg, 1 mmol) in dry THF (20 mL) was treated under N₂ with n-BuLi (2.5M in THF, 2 mmol, 0.8 mL) at -95°. The mixture was stirred at -80 to -100°C for 0.5 hr. and then a solution of 5β-pregnan-3,20-dione cyclic 20-(1,2-ethanediyl acetal) (720 mg, 2 mmol) in dry THF (10 mL) was added dropwise over a period of 10 min. After stirring the resulting mixture at -80°C for 1 hr, and then at 0°C for 1 more hr. it was quenched with NH₄Cl solution (3 mL). The solvent was removed and the residue was then dissolved in acetone (25 mL). After adding 2N HCl (10 mL) the solution was stirred at r.t. for 1 h. Saturated NaHCO₃ soln. was added to neutralize the acid. The solvents were removed and the residue was extracted with CH₂Cl₂. The organic layer was washed with water, and brine. After drying over anhyd. MgSO₄ the solution was filtered and evaporated to yield the crude product (400 mg). This crude product was then dissolved in a small amount of CH₂Cl₂ and poured on a column of silica gel. Elution with toluene:acetone mixture (96:4) gave the title compound as a brown solid (70 mg); TLC Rf (toluene:acetone 95:5) = 0.18.

### EXAMPLE 6

### 3α-hydroxy-3β-phenyl-5β-pregnan-20-one

A solution of 5β-pregnan-3,20-dione cyclic 20-(1,2-ethanediyl acetal) (720 mg, 2 mmol) in dry THF was treated with Phenyl magnesium bromide (3M in THF, 6 mmol, 2 mL) at -70°C. After stirring the mixture at this temp. for 3 h and then at r.t. for 2 h, it was quenched with 2N HCl (10 mL). The solvent was removed and the residue was dissolved in acetone (20 mL). After adding 1N HCl (5 mL) the solution was stirred at r.t. for 15 h. The solvents were removed and the residue was extracted with CH₂Cl₂. The organic layer was washed with water, dil. NaHCO₃ soln., water, and brine. After drying over anhyd. MgSO₄ the solution was filtered and evaporated to yield the crude product (1.3 g). This crude product was then dissolved in a small amount of CH₂Cl₂ and poured on a column of silica gel. Elution with toluene:acetone mixture (95:5) gave 3α-phenyl-3β-hydroxy-5β-pregnan-20-one (420 mg) as a first fraction. Further elution with the same solvent mixture yielded 3β-phenyl-3α-hydroxy-5β-pregnan-20-one (185 mg), m.p. 182-184°.

### EXAMPLE 7

### 3α-hydroxy-3β-benzyl-5β-pregnan-20-one

A solution of benzyl magnesium bromide (2M in THF, 2 mmol, 1 mL) was diluted with THF (15 mL) and was treated dropwise with a solution of 5β-pregnan-3,20-dione cyclic 20-(1,2-ethanediyl acetal) (360 mg, 1 mmol) in dry THF (15 mL) at -60°. After stirring the mixture at this temperature for 1 h and then at r.t. for 15 h, it was quenched with 2N HCl (10 mL). The solvent was removed and the residue was dissolved in acetone (20 mL). After adding lN HCl (5 mL) the solution was stirred at r.t. for 30 min. It was neutralized with 2N NaOH. The precipitated solid was collected by filtration, washed with water and dried to yield 3β-hydroxy-3α-benzyl-5β-pregnan-20-one (238 mg). The filtrate was extracted with EtOAc. The organic layer was washed with water, dil. NaHCO₃ soln., water, and brine. After drying over anhyd. MgSO₄ the solution was filtered and evaporated to give the crude product (160 mg). This crude product was then dissolved in a small amount of CH₂Cl₂ and poured on a column of silica gel. Elution with toluene:acetone mixture (95:5) gave 3β-hydroxy-3α-benzyl-5β-pregnan-20-one (40 mg) as a first fraction. Further elution with the same solvent mixture yielded 3α-hydroxy-3β-benzyl-5β-pregnan-20-one (30 mg), which was crystallized from hexane : CH₂Cl₂ (4:1) as colorless rods (15 mg); m.p. 133-141°C; TLC Rf (toluene:acetone 9:1) = 0.5.

### EXAMPLE 8

### 3β-(1-Heptynyl)-3α-hydroxy-5β-pregnan-20-one

A solution of 1-heptyne (0.327 mL, 2.5 mmol) in dry THF (15 mL) was treated with n-BuLi (2.5M in THF, 2.5 mmol, 1 mL) at -78°C. After stirring the mixture at this temperature for 1 hr, a solution of 5β-pregnan-3,20-dione cyclic 20-(1,2-ethanediyl acetal) (360 mg, 1 mmol) in dry THF (15 mL) was added and the mixture was stirred at -78°C for 1 hr. It was then quenched with 2N HCl solution (1 mL). The solvent was removed and the residue was then dissolved in acetone (10 mL). After adding 2N HCl (10 mL) the solution was stirred at r.t. for 1 hr. Saturated NaHCO₃ soln. was added to neutralize the acid. The solvents were removed and the residue was extracted with EtOAc. The organic layer was washed with water, dil. NaHCO₃ soln., water, and brine. After drying over anhyd. MgSO₄ the solution was filtered and evaporated to yield the crude product (400 mg). This crude product was then dissolved in a small amount of CH₂Cl₂ and poured on a column of silica gel. Elution with toluene:acetone mixture (93:7) gave 3β-(1-heptynyl)-3α-hydroxy-5β-pregnan-20-one (260 mg) as a colorless solid; mp 121-123°C.

An analagous method was used to prepare: 3β-(1-Hexynyl)-3α-hydroxy-5β-pregnan-20-one; 3β-(1-Octynyl)-3α-hydroxy-5β-pregnan-20-one; 3α-Hydroxy-3β-[6-oxo-1-heptynyl]-5β-pregnan-20-one, using 1-heptyne-6-one cyclic (1,2-ethanediyl acetal).

### EXAMPLE 9

### 3β-[3'(R/S)-Hydroxybutynyl]-3α-hydroxy-5β-pregnan-20-one

A solution of 3(R/S)-hydroxybutyne (0.470 mL, 6 mmol) in dry THF (15 mL) was treated with n-BuLi (2.5M in THF, 12 mmol, 4 mL) at -70°C. After stirring the mixture at this temperature for 0.5 hr, a solution of 5β-pregnan-3,20-dione cyclic 20-(1,2-ethanediyl acetal) (1.08 g, 3 mmol) in dry THF (30 mL) was added and the mixture was stirred at -78°C for 1 hr. The cooling bath was removed and the mixture was stirred at r.t. for another 1.5 hr. It was then quenched with NH₄Cl solution (3 mL). The solvent was removed and the residue was then dissolved in acetone (10 mL). After adding 2N HCl (5 mL), the solution was stirred at r.t. for 1 hr. Saturated NaHCO₃ soln. was added to neutralize the acid. The solvents were removed and the residue was extracted with EtOAc. The organic layer was washed with water, dil. NaHCO₃ soln., water, and brine. After drying over anhyd. MgSO₄ the solution was filtered and evaporated to yield the crude product (1.4 g). This crude product was then dissolved in a small amount of CH₂Cl₂ and poured on a column of silica gel. Elution with toluene:acetone mixture (85:15) gave 3β-[3'(RS)-hydroxy-butynyl]-3α-hydroxy-5β-pregnan-20-one (145 mg) as a colorless solid; TLC Rf (toluene:acetone 4:1) = 0.24.

An analogous method was used to prepare: 3β-[4'(R/S)-hydroxypentynyl]-3α-hydroxy-5β-pregnan-20-one.

### EXAMPLE 10

### 3β-(4'-Acetylphenylethynyl)-3α-hydroxy-5α-pregnan-20-one

A solution of 4-iodoacetophenone (95 mg, 0.39 mmol), 3β-ethynyl-3α-hydroxy-5α-pregnan-20-one (106 mg, 0.3 mmol) in dry degassed pyrrolidine (3 mL) was stirred under argon at r.t. Bis(triphenylphosphine)palladium chloride (5 mg) and CuI (5 mg) were added and the mixture was stirred at r.t. for 15 hr. The TLC showed 100% conversion of the starting material, hence, the mixture was quenched with NH₄Cl solution (15 mL) and was extracted with EtOAc. The organic layer was washed with water, dil. NaHCO₃ soln., water, and brine. After drying over anhyd. MgSO₄ the solution was filtered and evaporated to yield the crude product (150 mg). This crude product was then dissolved in a small amount of CH₂Cl₂ and poured on a column of silica gel. Elution with hexane:acetone mixture (4:1) afforded 3β-(4'-acetylphenylethynyl)-3α-hydroxy-5α-pregnan-20-one (35 mg) as a colorless solid; TLC Rf (hexane:acetone 7:3) = 0.4.

An analogous method was used to prepare: 3α-Hydroxy-3β-(4'-trifluoromethylphenylethynyl)-5β-pregnan-20-one; 3α-Hydroxy-3β-(4'-methylphenylethynyl)-5β-pregnan-20-0ne; 3α-Hydroxy-3β-(4'-acetoxyacetylphenylethynyl)-5β-pregnan-20-one; 3α-Hydroxy-3β-(pentafluorophenyl)ethynyl-5β-pregnan-20-one; 3β-(2'-Hydroxyphenyl)ethynyl-3α-hydroxy-5β-pregnan-20-one; 3β-(3'-Hydroxyphenyl)ethynyl-3α-hydroxy-5β-pregnan-20-one; and 3α-Hydroxy-3β-(4'-carboxyphenylethynyl)-5β-pregnan-20-one, ethyl ester.

### EXAMPLE 11

### 3α-Hydroxy-3β-methoxymethyl-5α-pregnan-20-one

The 3(R)-epoxy-5α-pregnan-20-one was prepared by the reaction of 5α-pregnan-3,20-dione with trimethylsulfoxonium methyliodide in the presence of a strong base, such as potassium t-butoxide, in THF.

To a solution (there were few undissolved solid particles) of 3(R)-epoxy-5α-pregnan-20-one (5.145 g, 15.57 mmol) in CH₃OH (600 mL) was added freshly cut Na (- 600 mg). CAUTION: H₂ gas evolution. After all Na had reacted, a clear solution resulted. The reaction solution was stirred overnight. Since TLC (3:1 hexane/acetone) indicated mainly the starting material, the mixture was heated (close to boiling point) for 8 h. and then left at room temperature overnight. TLC still showed a small amount of the starting material, besides two new, less-polar spots. Therefore, the mixture was heated for another 8 h. at which time there was no unreacted starting epoxide. After the reaction solution had come to room temperature, glacial acetic acid (3 mL) was added dropwise. The solvent was then removed under reduced pressure, and CH₂Cl₂ and water were added. The aqueous layer was back-extracted with CH₂Cl₂. The combined organics were washed with saturated NaHCO₃, dried (MgS04), filtered, and evaporated under reduced pressure to give a white solid (5.55 g), which was found to be a 84:16 mixture of 3α-hydroxy-3β-methoxymethyl-5α-pregnan-20-one and its 17-epimer on the basis of its ¹H NMR. Recrystallization from hot 1:1 hexane/acetone furnished only 3α-hydroxy-3β-methoxymethyl-5α-pregnan-20-one (3.270 g, 58%) as white crystals. mp 163-4 °C. As expected, the mother liquors contained both 17-epimers in approximately equal amounts.

An analogous method was used to prepare: 3α-hydroxy-3β-ethoxymethyl-5α-pregnan-20-one; 3α-hydroxy-3β-propoxymethyl-5α-pregnan-20-one;

### EXAMPLE 12

### 3α-hydroxy-3β-cyanomethyl-5α-pregnan-20-one

Potassium cyanide (210mg; 3.2mmol) and 20,20-ethylenedioxy-3R-spiro-2'-oxirane-5α-pregnan-20-one (1.0 g; 2.7mmol) were stirred in ethanol (175mL) for 2 days, refluxed for 5 hours and then allowed to cool overnight. Ether (100mL) and brine (100mL) were added to the reaction mixture. The separated aqueous phase was washed with ether and the combined organic phases were washed with brine. Aqueous HCl (1M; 6mL) and THF (15mL) were added to the organic phases and the solution stirred overnight. Aqueous NaHCO₃ (20mL) was added and then water (50mL). The separated aqueous phase was washed with ether and the combined organic phases were dried over MgSO₄ and evaporated. The white solid residue was purified by flash column chromatography (40:20:1 CH₂Cl₂/hexane/acetone) to give a white solid (493mg; 52%); mp 218.5 - 220° C.

### EXAMPLE 13

### 3α-Hydroxy-3β-azidomethyl-5α-pregnan-20-one

To a solution of 3(R)-epoxy-5α-pregnan-20-one (1.012 g, 3.06 mmol) in dry DMF (Aldrich; 50 mL) was added NaN₃ (226 mg, 3.48 mmol), all of which did not dissolve. Then glacial acetic acid (1.05 mL, 17.5 mmol) was added, and the resulting mixture was heated a little bit with a heat gun in order to dissolve all the solid. The mixture was stirred for a while, giving a clear solution. TLC (100:1 CH₂Cl₂/acetone or 3:1 hexane/acetone) after stirring the mixture for 4 h still indicated the presence of starting material. The reaction mixture was then heated with stirring at 35-50 °C for 3 days. By TLC the reaction was complete. CH₂Cl₂ and water were added. The aqueous layer was back-extracted with CH₂Cl₂. The combined organics were washed with saturated NaHCO₃, dried (MgSO₄), filtered, and concentrated on rotary evaporator to give a liquid containing a lot of DMF. Further Evaporation by distillation under high vacuum provided a solid (1.09 g). ¹H NMR of this solid revealed the presence of mainly the desired 3α-hydroxy-3β-azidomethyl-5α-pregnan-20-one and almost none of its 17-epimer. Purification by flash column chromatography using CH₂Cl₂ as eluant afforded. 3α-hydroxy-3β-azidomethyl-5α-pregnan-20-one (834 mg, 73%) as a white solid; mp 147-8°C.

### EXAMPLE 14

### 3α,20α-Dihydroxy-5α-19-nor-pregnane

To a solution of 3α-hydroxy-5α-19-nor-17(Z)-pregn-17(20)-ene (232 mg, 0.81 mmol) (prepared from the androstane-17-one with the Wittig reagent as described below) in THF (20 mL) was added diborane THF complex (1M solution in THF, 3.0 mL) dropwise at 0°C. The reaction was allowed to warm to 25°C for 2.5 h. Then a solution of sodium hydroxide (2N, 15 mL) was added very slowly at 0°C followed by an addition of hydrogen peroxide (30%, 8 mL). Water then was added and THF layer was separated by separatory funnel. The aqueous layer was extracted with ethyl acetate (2x40 mL). The organic solution was dried over potassium carbonate and the solvent removed in vacuo. A column chromatography using acetone and methylene chloride in hexane (8:8:84) resulted in a small amount of pure product (15mg).

### EXAMPLE 15

### 3α,21-hydroxy-3β-methyl-5α-pregnan-20-one, 21-hemisuccinate

A mixture of 3α,21-hydroxy-3β-methyl-5α-pregnan-20-one (600 mg, 1.72 mmol) and succinic anhydride (388 mg, 3.88 mmol) in 12 mL of pyridine was allowed to stir at rt for 23h. The reaction was concentrated in vacuo and the residue was partioned between EtOAc and an aqueous HOAc solution (pH 4). The organic layer was separated and washed with a sat. NaCl solution and concentrated. The crude hemisuccinate was purified by flash chromatography (12 cm of silica in a 3.5 cm dia. column, eluted with 400 mL of 45/55 EtOAc/CH₂Cl₂ and 200 mL each of 50/50 and 60/40 EtOAc/CH₂Cl₂. Recrystallization from MeOH/water gave 346 mg (45%) of the hemisuccinate as a white solid.

The sodium salt of the hemisuccinate was prepared by dissolving the acid in methanol and adding one equivalent of an aqueous solution of sodium bicarbonate. The solvent was removed in vacuo and the residue was triturated with ether/pet. ether to give a white solid.

### EXAMPLE 16

### a. Triphenylprop-2-ynylstannane

Propargyl bromide (80 w% solution in toluene; 1.7 mL, 11.43 mmol) was added to a mixture of Mg (471 mg, 19.38 mmol) and dry Et₂O (25 mL). HgCl₂ (a few crystals) was then added. The mixture was stirred for -5 min. Since no reaction occurred, more HgCl₂ (15 mg) was added. The resulting mixture was first stirred at room temperature and then warmed briefly. The mixture became turbid, and the reaction ensued. The mixture was stirred at <20°C (cold water bath) for -1 h after which time most of Mg had reacted. The amount of the unreacted Mg remained unchanged over a period of -30 min. Therefore, propargyl bromide (80 w% in toluene; 0.4 mL, 2.69 mmol) was added. The mixture was stirred at <20°C for -1 h. Subsequently, a suspension of triphenyltin chloride (95%; 5.045 g, 12.43 mmol) in Et₂O (50 mL) was added dropwise through a cannula to the above solution of propargylmagnesium bromide. The resulting mixture, which contained a white solid, was stirred at room temperature for -3 h. TLC (3:1 hexane/acetone) showed complete consumption of the starting tin chloride. The mixture was quenched with saturated aqueous NH₄Cl (10 mL). A white solid was formed. The organic layer was separated from the aqueous layer that contained the white solid. Finally, the organic phase was dried (MgSO₄), filtered, and evaporated under reduced pressure to give a white solid (4.78 g). Recrystallization of the solid from hot hexane (50 mL) furnished triphenylprop-2-ynylstannane as white plate-shaped crystals (3.92 g, 81%).

### b. 3α-Hydroxy-3β-(butadi-2',3'-enyl)-5α-pregnan-20-one (Reference: Baldwin, J. E.; Adlington, R. M.; Basak, A. J. Chem. Soc., Chem. Commun. 1984, 1284)

A mixture containing 3β-iodomethyl-3α-hydroxy-5α-pregnan-20-one (234 mg, 0.51 mmol), triphenylprop-2-ynylstannane (795 mg, 2.04 mmol), azobisisobutylnitrile (18 mg, 0.11 mmol), and dry benzene (2 mL) was degassed for -10 min. The above mixture was then refluxed for 2 h. As indicated by TLC (100:1 CH₂Cl₂/acetone), there was still some (-33%) unreacted starting material, the amount of which remained unchanged over a period of 2 h. TLC showed, besides the starting material, two spots in -1:1 ratio, the less-polar spot being a new spot while the other corresponding to 3β-methyl-3α-hydroxy-5α-pregnan-20-one. The reaction mixture was cooled to room temperature. After removing the solvent, the residue was subjected to flash chromatography to afford 3α-hydroxy-3β-(butadi-2',3'-enyl)-5α-pregnan-20-one as a white solid (20 mg, 11%).

### EXAMPLE 17

### 3α-Hydroxy-3β-(prop-2'-ynyl)-5α-pregnan-20-one

To a mixture of Mg (268 mg, 11.02 mmol), propargyl bromide (80 w% ; 1.5 mL, 10.09 mmol) and dry Et₂O (15 mL) was added HgCl₂ (10 mg). The resulting mixture was slightly warmed in a water bath in order to initiate the reaction. The mixture became turbid, and the reaction ensued. The mixture was stirred at <20°C until all Mg reacted (for -1 h).

A solution of 20,20-ethylenedioxy-5α-pregnan-3-one (711 mg, 1.97 mmol) in dry Et₂O (20 mL) and dry THF (10 mL) was cooled to -78°C. Some of the starting material precipitated on cooling. Subsequently, propargylmagnesium bromide solution (-0.6 M; 6.5 mL), prepared above, was added dropwise at -78°C. The resulting mixture was stirred at -78°C for -1 h. The mixture was then allowed to warm to room temperature where it was stirred for 1 h. IN HCl, p-toluenesulfonic acid monohydrate, and acetone were added in order to hydrolyze the ketal function. The two-phase mixture was stirred at room temperature overnight. TLC (7:1 hexane/acetone) showed completion of the reaction. Et₂O and water were added. The aqueous layer was back-extracted with Et₂O. The combined organics were washed with saturated aqueous NaHCO₃ and brine, dried (MgSO₄), and evaporated under reduced pressure to give a white solid (690 mg). ¹H NMR of the crude solid indicated it to be a 1:2 mixture of the desired 3α-hydroxy-3β-(prop-2'-ynyl)-5α-pregnan-20-one and its 3-epimer, respectively. The mixture was flash-chromatographed twice with 10:1 hexane/acetone to provide 3α-hydroxy-3β-(prop-2'-ynyl)-5α-pregnan-20-one as a white solid (70 mg, 11%). m.p. 220-225°C (dec.).

### EXAMPLE 18

### 3α-Hydroxy-3β-(but-3'-enyl)-5α-pregnan-20-one

### (Reference: Keck, G. E.; Yates, J. B. J. Am. Chem. Soc. 1982, 104, 5829)

Dry benzene (3 mL) was degassed for 15 min by bubbling Ar through it. Next were added successively 3β-iodomethyl-3α-hydroxy-5α-pregnan-20-one (263 mg, 0.574 mmol), allyltributyltin (97%; 0.5 mL, 1.56 mmol), 1,2'-azobisisobutyronitrile (20 mg, 0.122 mmol). The resulting mixture was refluxed for 3.5 h. TLC (100:1 CH₂Cl₂/acetone) showed complete consumption of the starting iodide derivative, with the appearance of two new spots in -1:1 ratio, the more-polar spot corresponding to 3β-methyl-3α-hydroxy-5α-pregnan-20-one. The reaction mixture was cooled to room temperature. After removing the solvent, the residue was subjected to flash chromatography twice, with CH₂Cl₂ and 7:1 hexane/acetone. 3α-Hydroxy-3β-(but-3'-enyl)-5α-pregnan-20-one was obtained as a white solid (75 mg, 35%). m.p. 140-141°C.

### EXAMPLE 19

### a. 3α-Hydroxy-5β-pregnan-20-one, 20-ketal

A mixture of 3α-hydroxy-5β-pregnan-20-one (10.8 g, 34 mmol), ethylene glycol (45 mL), and triethyl orthoformate (30 mL) was stirred at r.t. for 5 min. p-toluenesulfonic acid (200 mg) was then added and the stirring was continued at r.t. for 1.5 hr. The resulting thick paste was poured into a sat. NaHCO₃ solution (250 mL). The precipitated solid was collected by filtration, washed thoroughly with cold water and dried. This semi-dried product was dissolved in CH₂Cl₂ (350 mL) and dried over anhyd. K₂CO₃. The solution of the ketal was then filtered and used as such for the next step.

### b. 5β-Pregnan-3,20-dione, 20-ketal

The above solution of 3α-hydroxy-5β-pregnan-20-one, 20-ketal in CH₂Cl₂ was stirred with N-methylmorpholine-N-oxide (8.8 g, 75 mmol), and powdered 4Å molecular sieves (58 g) under N₂ for 15 min. Tetrapropylammonium perruthenate (400 mg) was then added and the stirring was continued at r.t. for 2 hr. The resulting dark green mixture was passed through a short column of Florisil and eluted with CH₂Cl₂. The fractions containing the product (TLC) were combined and evaporated. The crude product was then crystallized from a mixture of EtOAc:Hx (1:1) to yield the title compound (10.3 g) as long rods.

### c. Preparation of the lithium reagent from 1,2-dibromoethylene

A 100 mL three neck flask equipped with a N₂ gas bubbler, a thermometer, and a dropping funnel was charged with 1,2-dibromoethylene (cis/trans mixture, 98%, Aldrich, 0.164 mL, 2 mmol, mw = 186, d = 2.246). Dry THF (15 mL) was added and the solution was cooled to -78°C in a dry ice-acetone bath. n-BuLi (2.5M in THF, 1.6 mL, 4 mmol) was added dropwise over a period of 10 min. The mixture was stirred at this temperature for 1 hr and the resulting reagent was used immediately for the next step.

### d. 3β-Ethynyl-3α-hydroxy-5β-pregnan-20-one

The above solution of the reagent in THF, which was maintained at -78°C, was treated dropwise with a solution of 5β-pregnan-3,20-dione, 20-ketal (180 mg, 0.5 mmol) in THF (15 mL). The temperature was maintained below -70°C during the addition. The stirring was continued at this temperature for 15 min. (100% conversion as detected by TLC). The cooling bath was removed and the resulting solution was quenched with 2N HCl (pH 6). The solvent was removed and the residue was dissolved in acetone (10 mL). After adding 2N HCl (4 mL) the solution was stirred at ambient temperature for 0.5 h. The mixture was neutralized with dil. NaHCO₃ solution. The precipitated solid (158 mg, 93%) was collected by filtration, washed with water, and dried. The crude product is then purified by crystallization from EtOAc or from a mixture of acetonehexane to yield the title compound; mp 196-197°C, TLC-R. 0.45 (hexane:acetone 7:3).

### e. 3β-Ethyl-3α-hydroxy- 5β-pregnan-20-one

A mixture of Pd/C (5%, 24 mg) and MeOH (12 mL) was presaturated with hydrogen by stirring the mixture under hydrogen for 10 min. A solution of 3β-ethynyl-3α-hydroxy-5β-pregnan-20-one(155 mg) in MeOH (5 mL) was then added, and the mixture was hydrogenated for 1.5 h. under 200 Kpa pressure at r.t. Filtration of the catalyst followed by evaporation of the solvent yielded the title compound, which was purified by crystallization from hexane:acetone (9:1); yield : 105 mg; mp 115-117° C. TLC Rf (hexane:acetone 7:3) = 0.48.

### f. 3β-Ethyl-3α-hydroxy-5α-pregnan-20-one :

To a mixture of 570 mg (3 mmol) of cuprous iodide and 10 ml of dry ether cooled to -60° C under argon was added 4 ml of 1.5M methyl lithium. The reaction mixture was warmed to 0° C, stirred until a colorless solution was obtained and then recooled to -60° C. An additional 0.5-1 ml of methyl lithium solution was added to keep the solution colorless. A cooled (0° C) solution of 0.5 g (1.5 mmol) of 3*R*-spiro-2'-oxirane-5α-pregnan-20-one in 20 ml of ether was added slowly to the above reaction mixture while maintaining the temperature at -60° C. An additional 0.2 ml of methyl lithium was added and the reaction mixture was stirred at -60° C for 1 hr. It was then quenched by the addition of saturated ammonium chloride solution, allowed to warm to room temperature and diluted with ether and ammonium chloride solution. The organic phase was separated. The aqueous phase was extracted with ether. The combined organic extracts were dried and concentrated to leave 450 mg of crude product. Chromatography on silica gel using 5% acetone/hexane gave 356 mg of product.

### EXAMPLE 20

### Disodium 3α,20β-dihydroxy-5β-cregnane, bis(hemisuccinate)

A suspension of 3α,20β-dihydroxy-5β-pregnane (Steraloids; 250 mg, 0.78 mmol) in 5 mL of dry pyridine was treated with succinic anhydride (200 mg, 2.0 mmol). The mixture was heated at 100°C for a total of 10 h. An additional 6 mmol of succinic anhydride was added in three portions as the reaction was heated. The dark mixture was concentrated (0.05 mmHg, 30°C) to remove the solvent and then heated at 90°C (0.05 mmHg) to remove excess succinic anhydride. The residue was recrystallized from ether/hexanes giving a solid that consisted mainly of succinic acid. The mother liquor was concentrated and subjected to column chromatography (flash silica gel, eluted with 95/5/0.1 CH₂Cl₂/MeOH/HOAc) giving a white solid which was recrystallized from ether/hexane. The bis(hemisuccinate), mp 81-90°C was carried on to the bis(sodium salt).

The bis(hemisuccinate) (100 mg, 0.192 mmol) was dissolved in a minimum volume of methanol. A solution of NaHCO₃ (2 eq, 33 mg, 0.393 mmol) in 0.6 mL of water was added dropwise. After 3h, the solution was concentrated in vacuo to give a white solid.

### EXAMPLE 21

### a. 3α-Hydroxy-3β-methyl-5α-androstane17β-carboxylic acid

Bromine (2.0 mL, 38.8 mmol) was added dropwise to an aqueous solution of NaOH (3.746 g in 30 mL of water) at 0°C. Subsequently, a solution of 3α-hydroxy-3β-methyl-5α-pregnan-20-one in dioxane (250 mL) and water (30 mL) was added to the above solution at 0°C over a period of 1 h. The resulting yellow mixture that contained some solid was allowed to warm to room temperature. After some time all the solid dissolved. The reaction solution was stirred overnight at which point it turned colorless. Then CH₂Cl₂ (150 mL), water (100 mL) and 1N HCl (50 mL) were added. Brine was also added to facilitate the separation of the two liquid phases. The aqueous layer was separated and back-extracted with CH₂Cl₂ (200 mL). The two organic phases were combined and washed with water, dried (MgSO₄), filtered, and evaporated under reduced pressure to afford a white solid (3.851 g). Recrystallization of this solid from hot 1:1 hexane/acetone (500 mL) gave 3α-hydroxy-3β-methyl-5α-androstane 17β-carboxylic acid as a white solid (1.838 g, 50%). m.p. 222.5-224°C. Evaporation of the mother liquor followed by recrystallization from hot methanol furnished more product as white crystals (1.50 g, 40%). m.p. 223-225°C.

### b. 17β-(Hydroxymethyl)-3β-methyl-5α-androstan-3α-ol

To a stirred suspension of LiAlH₄ (95%; 546 mg, 13.67 mmol) in dry THF (30 mL) at 0°C was added a solution of 3α-hydroxy-3β-methyl-5α-androstane 17β-carboxylic acid (1.633 g, 4.88 mmol) in dry THF (50 mL + 10 mL for the rinse) through a pressure-equalizing funnel over a period of 30 min. The resulting mixture was then allowed to warmed to room temperature. The stirring was continued overnight. TLC (7:1 hexane/acetone) showed completion of the reaction. The reaction mixture was cooled to 0°C, and 1N HCl (25 mL) was added dropwise. Subsequently, Et₂O (100 mL), water (25 mL), and conc. HCl (5 mL) were added. The aqueous layer was still not completely clear. The two phases were separated. Conc. HCl (15 mL) was added to the aqueous layer which was then extracted with Et₂O (100 mL). The two organic layers were combined, washed with saturated aqueous NaHCO₃, dried (MgSO₄), filtered, and evaporated under reduced pressure to afford 17β-(hydroxymethyl)-3β-methyl-5α-androstan-3α-ol as a white solid (1.605 g). ¹H NMR of the product showed it to contain some (4%) unreacted starting material and THF (4%).

### c. 17β-Formyl-3β-methyl-5α-androstan-3α-ol

To a solution of 17β-(hydroxymethyl)-3β-methyl-5α-androstan-3α-ol (96% pure; 1.58g, 4.73 mmol) in dry CH₂Cl₂ (90 mL) at room temperature was added pyridinium chlorochromate (98%; 1.115 g, 5.07 mmol). The resulting mixture was stirred at room temperature for 7 h. TLC (7:1 hexane/acetone) showed complete consumption of the starting acid. The mixture was then filtered through neutral florisil and successively washed with CH₂Cl₂ (250 mL) and 5:1 hexane/acetone (100 mL). (The filtrate was checked by TLC for the presence of the product.) The solvents were removed to give a light yellow solid (1.390 g) which was purified by flash chromatography employing CH₂Cl₂ as eluent to furnish 17β-formyl-3β-methyl-5α-androstan-3α-ol as a white solid (1.081 g, 72%). m.p. 156-166°C.

### d. 17β-cyano-3α-hydroxy-5α-androstane

To a solution of 3α-hydroxy-5α-androstan-17-one (1.511 g, 5.20 mmol) in 135 mL of 1,2-dimethoxyethane (DME) was added a 1M solution of potassium tert-butoxide in THF (52 mL, 52 mmol). The resulting cloudy orange solution was treated over 2.5 h. with a solution of 2.06 g (10.6 mmol) of *p*-toluenesulfonylmethyl isocyanide in 30 mL of DME. After an additional 2.5 h., the reation was diluted with 100 mL of ether and 100 mL of water was added. The aqueous layer was extracted with ether (2 x 50 mL) and the pooled organic layers were washed with a brine solution, dried (MgSO₄) and concentrated. Column chromatography on silica gel (100% of CH₂Cl₂ as eluent) gave 860 mg of slightly impure product. Recrystallization from EtOAc gave 300 mg (19%) of pure nitrile, mp 159-160°C.

### EXAMPLE 22

### 3α-hydroxy-5β-pregn-17(20)-ene

To the ylide prepared from ethyltriphenylphosphonium bromide (Aldrich; 1.606 g, 4.33 mmol) and solid potassium *tert*-butoxide (475 mg, 4.23 mmol) was added 3α-hydroxy-50-androstan-17-one (Steraloids; 503 mg, 1.73 mmol) in one portion. The red-orange mixture was heated at reflux for 3.5 h and then allowed to cool. It was further cooled to 0°C and then added to an ice-cold sat. NH₄Cl solution/ether mixture. The aqueous layer was separated and washed twice with ether. The ether layers were then extracted with a sat. NaCl solution, dried (MgSO₄) and concentrated. Flash chromatography (17.5 cm of silica in a 4 cm dia. column, eluted with 400 mL of 2% acetone/CH₂Cl₂ and 200 mL of 2.5% acetone/CH₂Cl₂) afforded 530 mg (100%) of the alkene as a white solid. By ¹H NMR the compound was an 8:1 mixture of *Z-* and *E*-isomers.

### EXAMPLE 23

### 3α-hydroxy-3β-ethenyl-5β-pregnan-20-one

A solution of 5β-pregnan-3,20-dione, 20-ketal (1.18g, 3.3 mmol) in dry THF (20 mL) was treated with vinyl magnesium bromide (1M in THF, 3.7 mmol, 3.7 mL) at -70°. After stirring the mixture at this temp. for 5 min. and then at r.t. for 2.5 h, it was quenched with sat. NH₄Cl solution (10 mL). The solvent was removed and the residue was extracted with EtOAc. The organic layer was washed with water, dil. NaHCO₃ soln., water, and brine. After drying over anhyd. MgSO₄ the solution was filtered and evaporated to yield the crude product (1.2 g). This crude product was then dissolved in acetone (20 mL). After adding 1N HCl (10 mL) the solution was stirred at r.t. for 15 h. The solvents were removed and the residue was extracted with CH₂Cl₂. The organic layer was washed with water, dil. NaHCO₃ soln., water, and brine. After drying over anhyd. MgSO₄ the solution was filtered and evaporated to yield the crude product (890 mg). This crude product was then dissolved in a small amount of CH₂Cl₂ and poured on a column of silica gel. Elution with toluene:acetone mixture (94:6) gave 3α-ethenyl-3β-hydroxy-5β-pregnan-20-one (126 mg) as a first fraction. Further elution with the same solvent mixture yielded 3β-ethenyl-3α-hydroxy-5β-pregnan-20-one (189 mg), m.p. 113-116°.

### EXAMPLE 24

### a. 3α-Hydroxy-5α-androstan-17-one

To a solution of 3β-hydroxy-5α-androstan-17-one (6g) and diethyl azodicarboxylate (5.04 g) in THF (50 mL) was added trifluoroacetic acid (3.3 g) and the mixture became yellow. Then triphenylphosphine (7.6 g) was added. The reaction turned to colorless and heat was given off. Sodium benzoate was added after 5 min and then water (100 mL) was added. The mixture was extracted with methylene chloride (3x80 mL) and organics was dried over magnesium sulfate. The solvent was removed in vacuo and the crude product was hydrolyzed with potassium hydroxide (10%, 10 mL) in methanol (150 mL) for 1 h. Most of the methanol was then removed in vacuo and the remaining material was partitioned between methylene chloride and ammonium chloride. The product (4.7 g, 78%) was purified by chromatography (CH₂Cl₂: acetone=9:1).

### b. 3α-Hydroxy-21-methyl-5α-pregn-17(Z)-ene

3α-Hydroxy-5α-androstan-17-one (2 g, 6.9 mmol) was added to Wittig reagent produced from propyltriphenylphosphonium bromide (13.3 g) and potassium t-butoxide (3.9 g) in THF (20 mL). The reaction was refluxed for 12 h and cooled to 25°C. Then a solution of ammonium chloride (60 mL) was added and the organic layer was separated by separatory funnel. The aqueous layer was extracted with methylene chloride (2x50 mL). The organic solution was dried over potassium carbonate and the solvent removed in vacuo. The crude product was purified by chromatography (acetone:methylene chloride:hexane = 1:2:7) to produce 0.84 g of product (39%). It was mixture of Z and E isomers (Z:E=13:1).

An analogous procedure was used to prepare 3α-hydroxy-17-methylene-5α-androstane using the Wittig reagent produced from methyltriphenylphosphonium bromide.

### c. 3α-t-Butyldimethylsiloxy-21-methyl-5α-pregn-17(Z)-ene

The mixture of 5α-3α-hydroxy-21-methyl-pregn-17(Z)-ene (0.84 g, 2.66 mmol), TBDMSCl (1.2 g, 8.0 mmol) and imidazole (0.91 g, 13.3 mmol) in methylene chloride (10 mL) and DMF (30 mL) was stirred for 12 h and then. ammonium chloride was added. It was then extracted with methylene chloride (3x40 mL) and washed with brine (50 mL). The organic solution was dried over potassium carbonate and then solvent removed and it was found that there was still some DMF. It was then redissolved in ether and washed with brine (2x50 mL) and then dried over potassium carbonate. Chromatography with hexane resulted 1.14 g of the pure product (100%) .

### d. 3α-t-Butyldimethylsiloxy-20α-hydroxy-21-methyl-5α-pregnane

To a solution of 3α-*t*-butyldimethylsiloxy-21-methyl-5α-pregn-17(Z)-ene (1.14 g, 2.66 mmol) in THF (30 mL) was added diborane THF complex (1M solution in THF, 5.3 mL) dropwise at 0°C. The reaction was allowed to warm to 25°C for 1 h. Then a solution of sodium hydroxide (20%, 10 mL) was added very slowly at 0°C followed by an addition of hydrogen peroxide (30%, 10 mL). Aqueous ammonium chloride then was added and THF layer was separated by separatory funnel. The aqueous layer was extracted with ether (2x40 mL). The organic solution was dried over potassium carbonate and the solvent removed in vacuo. The pure product was obtained by column chromatography (0.52 g, 44%).

### e. 3α,20α-Dihydroxy-21-methyl-5α-pregnane

A solution prepared by mixing HF (48%, 5 mL) and CH₃CN (30 mL) was added to a flask containing 3α*-t*-butyldimethylsiloxy-20α-hydroxy-21-methyl-5α-pregnane (0.51 g) and a white precipitate appeared. The reaction was stirred for 1 h and then filtered. The white solid was washed with ether for three times and it gave satisfactory analytical results (0.3 g, 79%). m.p. 227-231°C.

### EXAMPLE 25

### 3α,21-Dihydroxy-3β-trifluoromethyl-5β-19-nor-pregnan-20-one:

To a solution of 3α-hydroxy-3β-trifluoromethyl-5β-19-nor-pregnan-20-one (300 mg, 0.87 mmol) in toluene (15 mL) was added MeOH (1 mL) and BF₃•OEt₂ (1.4 mL, 11.3 mmol). The resulting mixture was cooled to 0°C and Pb(OAc)₄ (0.54 g, 1.21 mmol) was added. The reaction was warmed to 25°C with stirring for 45 min and then NaHCO₃ solution (sat., 30 ml) was added, and the mixture was stirred for 1 h. It was poured into a separatory funnel containing water (50 mL) and extracted with ether (3x40 mL). The ethereal solution was washed with brine (50 mL) and then dried over K₂CO₃. The crude product obtained by removal of solvent was dissolved in MeOH (25 mL) and K₂CO₃ solution (sat., 8 mL) was added. The reaction was stirred for 5 h and then the reaction mixture was poured into a separatory funnel containing 50 mL of water. It was then extracted with CH₂Cl₂ (3x30 mL). The combined extracts were dried over K₂CO₃ and the crude material obtained was purified by chromatography to give the product (160 mg) along with 21-methoxy by-product (40 mg). The product was further purified by recrystallization from 10% acetone in hexane to give 88 mg of the pure product (28%) as a white solid, m.p. 140-142°C.

### EXAMPLE 26

### a. 3α-Hydroxy-3β-methyl-5β-19-nor-pregn-(Z)17(20)-ene:

To a solution of 5β-19-nor-pregn-17(z)-en-3-one (600 mg, 2.1 mmol) in THF (20 mL) at -78°C was added methylmagnesium bromide (3 M in ether, 1 mL, 3 mmol). The reaction was warmed to 25°C. Ammonium chloride solution (50 mL) was added and the mixture was extracted with ether (3x30 mL). The product (577 mg, 91%) purified by chromatography (10% acetone in hexane) was obtained as a white waxy solid.

### b. 3α-Hydroxy-3β-methyl-5β-19-nor-pregnan-20-one:

To a solution of 3α-hydroxy-3β-methyl-5β-19-nor-17(z)-pregn-17(20)-ene (0.57 g, 1.9 mmol) in THF (30 mL) was added BH₃•THF solution (1 M in THF, 3.8 mL, 3.8 mmol) at 25°C. The reaction was completed in 30 min and NaOH solution (20%, 8 mL) was added very cautiously followed by a addition of hydrogen peroxide solution (30%, 8 mL). The mixture was stirred for 20 min and NH₄Cl solution was then added. The mixture was extracted with CH₂Cl₂ (3x30 mL) and the combined extracts were dried over K₂CO₃. Removal of the solvent left the crude product (760 mg) which was dissolved in CH₂Cl₂ (40 mL). 4-Methylmorpholine N-oxide (0.6 g, 4.75 mmol) and freshly ground molecular sieves (4Å, 5 g) were added and the resulting mixture was stirred for 20 min. The catalyst tetrabutylammonium perruthenate (100 mg) was added. The oxidation was complete within 40 min and the reaction mixture was filtered through a pad of Florisil which was rinsed with a 2:1 mixture of ether and CH₂Cl₂ (60 mL). The crude material (0.62 mg) obtained by the removal of the solvent was purified by chromatography (20% EtOAc in hexane) to give the product (53 mg, 9%) as a white solid.

### EXAMPLE 27

### 3β-Ethynyl-3α,20α-dihydroxy-5β-pregnane and 3β-ethynyl-3α, 20β-dihydroxy-5β-pregnane

To a solution of 3β-ethynyl-3α-hydroxy-sp-pregnan-20-one (0.31 g, 0.91 mmol) in methanol (20 mL) was added sodium borohydride (200 mg, 5.3 mmol) and it was stirred at 25°C for 1 h. Ammonium chloride (50 mL) solution was then added and the mixture was extracted with CH₂Cl₂ (3x30 mL). A chromatography (EtOAc:Hex=3:7) gave 3β-ethynyl-3α,20β-dihydroxy-5β-pregnane (200 mg, 65%) as major product: m.p., 221-223°C. The minor product, 3β-Ethynyl-3α,20α-dihydroxy-5β-pregnane was further purified by another chromatography (25-30% ethyl acetate in hexane, 16 mg, 5%): m.p., 187-188°C.

### EXAMPLE 28

### a. 3α,21-dihydroxy-3β-ethynyl-5β-pregnan-20-one

To a solution of 3α,21-dihydroxy-3β-ethynyl-5β-pregnan-20-one 21-acetate (725 mg, 1.81 mmol) in 45 mL of methanol at 0°C was added an aqueous 10% K₂CO₃ solution (3.75 mL). After stirring for 30 m at rt, the mixture was recooled to 0°C and a 2N aqueous HOAc solution (1.8 mL) was added. The reaction was added to an EtOAc/water mixture. The aqueous layer was extracted twice with EtOAc and the pooled organic layers were extracted with a brine solution, dried (Na₂SO₄) and concentrated. Purification by flash chromatography (20 cm of silica gel in a 4 cm dia. column, eluted with 2 liters of 20% acetone/hexane) gave 582 mg (90%) of the diol as a white solid, mp 155.5-157°C.

### b. 3β-Ethynyl-3α-hydroxy-21-methoxy-5β-pregan-20-one

A solution of 3β-ethynyl-3α-hydroxy-5β-pregan-20-one (503 mg, 1.47 mmol) in methanol (4 mL) and toluene (17 mL) was cooled in an ice/water bath and neat BF₃-Et₂O (2.9 mL, 3.35 g, 23.6 mmol) was added followed by 1.0 g (2.25 mmol) of solid lead tetraacetate. The mixture was allowed to warm to r.t. and then stirred for 80 min. The reaction was then added to 25 mL of an ice-cold sat. NaHCO₃ solution. The aqueous layer was extracted with EtOAc (2 x 10 mL) and the combined organic layers were extracted with a sat. NaCl solution, dried (Na₂SO₄) and concentrated. Two flash columns (silica gel, eluted with 2.5% acetone/CH₂Cl₂) afforded 148 mg (27%) of the title compound as a white solid, mp 198.5-199.5°C.

### EXAMPLE 29

### Preparation of (3-R)-20,20-ethylenedioxy-5α-pregnane-spiro-2'-oxirane

A mixture of trimethylsulfoxonium iodide (Aldrich; 8.79 g, 39.9 mmol) and solid potassium *tert*-butoxide (Aldrich, 95%; 4.3 g; 36.6 mmol) in 125 mL of dry THF was heated at reflux for 1.5 h. The reaction was allowed to cool to r.t. and 20,20-ethylenedioxy-5α-pregnan-3-one (12.0 g, 33.3 mmol) was added as a solid in one portion. The resulting mixture was stirred at r.t. for 20h. and then concentrated in vacuo. The residue was dissolved in CH₂Cl₂ and extracted with water. The aqueous layer was back extracted with CH₂Cl₂, and the combined organic layers were washed with a saturated NaCl solution, dried (MgSO₄) and concentrated. The white solid obtained (12.0 g, 96%, mp 160-165°C) was recrystallized from 60/40 acetone/methanol.

### EXAMPLE 30

### 3α,20-dihydroxy-3β,20-dimethyl-5α-pregnane

5α-Pregnan-3,20-dione (1.5 g, 4.7 mmol) was dissolved in 250 mL of dry benzene and 15 mL of 3M methylmagnesium bromide was added. The reaction mixture was stirred at room temperature for 30 min. The excess methyl magnesium bromide was decomposed by adding 100 mL of sulfuric acid dropwise at 0°C. The product solution was extracted with 250 mL of diethyl ether. The organic extract was combined, dried over magnesium sulfate, and evaporated to give a 60:40 mixture of 3β-methyl and 3α-methyl isomers. The product mixture was separated by flash chromatography on silica gel using a 70:30 (hexane:ethyl acetate and 1% methanol). elution to give 0.476 g (24% yield) of the desired product.

### EXAMPLE 31

### 3α,21-dihydroxy-3β-trifluoromethyl-5β-pregnan-20-one

A solution of 3α,21-dihydroxy-3β-trifluoromethyl-5β-pregnan-20-one, 21-acetate (1.36g, 3.06mmol) inMeOH (75mL) was cooled to 0°C. A solution of K₂CO₃ (10% aqueous, 6.45mL, 4.67mmol) was then added dropwise. After stirring for 1.5 hours at 0°C, a solution of acetic acid (2N aqueous, 2.5mL, 5.0mmol) was added dropwise and the mixture was allowed to warm to room temp. EtOAc, CH₂Cl₂ and water (100mL each) were added and thoroughly mixed. The organic phase was isolated, washed with aqueous NaHCO₃ and NaCl solutions, dried over MgSO₄ and evaporated *in vacuo*. The residue was purified by flash column chromatography (hexane/EtOAc 3:1) to yield a white solid (973mg, 79%). mp. 148-150°C.

### EXAMPLE 32

### a. 3α-Hydroxy-5α-21-ethyl-pregn-17(z)-ene

To a Wittig reagent prepared by stirring the mixture of *n-*butyltriphenylphosphonium bromide (3.9 g, 9.66 mmol) and potassium t-butoxide (1.1 g, 9.66 mmol) in THF (10 mL) for 30 min was added 3α-hydroxy-5α-androstan-17-one (0.7 g, 2.4 mmol) and the mixture was heated to reflux for 16 h. The reaction was then quenched with ammonium chloride solution and extracted with CH₂Cl₂. The pure product (230 mg, 29%) was obtained by chromatography (10% acetone in hexane).

### b. 3α-t-Butydimethysiloxy-5α-21-ethyl-pregn-17(z)-en

The mixture of t-butyldimethylsilylchloride (0.32 g, 2.1 mmol) imidazole (0.24 g, 3.5 mmol) and 3α-hydroxy-5α-21-ethyl-pregn-17(z)-ene (0.23 g 0.7 mmol) was stirred for 12 h at 25°C. Ammonium chloride solution was then added and extraction was done with ether (3x30 mL). The obtained extract was washed with brine (3x30 mL). The pure product (0.27 g, 87%) was then obtained by chromatography (Hexane).

### c. 3α,20α-Dihydroxy-5α-21-ethylpregnane

To a solution of 3α*-t*-butyldimethylsiloxy-5α-21-ethyl-pregn-17(Z)-ene (0.27 g, 0.6 mmol) in THF (20 mL) was added BH₃•THF (1M in THF, 1.2 mL) at 25°C. Then sodium hydroxide (20%, 5 mL) was added cautiously after the reaction was stirred for 1 h. Then hydrogen peroxide solution (30%, 5 mL) was added and the mixture was stirred for another hour. Ammonium chloride solution was then added and it was extracted with CH₂Cl₂. The 20α-hydroxy-3α-silyl ether compound was obtained by chromatography (5% acetone in hexane). 60 mg of this compound was desilylated using hydrofluoric acid in acetonitrile. Chromatography (20-30% ethyl acetate in hexane) gave 11 mg of the pure title compound (m.p., 229-232°C, Rf=0.28, EtOAc:Hex=3:7).

### EXAMPLE 33

### 3β-Fluoromethyl-3α-hydroxy-5α-pregnan-20-one

A mixture of *n-*BU₄NF•XH₂O (7.873 g) and benzene (50 mL) was refluxed with a Dean-Stark trap overnight. The mixture, which was not a clear solution, was then concentrated (normal pressure) to ∼10 mL and allowed to cool to room temperature. A solution of 3(*R*)-5α-pregnan-3-spiro-2'oxirane-20-one ethyleneketal (2.55 g, 6.81 mmol) in dry benzene (15 mL + 5 mL for the rinse) was added, using a double-ended needle, to the above concentrated solution. The resulting solution was concentrated, using a short-path distillation apparatus, to ∼10 mL and refluxed for 15 min. Since it was very difficult to spot the concentrated reaction solution on the TLC plate, dry benzene (5 mL) was added. TLC (100:1 CH₂Cl₂/acetone or 3:1 hexane/acetone) showed some unreacted starting material, besides two new less-polar spots. The reaction mixture was again concentrated and then refluxed for 30 min; TLC (after diluting the mixture with benzene) now showed almost complete consumption of the starting epoxide. As before, the mixture was concentrated and refluxed for a while. It must be noted here that this reaction takes place only when the mixture is highly concentrated. After the mixture had come to room temperature (giving a light-yellow solid), ether and water were added. Since all the solid did not dissolve, CH₂Cl₂ was added. The aqueous layer was back extracted with CH₂Cl₂. The combined organic extracts were washed with water (x2), dried (Na₂SO₄), filtered, and evaporated under reduced pressure to provide a white solid (3.33 g) whose ¹H NMR showed it to be a 4:1 mixture of 3β-fluoromethyl-3α-hydroxy-3α-pregnan-20-one ethyleneketal and 3α-fluoro-3β-hydroxymethyl-5α-pregnan-20-one ethylene ketal.

In order to hydrolyze the ketal, acetone (100 mL), water (5 mL), and *p-*TsOH•H₂O (143 mg, 0.752 mmol) were added to the above solid. The pH was adjusted by adding in HCl until slightly acidic. The mixture was heated with a heat gun for a while in order to get a clear solution, which was stirred at room temperature for 2 h. The mixture became turbid, so CH₂Cl₂ was added to obtain a clear solution. TLC indicated completion of the reaction. The solvent was removed under reduced pressure, giving a white solid to which were added CH₂Cl₂ and water. The aqueous layer was back-extracted with CH₂Cl₂. The combined organics were washed with saturated NaHCO₃, dried (MgSO₄), filtered, and evaporated under reduced pressure to furnish a white crystalline solid (2.5 g). Flash column chromatography of mecrude product with CH₂Cl₂ as eluent provided the desired 3β-fluoromethyl-3α-hydroxy-5α-pregnan-20-one (1.41g, 59%). mp 201-3°C.

### EXAMPLE 34

### 3α-hydroxy-3β-methyl-5α-pregn-17(20)-ene

To a solution of 5α-pregn-17(20)-en-3-one (207 mg, 0.689 mmol) in 8 mL of dry THF at -30°C (At -78°C, not all of the steroid dissolved) was added a 3M solution of MeMgBr in ether (Aldrich; 0.34 mL, 1.02 mmol) dropwise via syringe. After stirring at -30°C for 4.75 h, the reaction was added to a sat. NH₄Cl solution/ether mixture. The aqueous layer was extracted with ether (3x30 mL) and the organic layers were combined and washed with a brine solution, dried (MgSO₄) and concentrated at reduced pressure. The crude material was dissolved in CH₂Cl₂ and added to 32 cm of flash silica gel in a 2 cm column. The column was eluted with 100% CH2Cl2 until unreacted ketone (8.7 mg, 4%) had eluted. Elution with 100mL each of 2.5 and 5% acetone/CH2Cl2 gave 38 mg (18%) of 3α-hydroxy-3β-methyl-5α-pregn-17(20)-ene (R_{f} 0.35, 100% CH₂Cl₂) and 3β-hydroxy-3α-methyl-5α-pregn-17(20)-ene (R_{f} 0.16, 100% CH₂Cl₂).

### EXAMPLE 35

### 3α-hydroxy-17Z- and 17E-methoxymethylene-58-androstane

Solid 3α-hydroxy-5β-androstan-17-one (504 mg, 1.74 mmol) was added in portions to the ylide prepared from methoxymethyltriphenylphosphonium chloride (Aldrich; 1.484 g, 4.33 mmol) and potassium *tert*-butoxide (479 mg, 4.27 mmol). This mixture was refluxed for 7h and then cooled to 0°C and added to an ice-cold sat. NH₄Cl solution/ether mixture. The aqueous layer was extracted twice with ether and the combined organic layers were washed with a sat. NaCl solution, dried (MgSO₄) and concentrated. The crude alkene was dissolved in CH₂Cl₂ and added to 13 cm of flash silica in a 3.5 cm dia. column. The column was eluted with 400 mL of 5% acetone/CH₂Cl₂ and 200 mL of 7.5% acetone/CH₂Cl₂. The desired alkene was isolated as a 1:1 mixture of *Z*- and *E-*isomers (158 mg, 28%) along with recovered 17-one (220 mg, 44%). The mixture of alkene isomers was resubjected to flash chromatography (15 cm of silica in a 3.5 cm dia. column, eluted with 500 mL of 4% acetone/toluene and 200 mL each of 5,6, and 7% acetone/toluene) without good separation. Preparative HPLC (21.4 5 mm id x 25 cm Microsorb 5m column; solvent system 2.5% acetone/CH₂Cl₂, 10 mL/min; RI detection) allowed the separation of Z-isomer, mp 150-152°C, (more polar) and *E-*isomer, mp.151-153°C (less polar).

### EXAMPLE 36

### 3α,20β-Dihydroxy-30-methyl-5α-pregn and 3α 20α-Dihydroxy-3β-methyl-5α-pregnane

Reference: House, H. O.; Muller, H. C.; Pitt, C. G.; Wickam, P. P. *J*. *Org. Chem.* 1963, *28,* 2407.

To a mixture of freshly cut sodium (250 mg, 10.87 mmol) and boiling toluene (5 mL) were added dropwise over a period of 30 min a solution of 3α-hydroxy-3β-methyl-5αpregnan-20-one (340 mg, 1.022 mmol) in isopropyl alcohol (3 mL, 39.18 mmol) and toluene (4 mL + 2 mL for the rinse). The resulting mixture was refluxed for 2.5 h at which point TLC (3:1 hexane/acetone) indicated completion of the reaction and displayed two new spots that had lower R_{f}'s than the starting ketone and had approximately same intensity. The reaction solution was allowed to come to room temperature and then was cooled in an ice-bath. 1N HCl (10 mL) was added dropwise. After diluting the mixture with CH₂Cl, (∼50 mL), the aqueous layer was separated and back-extracted with CH₂Cl₂ (x2). The combined organics were washed with saturated NaHCO₃, dried (MgSO₄), filtered, and evaporated under reduced pressure to give a white solid (354 mg). Purification of these two products was accomplished by flash chromatography employing 15:1 and 10:1 hexane/acetone as eluant.

Evaporation of the early fractions provided 3α,20β-dihydroxy-3β-methyl-5α-pregnane (80 mg) as a white solid. ¹H NMR and capillary GC were employed to determine the purity.

Further elution provided a 9:1 mixture (on the basis of GC) of 3α,20α-dihydroxy-3β-methyl-5α-pregnane and 3α,20β-dihydroxy-3β-methyl-5α-pregnane (total 248 mg). This mixture was subjected to recrystallization from hot ethanol (∼10 mL); No crystals were obtained. Attempted next was hot 5:1 hexane/acetone (~50 mL), which led to white crystals (110 mg). According to GC, these crystals had a purity of only 96%. Finally, pure 3α,20α-dihydroxy-3β-methyl-5α-pregnane (80 mg) could be obtained by another recrystallization from hot ethanol.

### EXAMPLE 37

### 3α-Hydroxy-3β-iodomethyl-5α-pregan-20-one

To a solution of 3[R]-spiro-2'-oxirane-5α-pregnan-20-one (40 mg, 0.12 mmol) in acetone (3 mL) was added NaI (39 mg, 0.26). The resulting solution was stirred at room temperature for -4 h. TLC (3:1 hexane/acetone) indicated consumption of only a small amount of the starting material, with appearance of a new, slow-moving spot; the relative intensities of these two spots did not change with time, suggesting presence of an equilibrium between the starting material and the iodomethyl product (as alkoxide). Subsequently acetic acid (∼0.02 mL) was added to the above reaction solution. The mixture was stirred at room temperature overnight. TLC now showed complete disappearance of the starting epoxide. The reaction mixture contained some white crystals (sodium acetate). The solvent was removed under reduced pressure, and then ether and water were added. The aqueous phase was separated and back-extracted with ether. The combined ether solutions were successively washed with water and brine, dried (MgSO₄), filtered, and evaporated under reduced pressure to give 3α-hydroxy-3β-iodomethyl-5α-pregnan-20-one as white solid (51 mg, 92%).

### EXAMPLE 38

### a. 3,3-Ethylenedioxy-5β-pregn-17(20)-ene

5β-3-Ethylenedioxy-androstan-17-one (6 g) was added to Wittig reagent produced from ethyltriphenylphosphonium bromide (15 g) and potassium t-butoxide (4.5 g) in THF (15 mL). The reaction was refluxed for 2 h and cooled to 25°C. Then methylene chloride (80 mL) and a solution of ammonium chloride (60 mL) was added and the organic layer was separated by separatory funnel. The aqueous layer was extracted with methylene chloride (2x50 mL). The organic solution was dried over potassium carbonate and the solvent removed in vacuo. Most of the phosphoroxide was removed by washing with hexane. The obtained product (6 g) was dissolved in acetone (100 mL), and hydrochloric acid (2 N, 10 mL) added. The crude product (5.5 g) obtained by a basic work-up followed by methylene chloride extraction was purified by chromatography to produce 4.5 g of product (83%). The pure stereoisomer was obtained by repeated recrystalization from hexane.

### b. 3α-Hydroxy-3β-trifluoromethyl-5β-pregn-(Z)17(20)-ene

To a solution of 5β-pregn-(Z)-17(20)-en-3-one (950 mg, 3.17 mmol) in THF (15 mL) was added trifluoromethyltrimethylsilane (0.5 M solution in THF, 9.5 mL) at 0°C. The solution turned to brown gradually and the reaction was finished in 30 min. Water (30 mL) was added and organic layer collected. The aqueous layer was extracted with ether (3x50 mL) and organic solution was dried over potassium carbonate. The pure product (680 mg, 58%) was isolated by column chromatography using ethyl acetate and hexane (1:9) as eluent.

### EXAMPLE 39

### a. 3α,21-Dihydroxy-3β-ethynyl-5β-pregnan-20-one 21-acetate

A suspension of 3β-ethynyl-3α-hydroxy-5β-pregnan-20-one (1.00 g, 2.92 mmol) in 35 mL of toluene was treated with 2 mL of methanol. The resulting solution was cooled in an ice/water bath and neat BF₃-Et₂O (Aldrich; 5.8 mL, 5.02 g, 35.4 mmol) was added. Solid lead tetraacetate (Aldrich; 1.96 g, 4.42 mmol) was added in a few portions. A light purple solution formed initially and became light brown as stirring continued at 0°C. The mixture was stirred at r.t. for 3 h. and then recooled to 0°C. The cold reaction was added to a mixture of 52 mL of a sat. NaHCO₃ solution, water and crushed ice. The resulting mixture was extracted with EtOAc (2 x 75 mL). The combined organic layers were extracted with a sat. NaCl solution, dried (Na₂SO₄) and concentrated. The crude product was purified by column chromatography (25 cm of flash silica gel in a 5 cm dia. column eluted with 3 liters of 20% acetone/hexane) affording 749 mg (64%) of the acetate, mp 196-198°C.

### b. 3α,21-Dihydroxy-3β-ethynyl-5β-pregnan-20-one 21-hemisuccinate

To a stirred solution of 21-bromo-3β-ethynyl-3α-hydroxy-5β-pregnan-20-one (1.5g, 3.56 mmol) in 50 mL of acetone was added succinic acid (2.1g, 17.8 mmol) and triethylamine (3mL, 21.4 mmol). The reaction was heated to reflux for 3.5 h. then cooled to r.t. before partitioning between ethyl acetate and dilute aq. HCl. The organic layer was washed with sat. NaCl, dried with Na₂SO₄ and concen-trated *in vacuo* to give a crude solid. The solid was triturated with dichloromethane to remove succinic acid as a solid, and the liquid was concentrated *in vacuo* to give a foam (1.36g). Flash chromatography on 6 in. of silica gel in a 3cm column collecting 50 mL fractions and eluting with 1:3 -> 3:1 ethyl acetate : hexane with a gradual adjustment of the gradient to give 830mg (51%) of the title compound.

### c. Sodium 3α,21-dihydroxy-3β-ethynyl-5β-pregnan-20-one 21-hemisuccinate

The hemisuccinate, 633 mg, mp 62-68 °C, was dissolved in methanol and an aqueous solution of 116 mg (0.253 mmol) of NaHCO₃ was added. After stirring for 3.5 h, the solvent was removed under reduced pressure and the residue was triturated with an ether/hexane mixture. The light yellow solid obtained, weight 616 mg, was soluble in water at > 20 mg/mL.

### EXAMPLE 40

### 3α, 21-dihydroxy-3B-trifluoromethyl-5β-pregnan-20-one 21-acetate

Under an anhydrous argon atmosphere, 3α-hydroxy-3β-trifluoromethyl-5β-pregnan-20-one (1.94g, 5.02mmol) was dissolved in toluene (86mL) and MeOH (5.2mL). Boron trifluoride etherate (10.4mL, 84.3mmol) was added via syringe. Lead tetraacetate (2.89g, 6.51mmol) was then added. The mixture was stirred for 70min, poured into water and extracted three times with CH₂Cl₂. The organic phases were combined, washed with aqueous NaHCO₃ and NaCl solutions, dried over MgSO₄ and evaporated *in vacuo* to give a pale yellow solid (2.18g). This solid was purified by flash column chromatography (CH₂Cl₂/EtOAc 150:1 and hexane/EtOAc 4:1) to give a white solid (1.54g; 69%). mp. 167-168.5°C.

### EXAMPLE 41

### 3β-Chloromethyl-3α-hydroxy-5α-pregnan-20-one

A mixture of 3[R]-spiro-2'-oxirane-5α-pregnan-20-one (46 mg, 0.14 mmol), tetramethylammonium chloride (25 mg, 0.23 mmol) and dry DMF (Aldich; 5 mL) was heated at ∼130°C. A clear solution was obtained after some time. TLC (7:1 hexane/acetone) showed, besides the starting material, a new spot that moved slower than the starting epoxide. There was no further progress in the reaction after 2 h. More tetramethylammonium chloride (∼25 mg) was added to the reaction mixture. The resulting mixture was stirred at 130 - 150°C for 1 h. Not all the solid dissolved. There was no change in TLC. Presumably there is an equilibrium between the chloromethyl product (as alkoxide) and the starting epoxide. Acetic acid (0.1 mL, 1.75 mmol) was then added to the reaction mixture while the mixture was still very hot. All the solid particles that were present in the mixture now dissolved, and a clear solution resulted. This solution was heated at ∼100°C for 2 h at which point TLC indicated completion of the reaction, displaying only one spot. The solvent was removed by distillation under vacuum. To the resulting solid were added CH₂Cl₂ and water. The aqueous layer was back-extracted with CH₂Cl₂. The combined organics were dried (MgSO₄), filtered, and evaporated under reduced pressure to give a solid (46 mg). ¹H NMR of this solid showed only the desired regioisomer (i.e., 3β-chlororomethyl-3α-hydroxy-5α-pregnan-20-one). Finally, flash chromatography employing 9:1 hexane/acetone as eluant provided the title compound (35 mg, 68%) as a white solid. m.p.209-10°C (dec.).

### EXAMPLE 42

### a. 3β-(3'-Bromo-1-propynyl)-3α-hydroxy-5α-pregnan-20-one

A solution of propargyl bromide (80% soln. in toluene, 0.4 mL, 3.6 mmol) in dry THF (7 mL) was treated with n-BuLi (2.5M in THF, 3 mmol, 1.2 mL) at -78°. After stirring the mixture at this temp. for 10 min. a solution of 5β-pregnan-3,20-dione, 20-ketal (360 mg, 1 mmol) was added and the mixture was stirred at -78°C for 2 hr. It was then quenched with sat. NH₄Cl solution (1 mL). The solvent was removed and the residue was then dissolved in acetone (40 mL). After adding 2N HCl (10 mL) the solution was stirred at r.t. for 3.5 h. Sat. NaHCO₃ soln. was added to neutralize the acid. The solvents were removed and the residue was extracted with CH₂Cl₂. The organic layer was washed with water, dil. NaHCO₃ soln., water, and brine. After drying over anhyd. MgSO₄ the solution was filtered and evaporated to yield the crude product (360 mg). This crude product was then dissolved in a small amount of CH₂Cl₂ and poured on a column of silica gel. Elution with hexane:acetone mixture (90:10) gave 3α-(3'-bromo-1-propynyl)-3β-hydroxy-5β-pregnan-20-one (50 mg) as a first fraction. Further elution with the same solvent mixture yielded 3β-(3'-bromo-1-propynyl)-3α-hydroxy-5β-pregnan-20-one (181 mg).

### EXAMPLE 43

### 3β-Bromomethyl-3α-hydroxy-5α-pregnan-20-one

A mixture of 3[R]-spiro-2'-oxirane-5α-pregnan-20-one (362 mg, 1.10 mmol), tetramethylammonium bromide (322 mg, 2.16 mmol), acetic acid (1.0 mL, 17.5 mmol), and dry DMF (Aldich; 30 mL) was heated at ∼130°C. There was still some undissolved tetramethylammonium bromide present in the mixture, which was stirred at 110 - 120°C. TLC (3:1 hexane/acetone) after heating for 30 min at 110 -120°C showed, besides the starting material, a new, more-polar spot. There were also two other very light spots that moved slower than the major product spot; the intensity of these two very light spots increased with time. After 3 h at 110 -120°C, TLC displayed three spots of almost equal intensity, besides the spot corresponding to the unreacted starting material whose amount did not change with time. The solvent was removed by distillation under vacuum. To the resulting solid were added CH₂Cl₂, water, and brine (to prevent the emulsion formation). The organic layer was washed with saturated NaHCO₃, dried (MgSO₄), filtered, and evaporated under reduced pressure to give a solid (380 mg). ¹H NMR of this solid indicated presence of three products, namely, 3β-bromomethyl-3α-hydroxy-5α-pregnan-20-one, 3-hydroxymethyl-5α-pregn-2-en-20-one, and 3β-(acetoxymethyl)-3α-hydroxy-5α-pregnan-20-one and the unreacted starting material. Finally, flash chromatography employing 9:1 hexane/acetone as eluent provided pure 3β-bromomethyl-3α-hydroxy-5α-pregnan-20-one (98 mg, 22%) as a white solid. m.p. 196- 97°C (dec.).

### EXAMPLE 44

### a. 3α-Hydroxy-19-nor-5α-17-methoxymethleneandrostane

To a solution of 19-nor-5α-17-methoxymethleneandrostan-3-one (200 mg, 0.65 mmol) in THF (20 mL) was added K-selectride (1 M in THF) by a syringe at -78°C and the reaction was stirred at this temperature for 4.5 h. Then mixture was poured into a separatory funnel containing ammonium chloride solution (saturated, 20 mL) and methylene chloride (40 mL). The aqueous layer was extracted with methylene chloride (2x15 mL). The organic solution was dried over potassium carbonate and the solvent removed in vacuo. A column chromatography (4% acetone, 4% methylene chloride in hexane) resulted 130 mg product (64%).

### b. 3α-Hydroxy-19-nor-5α-androstan-17β-carboxaldehyde

To a solution of 3α-hydroxy-19-nor-5α-17-methoxymethleneandrostane (130 mg, 0.43 mmol) in THF (10 mL) was added a solution of hydrochloric acid (1N, 1 mL) at 25°C and the reaction was stirred for 6 h. Then sodium bicarbonate solution (NaHCO₃, saturated, 20 mL) and methylene chloride (30 mL) were added. The organic layer was separated and aqueous layer extracted with methylene chloride (2x20 mL). The organic solution was dried over potassium carbonate and the solvent removed in vacuo. The pure product (80 mg, 65%) was isolated by column chromatography using acetone and hexane (1:4) as eluent.

### EXAMPLE 45

### 3α,21-Dihydroxy-3β-methyl-5α-pregana-20-one 21-Disodium Phosphate.

To a solution of 21-bromo-3α-hydroxy-3β-methyl-5α-pregnan-20-one(1.0 g, 2.43 mmol) in 10 mL THF at RT was added dibenzyl hydrogen phosphate(2.1 g, 7.3 mmol) and triethylamine (1.085 mL, 7.53 mmol) with stirring. The reaction was then heated to reflux for 4.5 h and then cooled to RT. Dichloromethane(25 mL) was then added and the solution transferred to a separatory funnel, washed with 1N HCl, sat. aq. NaHCO₃, dried with MgSO₄ and concentrated in vacuo to give the dibenzylphosphate as a crude oil (1.205 g) The dibenzylphosphate (790 mg, 1.3 mmol) was dissolved in 2 : 1 EtOH : THF (30 mL) with a few drops of sulfuric acid, charged with 5% Pd/C (180 mg, 20% by weight) and subjected to 50 psi of H₂ at rt until the reaction was complete by TLC. The catalyst was removed by filtration and the filtrate was concentrated. The residue was dissolved in 4 : 1 MeOH : water (10 mL) and titrated to pH 11 with 1M NaOH. (The solution was cloudy) The solution was treated with acetone until a readily filterable solid precipitated after which the mixture was cooled to 0°C and filtered to isolate a crude solid 260 mg. The solid was dissolved in 20 mL water forming a cloudy solution that was filtered and then concentrated to give 220 mg of the title compound as a white solid.

### EXAMPLE 46

### a. 3α,21-dihydroxy-3β-trifluoromethyl-5β-pregnan-20-one 21-hemisuccinate

Succinic acid (20 g, 170 mmol) and triethylamine (19 mL, 140 mmol) were added to a solution of crude 21-bromo-3α-hydroxy-3β-trifluoromethyl-5β-pregnan-20-one (5.36 g; ≤ 10.9 mmol) in acetone (200 mL). The mixture was refluxed for 1.75 hr. The residue was poured into ice water (350 mL). More water (100 mL) was then added. The mixture was stirred and then extracted three times with CH₂Cl₂ (200 mL, 200 mL and 100 mL). The combined organic phases were dried over MgSO₄ and evaporated *in vacuo* to give a semi-solid residue (5.71 g) which was purified by flash column chromatography (CH₂Cl_{2/}MeOH; gradient 90:1 to 20:1) to give a white solid (3.63 g, 66% over 2 steps). mp: dec. (over broad range).

### b. 3α,21-dihydroxy-3β-trifluoromethyl-5β-pregnan-20-one. 21-hemisuccinate, sodium salt

3α,21-dihydroxy-3β-trifluoromethyl-5β-pregnan-20-one, 21-hemisuccinate (859mg, 1.71mmol) was dissolved in MeOH (35mL). NaHCO₃ (143mg, 1.70mmol) in water (10mL) was then added dropwise. More MeOH (10mL) was added. After stirring for 2.5 hours, the solvent was evaporated. Toluene was added and evaporated *in vacuo*. The residue was treated with ether/hexane and then with hexane to give a white solid. MeOH was added and removed. Heptane was added and removed *in vacuo*. The residue was treated with hexane and dried *in vacuo* to give a white solid (878mg, 98%).

### EXAMPLE 47

### a. 21-(N,N-dimethylamino)methyl-3α-hydroxy-3β-methyl-5α-pregnan-20-one

A solution of 3α-hydroxy-3β-methyl-5α-pregnan-20-one (3.32 g, 10 mmol) and N,N-dimethylmethyleneammonium chloride (1.6 g, 17 mmol) in acetonitrile (15 mL) was refluxed for 2.5 hr. After cooling the solvent was removed and the residue was made basic by adding sat. NaHCO₃ soln. The mixture was then extracted with CH₂Cl₂, washed with brine, dried over anhyd. MgSO₄, and evaporated to yield the crude product. This product was crystallized from acetone to give 21-(N,N-dimethylamino)methyl-3α-hydroxy-3β-methyl-5α-pregnan-20-one as colorless rods (550 mg).

The mother liquor was evaporated to dryness and the residue was chromatographed over silica gel. Elution with hexane:acetone (2:3, 1:4) afforded 21-(N,N-dimethylamino) methyl-3α-hydroxy-3β-methyl-21-methylene-5α-pregnan-20-one as a colorless solid (200 mg). Further elution with acetone yielded more 21-(N,N-dimethylamino)methyl-3α-hydroxy-3β-methyl-5α-pregnan-20-one as colorless solid (1 g).

### b. 3α-hydroxy-3β-methyl-21-methylene-5α-pregnan-20-one

A mixture of 21-(N,N-dimethylamino)methyl-3α-hydroxy-3β-methyl-5α-pregnan-20-one (500 mg) and MeI (4 mL) was stirred at r.t. for 1.5 hr. 4 mL of CH₂Cl₂ was added and the stirring was continued for another 15 hr. MeI and CH₂Cl₂ were removed and the residue was taken up in CH₂Cl₂ (20 mL) and was treated with sat. NaHCO3 solution (15 mL). After stirring at r.t. for 2.5 hr the organic layer was separated, washed with brine, dried over anhyd. MgSO₄, and evaporated. The residue (500 mg) was chromatographed over silica gel. Elution with CH₂Cl₂:acetone (95:5) gave 3α-hydroxy-3β-methyl-21-methylene-5α-pregnan-20-one (300 mg).

### c. 3α-hydroxy-3β,21-dimethyl-5α-pregnan-20-one

A solution of 3α-hydroxy-3β-methyl-21-methylene-5α-pregnan-20-one (250 mg) in EtOAc (30 mL) was hydrogenated (2.5 atm of H₂) in the presence of Pd/C (27 mg, 5%) at r.t. for 1 hr. After filtration and then removal of the solvent gave the title compound as colorless solid (198 mg); mp 190-192°C.

### EXAMPLE 48

### a. 21-(N,N-dimethylamino)methyl-3α-hydroxy-5β-pregnan-20-one

A solution of 3α-hydroxy-5β-pregnan-20-one (318 mg, 1 mmol) and N,N-dimethylmethyleneammonium chloride (100 mg, 1.1 mmol) in acetonitrile (3 mL) was refluxed for 2 hr. After cooling, the solvent was removed and the residue was made acidic by adding 5 mL of 2N HCl. The mixture was then extracted with EtOAc. The aqueous layer was separated and neutralized with 2N NaOH. The precipitated solid was extracted in CH₂Cl₂. The organic layer was separated, washed with brine, dried over anhyd. MgSO₄, and evaporated to yield the crude 21-(N,N-dimethylamino)methyl-3α-hydroxy-5β-pregnan-20-one as a colorless solid (200 mg), which was used as such for the next step.

### b. 3α-hydroxy-21-methylene-5β-pregnan-20-one and 3α-hydroxy-21-methoxymethyl-5β-pregnan-20-one

A mixture of 21-(N,N-dimethylamino)methyl-3α-hydroxy-5β-pregnan-20-one (550 mg), CH₂Cl₂ (2 mL), and MeI (4 mL) was stirred at r.t. for 1 hr. MeI and CH₂Cl₂ were removed and the residue was refluxed in MeOH for 72 hr. After cooling, the mixture was treated with ice-water and extracted with EtOAc. The organic layer was separated, washed with brine, dried over anhyd. MgSO₄, and evaporated. The residue (500 mg) was chromatographed over silica gel. Elution with CH₂Cl₂:acetone (95:5) gave 3α-hydroxy-21-methylene-5β-pregnan-20-one (150 mg). Further elution with the same solvent afforded 3α-hydroxy-21-methoxymethyl-5β-pregnan-20-one as a colorless solid (120 mg).

### EXAMPLE 49

### a. 3α,21-dihydroxy-3β-trifluoromethyl-5β-pregnan-20-one 21-phosphate disodium salt

3α,21-dihydroxy-3β-trifluoromethyl-5β-pregnan-20-one 21-dibenzylphosphate (712 mg, 1.07 mmol) and palladium on activated carbon (5% Pd, 180 mg) in ethanol (20 mL) were put under a 50 psi atmosphere of hydrogen (on the Parr apparatus) for 2 hours. The palladium on carbon was removed by filtration and the filtrate was evaporated giving an oil. This oil was dissolved in methanol/water 4:1 (10mL). 1.00 M NaOH (1.97 mL) was added dropwise to bring the pH to 11 (± 1). A small amount of solid was removed by filtration and most of the solvent from the filtrate was removed by evaporation. The remainder (mostly water) was removed by freeze-drying to give a white solid (494 mg, 88%). mp: 224° C dec. ¹H NMR available.

### b. 3α,21-dihydroxy-3β-trifluoromethyl-5β-pregnan-20-one 21-dibenzylphosphate

3α-dihydroxy-3β-trifluoromethyl-21-bromo-5β-pregnan-20- one (1.20 g, 2.58 mmol), dibenzylphosphate (2.15 g, 7.74 mmol) and triethylamine (1.08 mL, 7.74 mmol) were stirred in THF (10 mL) and then heated to reflux for 3 hours. After cooling to room temp., EtOAc and 0.5 M HCl were added. The organic phase was isolated and washed with aqueous NaHCO₃ and NaCl, dried over MgSO₄ and evaporated *in vacuo* to give a brown oil. This oil was purified by flash column chromatography (hexane/acetone 4:1) to give a clear oil (712 mg, 42%).

### c. 3α-hydroxy-3β-trifluoromethyl-21-bromo-5β-pregnan-20-one

Hydrobromic acid (48%, 2 drops) was added to a stirred solution of 3α-hydroxy-3β-trifluoromethyl-5β-pregnan-20-one (4.20 g, 10.9 mmol) in methanol (100 mL). A solution of bromine (2.17 g, 13.6 mmol) in methanol (60 mL) was then added dropwise over a period of 10 min. After stirring for 30 min., solid NaHCO₃ was added (until all hydrobromic acid was neutralized) and the solvent was evaporated. The residue was partitioned between CH₂Cl₂ and water. The organic phase was isolated, washed with brine, dried over MgSO₄ and evaporated *in vacuo* to give a yellow semi-solid residue (5.36 g) which was used without further purification.

### d. 3α-hydroxy-3β-trifluoromethyl-5β-pregnan-20-one

### (Reference: Krishnamurti, R.; Bellew, D.R.: Surya Prakash, G.K. J. Org. Chem. 56:984 (1991))

To a solution of 5β-pregnan-3,20-dione 20-ethyleneketal (60 mg, 0.166 mmol) in dry THF (3 mL) was added 0.5 M F₃CSi(CH₃)₃ in THF (0.5 mL, 0.25 mmol). The resulting colorless solution was cooled to 0 °C., and η-Bu₄NFxH₂O (few crystals) was added. The cooling bath was removed, and the mixture was allowed to warm to r.t. Unlike the same reaction involving 5α-pregnan-3,20-dione 20-ethyleneketal, the above reaction mixture did not turn yellow and there was no gas generation. More 0.5 M F₃CSi(CH₃)₃ in THF (0.5 mL, 0.25 mmol) was added. The resulting mixture was stirred at room temperature for a few minutes. TLC (3:1 hexane/acetone) displayed a new spot with an R_{f} close to 1, but there was still some unreacted starting material present. Therefore, more 0.5 M F₃CSi(CH₃)₃ in THF (0.5 mL, 0.25 mmol) was added. The mixture was again stirred at room temperature for a while. No unreacted starting material was found. 1N HCl (∼3 mL) was added, and the resulting two-phase mixture was stirred at room temperature overnight. The spot that was formed as a result of trifluoromethylation had now completely disappeared, and there were two new, less polar spots, the lower one being the major product. The mixture was then diluted with ether and water. The aqueous layer was separated and extracted with ether. The combined organice layers were washed with saturated NaHCO₃ and brine, dried (MgSO₄), filtered, and evaporated under reduced pressure to give a white crystalline (foamy) solid. The two epimers were separated by flash chromatography with 15:1 hexane:acetone. Evaporation of the early fractions gave the minor isomer. Further elution of the column gave 50 mg of the title compound.

### EXAMPLE 50

### a. 3β-Trifluoromethyl-3α-hydroxy-21-bromo-5β-19-nor-pregnan-20-one

To a flask containing a solution of 3α-hydroxy-3β-trifluoromethyl-5β-19-nor-pregnan-20-one (0.65 g, 1.75 mmol) in methanol (10 mL) was added a solution of bromine (0.15 mL) in methanol (10 mL) dropwise in such a rate to maintain the brown color the bromine until this color was persistent. Then water (50 mL) was added the mixture was extracted with CH₂Cl₂ (3x40mL). The combined extracts were dried over K₂CO₃. Removal of the solvent resulted in the product as a foamy white solid (0.805 g).

### b. 3β-Trifluoromethyl-3α-hydroxy-5β-19-nor-preanan-20-one-21-dibenzylphosphate

To a solution of 3β-trifluoromethyl-3α-hydroxy-21-bromo-5β-19-nor-pregnan-20-one (0.805 g) in THF (10 mL) was added dibenzylphosphate (1.48 g, 5.32 mmol) and triethylamine (538 mg, 5.32 mmol) and the mixture was heated to reflux for 1.5 h. HCl (0.5 N, 40 mL) was added and it was extracted with CH₂Cl₂ (3x40 mL). The combined extracts were dried over Na₂SO₄. Removal of the solvent resulted in the crude material which was purified by chromatography to give 733 mg product (65%).

### c. 3α-Hydroxy-3β-trifluoromethyl-5β-19-nor-preagan-20-one-21-phosphate, disodium salt

A mixture of 3β-trifluoromethyl-3α-hydroxy-5β-19-nor-pregnan-20-one-21-dibenzylphosphate (733 mg) and Pd/C (5%, 200 mg) in ethanol (20 mL) was attached to a Parr hydrogenator. The hydrogenolysis was done within 30 min. under about 50 psi and the resulting mixture was filtered through #5 filter paper to remove the catalyst. A white foamy solid (494 mg) was obtained after the solvent was removed. This solid was dissolved in methanol (10 mL) and a solution of NaHCO₃ (190 mg) in water (8 mL) was added at 0° C. The methanol was then removed *in vacuo* and the resulting aqueous solution was freeze-dried to give the product (530 mg, 92%).

### EXAMPLE 51

### 3α-Hydroxy-21-(pyrid-4-ylthio)-5β-pregnan-20-one.

To a solution of 21-bromo-3α-hydroxy-5β-pregnan-20-one(2. 8 g, 7.05 mmol) and 4-mercaptopyridine(940 mg, 8.46 mmol) in 30 mL dry THF at 25° C was added triethylamine (1 mL) dropwise and the mixture heated to 60° C and stirred for 1.5 h. The solution was then partitioned between ethylacetate and 1:1 sat. NaHCO₃ and water. The organic layer was then washed with sat. NaCl, dried with MgSO₄ and concentrated *in vacuo* to a crude solid weighing 3.19g. Flash chromatography on 6 in. of silica gel in a 5.5 cm column collecting 50 mL fractions and eluting with 10%-15% acetone:CH₂Cl₂ resulted in 2.246g(75%) of the title compound as a yellow solid. MP 192-4°C.

### EXAMPLE 52

### a. 3β-Ethynyl-3α-hydroxy-5α-pregnan-20-one, 20-ketal

A 250 mL three neck flask equipped with a gas inlet, a thermometer and a condenser was charged with lithium acetylide-EDA complex (2.75 g, 90%, 27.5 mmol). Dry benzene (60 mL) was added and acetylene gas was bubbled through the mixture at a moderate rate. The mixture was then heated to 50-55° C in an oil bath and treated in parts with 5α-pregnan-3,20-dione, 20-ketal (9 g, 25 mmol). The stirring was continued at this temp. for 5 h. and then at r.t. for another 17 h. The resulting suspension was cooled to 10° C and was treated with sat. NaCl soln. (5 mL). The solvent was removed and water was added to the residue. The water insoluble product was collected by filtration, washed with water, and dried under vacuum. This crude product was then crystallized from EtOAc to yield 3α-ethynyl-3β-hydroxy-5α-pregnan-20-one, 20-ketal (3.35 g). The mother liquor was evaporated to dryness and the residue was purified by column chromatography over silica gel. Elution with toluene:acetone mixture (92:8) gave the unreacted starting ketone (1.3 g) followed by 3β-ethynyl-3α-hydroxy-5α-pregnan-20-one, 20-ketal (1.3 g) as a second fraction. Further elution with the same solvent mixture yielded the more polar 3α-ethynyl-3β-hydroxy-5α-pregnan-20-one, 20-ketal (270 mg).

### b. 3β-Ethynyl-3α-hydroxy-5α-pregnan-20-one

3β-Ethynyl-3α-hydroxy-5α-pregnan-20-one, 20-ketal (550 mg) was dissolved in a mixture of acetone (20 mL) and 2N HCl (10 mL) and the mixture was stirred at r.t. for 15 h. The solvents were removed and the residue was extracted with CH₂Cl₂. The organic layer was washed with water, dil. NaHCO₃ soln., water, and brine. After drying over anhyd. MgSO₄ the solution was filtered and evaporated to yield the crude product (414 mg). This crude product was then dissolved in a small amount of CH₂Cl₂ and poured on a column of silica gel. Elution with toluene:acetone mixture (92:8) gave the title compound (280 mg), mp 175-177°C.

### c. 3α-ethynyl-3β-nitrooxy-5α-pregnan-20-one

A solution of 3α-ethynyl-3β-hydroxy-5α-pregnan-20-one, 20-ketal (2.15 g) in CHCl₃ (45 mL) was cooled to -20° C and treated with acetic anhydride (20 mL). Fuming nitric acid (4 mL) was then added and the mixture was stirred at this temp. for 45 min. After warming to -5° C, the yellow solution was poured into a mixture of 2N NaOH (70 mL) and water (150 mL) to yield the resultant solution of pH 3-4, which was then extracted with CHCl₃, washed with water, sat. NaHCO₃ solution, brine, dried (MgSO₄) and evaporated to yield the title compound as a viscous material (3 g), which was used as such for the next step.

### d. 3β-Ethynyl-3α-hydroxy-5α-pregnan-20-one

Crude 3α-ehtynyl-3β-nitrooxy-5α-pregnan-20-one (3 g) from the above step was taken in a mixture of THF and water (30 mL, 1:1). AgNO₃ (516 mg) was added. After stirring at r.t. for 15 h. the solvents were removed and the residue was extracted with CH₂Cl₂. The organic layer was washed with water, dil. NaHCO₃ soln., water, and brine. After drying over anhyd. MgSO₄, the solution was filtered and evaporated to yield the crude product (2 g). This crude product was then dissolved in a small amount of CH₂Cl₂ and poured on a column of silica gel. Elution with toluene:acetone mixture (93:7) gave 3β-ethynyl-3α-hydroxy-5α-pregnan-20-one (550 mg) as a first fraction.

### EXAMPLE 53

### a. Preparation of the lithium reagent from 1,2-dibromoethylene

A 250 mL three neck flask equipped with a N₂ gas bubbler, a thermometer, and a dropping funnel was charged with 1,2-dibromoethylene (cis/trans mixture, 98%, Aldrich, 2.1 mL, 26 mmol, mw = 186, d = 2.246). Dry THF (40 mL) was added and the solution was cooled to -78°C in a dry ice-acetone bath. n-BuLi (2.5M in THF, 20 mL, 50 mmol) was added dropwise over a period of 35 min. The mixture was stirred at this temperature for 40 min. and the resulting reagent was used immediately for the next step.

### b. 3β-Ethynyl-3α-hydroxy-5α-pregnan-20-one 20-ketal

The above solution of the reagent in THF, which was maintained at -78°C, was treated dropwise with a solution of 5α-pregnan-3,20-dione, 20-ketal (4.68g, 13 mmol) in THF (50 mL) over a period of 1 hr. The temperature was maintained below -70°C during the addition. The stirring was continued at this temperature for 15 min. (100% conversion as detected by TLC). The cooling bath was removed and the resulting solution was quenched with 2N HCl (pH 6-7). The solvent was removed and the residue was extracted with chloroform, the organic layer was separated, washed with water, and dried over anhydrous MgSO4. Removal of the solvent gave the title product (3.9 g) as an epimeric mixture with the corresponding 3β-hydroxy epimer (85:15).

### EXAMPLE 54

### 3α-Hydroxy-3β-trifluoromethyl-5β-19-nor-pregnan-20-one

To a solution of 3α-hydroxy-3β-trifluoromethyl-5β-19-nor-pregn-17(20)-ene (2.6 g, 7.3 mmol) in THF (80 mL),was added diborane THF complex (1M solution in THF, 22 mL) dropwise at 25° C. The reaction was complete in 1 h. and then a solution of sodium hydroxide (20%, 50 mL) was added very slowly at 0° C followed by an addition of hydrogen peroxide (30%, 30 mL). Water was then added and the THF layer was separated by separatory funnel. The aqueous layer was extracted with methylene chloride (2x40 mL). The organic solution was dried over potassium carbonate and the solvent removed *in vacuo*. A quick column (hex:acetone=1:1) gave 1.8 g of product which was subjected to PCC oxidation (PCC, 2.1 g, 9.6 mmol; sodium acetate, 0.8 g, 9.6 mmol). The pure product (700 mg, 26%) was purified by column chromatography using ethyl acetate and hexane (15:85) as eluent; m.p. 151.5-153.0° C.

### EXAMPLE 55

### a. Synthesis of benzyl phenyl sulfoxide

To a solution of benzyl phenyl sulfide (Aldrich; 1.068 g, 5.33 mmol) in 25 mL of CH₂Cl₂ at -78° C was slowly added a solution of m-chloroperbenzoic acid (Aldrich, 50-60%; 760 mg, 2.64 mmol if 60%) in 10 mL of CH₂Cl₂. After warming to room temperature and stirring overnight, the solution was added to 20 mL of a saturated NaHCO₃ solution. The aqueous layer was separated and extracted with CH₂Cl, (2 x 10 mL). The pooled organic layers were then dried (MgSO₄) and concentrated. The residue was subjected to flash column chromatography (silica gel, 10% acetone/hexane and 15% acetone/hexane) affording the sulfoxide (632 mg, 55%) as a white solid, mp 123-126° C.

### b. 20, 20-ethylenedioxy-3α-hydroxy-3β-[[2-(phenylsulfinyl)-2-phenyl]ethyl]-5α-pregnane

A solution of diisopropylamine (Aldrich, freshly distilled from CaH₂; 0.5 mL, 361 mg, 3.57 mmol) in 2 mL of dry THF was cooled to -10° C and treated with a 1.6 M solution of *n-*BuLi in hexanes (Aldrich; 1.0 mL, 1.6 mmol) added dropwise via syringe. After 10 min., the reaction was cooled to -75° C and a solution of benzyl phenyl sulfoxide (347 mg, 1.60 mmol) in 5 mL of dry THF was added dropwise via syringe over 30 min. To the resulting deep yellow solution was added 297 mg (0.79 mmol) of solid 20,20-ethylenedioxy-3(*R*)-5α-pregnan-3-spiro-2'-oxirane. The reaction was allowed to warm to r.t. and then warmed to 50°C. After 5 h, the reaction was allowed to cool to r.t. and added to 30 mL of ice-cold water. The resulting mixture was extracted with EtOAc (3 x 20 mL). The combined EtOAc layers were back extracted with a sat. NaCl solution, dried (Na₂SO₄) and concentrated. The residue was purified by flash chromatography (silica gel, gradient from 100% CH₂Cl₂ to 20% acetone/CH₂Cl₂) affording the sulfoxide (405 mg, 86%) as a mixture of two diastereomers. This mixture was carried on to the elimination step.

### c. 3α-hydroxy-3β-[2-(E)-phenletheny]-5α-pregnan-20-one

A suspension of the sulfoxide (200 mg, 0.339 mmol) in 1.5 mL of *p*-isopropyltoluene containing 0.2 mL of 2,4,6-collidine (distilled from CaH₂) was heated in an oil bath at 135° C for 60 min. After cooling to r.t., the solution was allowed to stir overnight. A white precipitate formed and was isolated and washed with *p-*isopropyltoluene (3 x 1mL). By ¹H NMR and TLC, the precipitate (69 mg, 44%) was the desired 20,20-ethylenedioxy-3α-hydroxy-2-(*E*)-phenylethenyl-5α-pregnane. A solution of the ketal in acetone at 0° C was treated with a 1M HCl solution and stirred cold for one hour. The reaction was poured into an EtOAc/water mixture and the organic layer was washed with water and a sat. NaCl solution. After drying (Na₂SO₄) the solvent was removed *in vacuo* and the residue was purified by flash chromatography (silica gel, eluted with 1% acetone/CH₂Cl₂).

### EXAMPLE 56

### 3α-hydroxy-3β-(2'-propenyl)-5β-pregan-2-one

A solution of 5β-pregnan-3,20-dione, 20-ketal (180 mg, 0.5 mmol) in dry THF (20 mL) was treated with allyl magnesium bromide (2M in THF, 1 mmol, 0.5 mL) at -70° C. After stirring the mixture at this temp. for 5 min. and then at r.t. for 1.5 h, it was quenched with 2N HCl (1 mL). The solvent was removed and the residue was dissolved in acetone (15 mL). After adding Dowex-40 resin (2 g) the solution was stirred at r.t. for 20 min. The resin was filtered off and the solvent was removed. The residue was extracted with CH₂Cl₂. The organic layer was washed with water, dil. NaHCO₃ soln., water, and brine. After drying over anhyd. MgSO₄, the solution was filtered and evaporated to yield the crude product (250 mg). This crude product was then dissolved in a small amount of CH₂Cl₂ and poured on a column of silica gel. Elution with toluene:acetone mixture (95:5) gave 3α-(2'-propenyl)-3β-hydroxy-5β-pregnan-20-one (45 mg) as a first fraction. Further elution with the same solvent mixture yielded 3β-(2'-propenyl)-3α-hydroxy-5β-pregnan-20-one (85 mg), m.p. 119-120° C.

### EXAMPLE 57

### a. 3α-Hydroxy-17b-ethynyl-5α-androstane

3α-*tert*-butyldimethylsilyloxy-17b-ethynyl-5α-androstane (100 mg, 0.24 mmol) was dissolved in acetonitrile/THF (30 mL, 2:1 v/v) and cooled to 0° C. Aqueous hydrofluoric acid (48%, 2 mL, 60 mmol) was added dropwise and the reaction solution was allowed to warm to room temperature. After 2 hours, the reaction solution was poured into water and extracted with CH₂Cl₂. The organic phase was washed with aqueous sodium bicarbonate, dried over MgSO₄ and evaporated *in vacuo* to give a white solid (69 mg). This solid was purified by flash column chromatography (hexane/acetone 12:1) to give a white solid (59 mg, 82%); mp: 158 - 160° C.

### b. 3α-tert-butyldimethylsilyloxy-17b-ethynyl-5α-androstane

Under a dry argon atmosphere, diisopropylamine (125 mL, 0.95 mmol, distilled from CaH₂) was dissolved in THF (2 mL) and the solution was cooled to 0° C. *n*-Butyllithium (2.5 M in hexane, 380 mL, 0.95 mmol) was added dropwise. After stirring at this temperature for 5 min., the solution was cooled to -78° C. 3α-*tert*-butyldimethylsilyloxy-5α-pregnan- 20-one 20-enol diethylphosphate (164 mg, 0.288 mmol) was dissolved in THF (1mL) and this solution was added dropwise to the previously prepared solution of lithium diisopropylamide (still at -78° C). The reaction mixture was allowed to warm to room temperature over 2.5 hours. Water (1 mL) was added, then the mixture was diluted with 75 mL ether. The organic phase was washed with 1 M HCl, water and aqueous sodium bicarbonate, dried over MgSO₄ and evaporated *in vacuo* to give a semi-solid residue (100 mg, 83%) which was used without further purification.

### c. 3α-tert-butyldimethylsilyloxy-5α-pregnan-20-one 20-enoldiethylphosphate

Under a dry argon atmosphere, lithium bis(trimethylsilyl) amide (1.0 M solution in THF, 1.02 mL, 1.02 mmol) was diluted with THF (1 mL). The orange solution was cooled to -78° C. 3α-*tert*-butyldimethylsilyloxy-5α-pregnan-20-one (400 mg, 0.92 mmol) was dissolved in THF (1 mL) and added dropwise to the previously prepared solution of lithium bis(trimethylsilyl) amide (still at -78° C). After stirring at this temperature for 45 min., diethylchlorophosphate (140 mL, 0.97 mmol) was added dropwise and the resulting solution was allowed to warm to room temperature. Water (1 mL) was added and then ether. The organic phase was washed with 1N HCl, water and aqueous sodium bicarbonate, dried over MgSO₄ and evaporated *in vacuo* to give a yellow oil (394 mg) which was purified by flash column chromatography (hexane/acetone 10:1) to give a clear oil (164 mg, 31%).

### d. 3α-tert-butyldimethylsilyloxy-5α-pregnan-20-one

Under a dry argon atmosphere, 3α-hydroxy-5α-pregnan-20-one (1.1 g, 3.5 mmol) was dissolved in anhydrous DMF (17 mL). Imidazole (0.94 g, 13.8 mmol) was added and the mixture was stirred until all dissolved. *tert*-Butyldimethylsilylchloride (1.04 g, 6.91 mmol) was then added and the reaction mixture was stirred overnight. Ether (approx. 50 mL) was added and the organic phase was washed with 1N HCl, water and aqueous sodium bicarbonate, dried over MgSO₄ and evaporated *in vacuo* to give a white solid (1.93 g). This solid was purified by flash column chromatography (100% CH₂Cl₂) to give a white solid (1.16 g, 78%).

### EXAMPLE 58

### 3α,21-hydroxy-3β-methyl-5α-pregnan-20-one, 21-mesylate

To a solution of 3α,21-hydroxy-3β-methyl-5α-pregnan-20-one (77 mg, 0.22 mmol) in pyridine (1 mL) at 0° C was added 29 mL (42 mg, 0.37 mmol) of methanesulfonyl chloride. After stirring cold for 2h. and 40 min., the reaction was poured into water and the resulting precipitate was collected. Flash chromatography (6" of silica in a 20 mm dia. column, eluted with a gradient from 0 to 5% EtOAc/hexane) gave 54 mg (57%) of the mesylate as a white solid.

### EXAMPLE 59

### 21-Bromo-3β-ethynyl-3α-hydroxy-5β-pregnan-20-one

To a stirred solution of 3β-ethynyl-3α-hydroxy-5β-pregnan-20-one(2.5 g, 7.3 mmol) in 125 mL methanol at 0° C was added a few drops of HBr followed by slow dropwise addition of a solution of bromine (1.28g, 8.0 mmol) in 25 mL of methanol. The reaction was slowly warmed to 25° C, and upon complete decolorization the solution was added to ice water containing a small amount of NaHCO₃. The precipitate was filtered, dissolved in dichloromethane, dried with MgSO₄ and concentrated *in vacuo* to yield a crude foam. (2.837g) Flash chromatography on 6 in. of silica gel in a 5.5 cm column collecting 50 mL fractions and eluting with 1% acetone : CH₂Cl₂ resulted in 1.96g (64%) of the title compound as a foam.

### EXAMPLE 60

### 3β-Chloroethynyl-3α-hydroxy-5β-pregnan-20-one

A solution of cis-1,2-dichloroethylene (194 mg, 0.16 mL, 2 mmol) in dry THF (7 mL) was treated under N₂ with n-BuLi (2.5M in THF, 4 mmol, 1.4 mL) at -10° C. The mixture was stirred at -30° C for 20 min and then at -5° C for 10 min. It was recooled to -30° C and a solution of 5β-pregnan-3,20-dione, 20-ketal (360 mg, 1 mmol) in dry THF (10 mL) was added dropwise over a period of 10 min. The cooling bath was removed and the mixture was stirred at room temperature for 0.5 hr. NH₄Cl solution (3 mL) was added to quench the reaction. The solvent was removed and the residue was then dissolved in acetone (25 mL). After adding 2N HCl (10 mL) the solution was stirred at r.t. for 1 h. Sat. NaHCO₃ soln. was added to neutralize the acid. The solvents were removed and the residue was extracted with CH₂Cl₂. The organic layer was washed with water and then brine. After drying over anhyd. MgSO₄, the solution was filtered and evaporated to yield the crude product (427 mg). This crude product was then dissolved in a small amount of CH₂Cl₂ and poured on a column of silica gel. Elution with toluene:acetone mixture (95:5) gave the title compound as a colorless solid (130 mg).

### EXAMPLE 61

### 3α-hydroxy-3β-methyl-5α-pregnan-20-one

To a solution (light yellow) of the (3R)-5α-pregan-3-spiro-2'-oxirane-20-one (101 mg, 0.305 mmol) and NaI (115 mg, 0.767 mmol) in anhydrous 1,2-dimethoxyethane (DME) (5mL) at room temperature was added η-Bu₃SnH (0.22 mL, 0.238 g, 0.818 mmol). The reaction solution became colorless. Azobisisobutylnitrile (AIBN) (10 mg, 0.061 mmol) was then added. The resulting solution was refluxed under a nitrogen atmosphere for 21 h. at which point TLC (3:1 hexane/acetone) indicated completion of the reaction. The reaction was quenched with methanol; the mixture was stirred at room temperature for a while. The solvent was removed in vacuo to give an oil which did not dissolve in either. Addition of CH₂Cl₂ gave a solution which was washed with water, 1N HCl, and saturated NaHCO₃. The organic layer was dried (MgSO₄), filtered, and concentrated under reduced pressure to a white solid. Purification by gradient flash chromatography (hexane, 7:1 hexane/acetone, 5:1 hexane/acetone) furnished the titled compound (93mg, 92%).

It will be obvious to one skilled in the art that the above described compounds may be present as mixtures of diastereomers which may be separated into individual diastereomers. Resolution of the diastereomers may be conveniently accomplished by gas or liquid chromatography or isolation from natural sources. All above examples beyond the scope of the claims are for reference only.

Where isomers are separated, the desired pharmacological activity will often predominate in one of the diastereomers. As disclosed herein, the biological activity of these compounds display a high degree of stereospecificity.

TBPS binding assays were conducted according to the following procedures, which are described in Gee, K.W.; Lawrence, L.J.; and Yamamura, H. I., "Modulation of the Chloride Ionophore by Benzodiazepine Receptor Ligands Influence of Gamma-Aminobutyric Acid and Ligand Efficacy," Molecular Pharmacology, 30:218, 1986. Male Sprague-Dawley rats (200-250g) were anesthetized with carbon dioxide and decapitated. The rats' brains were removed and the cerebral cortices were dissected and homogenized in 10 volumes of 0.32 M sucrose. Tissue was centrifuged at 1000 x g for 10 minutes and the resultant supernatant was centrifuged at 9,000 g for 20 minutes. The resultant pellet was resuspended in 10 volumes of 200 mM NaCl/50 mM Na-K phosphate pH 7.4 buffer (binding buffer) and then centrifuged at 9,000 x g for 10 minutes. This washing procedure was repeated twice and the final P2 pellet was resuspended in the binding buffer. For the binding assay, [³⁵S] TBPS (2 nM) was co-incubated in binding buffer with P2 membranes and 5 µM GABA and either 1) no additions to measure total binding; 2) 2 µM unlabeled TPBS to measure nonspecific binding; 3) the neurosteroid ranging in concentration from 1 Nm to 10µM. Following a 90 minute incubation at 22°C, the suspension was filtered and the bound radioactivity was determined by liquid scintillation counting. The IC₅₀, which is the concentration of the neurosteroid producing a 50% inhibition of TBPS binding, was calculate using the non-linear curve fitting routine on Excel^{™} Solver by Microsoft^{™}. A lower IC₅₀ indicates higher affinity for the steroid site on the GABA_{A} receptor-chloride channel complex. Table 1 gives the IC₅₀ values of representative steroids used in the present invention.

**TABLE 1**

| Neuroactive Steroid | IC₅₀ nM) |
|---|---|
| 3β-(4'-Acetylphenyl)ethynyl-3α-hydroxy-5α-pregnan-20-one | 5 |
| 3β-(4'-Carboxyphenyl)ethynyl-3α-hydroxy-5β-pregnan-20-one ethyl ester | 8 |
| 3β-(4'-Acetylphenyl)ethynyl-3α-hydroxy-5β-pregnan-20-one | 14 |
| 3α-hydroxy-21-(pyrid-4-ylthio)-5α-pregnan-20-one | 23 |
| 3α-hydroxy-5β-pregnan-20-one | 25 |
| 3β-(chloroethynyl)-3α-hydroxy-5β-pregnan-20-one | 26 |
| 3α-Hydroxy-3β-methyl-5β-19-nor-pregnan-20-one | 26 |
| 3α,20a-Dihydroxy-21-ethyl-5α-pregnane | 27 |
| 3β-(4'-Dimethylaminophenyl)ethynyl-5β-pregnan-20-one | 28 |
| 3β-azidomethyl-3α-hydroxy-5α-pregnan-20-one | 34 |
| 3α,20a-Dihydroxy-21-methyl-5α-pregnane | 35 |
| 3α-hydroxy-5α-pregnan-20-one | 37 |
| 3β-ethynyl-3α-hydroxy-5β-pregnan-20-one | 39 |
| 3α-hydroxy-30-methoxymethyl-5β-pregnan-20-one | 40 |
| 3β-(4'-biphenyl)ethynyl-3α-hydroxy-5β-pregnan-20-one | 43 |
| 3β-Ethynyl-3α-hydroxy-19-nor-5β-pregnan-20-one | 44 |
| 3β-(1-Hexynyl)-3α-hydroxy-5β-pregnan-20-one | 44 |
| 3β-ethenyl-3α-hydroxy-5β-pregnan-20-one | 46 |
| 3α-Hydroxy-30-(41-nitrophenyl)ethynyl-5β-pregnan-20-one | 46 |
| 3α-Hydroxy-3β-(4'-methyoxyphenyl) ethynyl-5β-pregnan-20-one | 47 |
| 3β-(4'-Trifluoromethylphenyl)ethynyl-3α-hydroxy-5β-pregnan-20-one | 52 |
| 3α-hydroxy-3β-ethynyl-5β-pregnan-20-one | 53 |
| 3α-Hydroxy-3β(E)-(2-phenylethenyl)-5α-pregnan-20-one | 53 |
| 3α-Hydroxy-3β-(2'-propynyl)-5α-pregnan-20-one | 56 |
| 3β-(4'-Chlorophenyl)ethynyl-3α-hydroxy-5β-pregnan-20-one | 58 |
| 3α-Hydroxy-21-(pyrid-4-ylthio)-5β-pregnan-20-one | 59 |
| 3α-hydroxy-3β-methyl-5α-pregnan-20-one | 62 |
| 3β-(1-Octynyl)-3α-hydroxy-5β-pregnan-20-one | 63 |
| 3β-(1-Heptynyl)-3α-hydroxy-5β-pregnan-20-one | 65 |
| 3α-hydroxy-3β-methoxymethyl-5α-pregnan-20-one | 67 |
| 3α-hydroxy-17b-cyano-5α-androstane | 67 |
| 3β-(4'-cyanophenyl)ethynyl-3α-hydroxy-5β-pregnan-20-one | 73 |
| 3α,21-dihydroxy-5α-pregnan-20-one 21-acetate | 75 |
| 3α-Hydroxy-3β-(2'-propenyl)-5β-pregnan-20-one | 75 |
| 3α-hydroxy-21-methyl-5β-pregnan-20-one | 78 |
| 3α-Hydroxy-3β-(pentafluorophenyl) ethynyl-5β-pregnan-20-one | 79 |
| 3α-Hydroxy-3β-trifluoromethyl-19-nor-5β-pregnan-20-one | 80⊛ |
| 3α-Hydroxy-3β-(4'-methylphenyl) ethynyl-5β-pregnan-20-one | 88 |
| 3α-hydroxy-3β,21-dimethyl-5α-pregnan-20-one | 89 |
| 3α-Hydroxy-3β-(6-oxo-1-heptynyl)-5β-pregnan-20-one | 90 |
| 3α-hydroxy-3β-methyl-5α-19-nor-pregnan-20-) one | 91 |
| 3α-hydroxy-3β-chloromethyl-5α-pregnan-20-one | 93 |
| 3β-(3'-Bromo-1-propynyl)-3α-hydroxy-5β-pregnan-20-one | 94 |
| 3α-hydroxy-21-methoxymethyl-5β-pregnan-20-one | 96 |
| 3α,20a(S)-dihydroxy-5α-pregnane | 97 |
| 3α-Hydroxy-3β-(phenylethynyl)-5β-pregnan-20-one | 98 |
| 3β-Henzyl-3α-hydroxy-5β-pregnan-20-one | 109 |
| 3β-(2,4-Difluorophenyl)ethynyl-3α-hydroxy-5β-pregnan-20-one | 109 |
| 3α,21-dihydroxy-5α-pregnan-20-one 21-hemisuccinate | 117 |
| 3α,21-dihydroxy-5α-pregnan-20-one 21-acetate | 119 |
| 3α-Hydroxy-3β-(2'-phenylethyl)-5β-pregnan-20-one | 135 |
| 3β-ethyl-3α-hydroxy-5β-pregnan-20-one | 136 |
| 3α-Hydroxy-3β-(3'-hydroxypropynyl)-5β-pregnan-20-one | 137 |
| 3α-Hydroxy-3β-[3'(RS)-hydroxybutynyl]-5α-pregnan-20-one | 140 |
| 3α,20-dihydroxy-20-methyl-5α-pregnane | 151 |
| 3α-Hydroxy-3β-[4'(R/S)-hydroxypentynyl]-5β-pregnan-20-one 4'(R/S)-hemisuccinate sodium salt | 153 |
| 3β-(Ethoxymethyl)-3α-hydroxy-5α-pregnan-20-one | 154 |
| 3α-Hydroxy-3β-methyl-5β-19-nor-pregn-17 (Z)-ene | 154 |
| 3β-(5'-Cyanopentynyl)-3α-hydroxy-5β-pregnan-20-one | 158 |
| 3α,20a-dihydroxy-30-methyl-5α-pregnane | 166 |
| 3β-(4'-Acetoxyacetylphenyl)ethynyl-3α-hydroxy-5β-pregnan-20-one | 171 |
| 20,20-ethylenedioxy-3α-hydroxy-5α-pregnan-20-one | 176 |
| 3α-Hydroxy-3β-[4'(R/S)-hydroxypentynyl]-5α-pregnan-20-one | 178 |
| 3α,21-dihydroxy-50-pregnan-20-one (5β-THDOC) | 180 |
| 3α-hydroxy-5β-pregnan-cis(Z)-17(20)-ene | 180 |
| 3β-(Bromomethyl)-3α-hydroxy-5α-pregnan-20-one | 190 |
| 3β-(2'-Hydroxyphenyl)ethynyl-3α-hydroxy-5β-pregnan-20-one | 192 |
| 3α-hydroxy-3β-fluoromethyl-5α-pregnan-20-one | 200 |
| 3α,20b-dihydroxy-5β-pregnane | 206 |
| 3α-Hydroxy-5β-19-nor-pregn-17(z) ene | 209 |
| 3α,21-dihydroxy-3β-methyl-5α-pregnan-20-one 21-Hemisuccinate, sodium salt | 211 |
| 3α,21-Dihydroxy-3β-ethynyl-5α-pregnan-20-one | 216 |
| 3α-hydroxy-21-methyl-5β-pregnan-17(20)(Z)-ene | 217 |
| 3α,21-dihydroxy-5β-pregnan-20-one 21-hemisuccinate | 224 |
| 3α-hydroxy-30-(2'-methoxyphenyl) ethynyl-5β-pregnan-20-one | 238 |
| 3α,21-dihydroxy-3β-methyl-5α-pregnan-20-one 21-Hemisuccinate | 241 |
| 3β-(But-3-enyl)-3α-hydroxy-5α-pregnan-20-one | 242 |
| 3α,21-dihydroxy-5α-pregnan-20-one 21-hemisuccinate, sodium salt | 251 |
| 3α-Hydroxy-3β-trifluoromethyl-19-nor-5α-pregnan-20-one | 251⊛ |
| 3α, 21-Dihydroxy-3β-ethynyl-5β-pregnan-20-one, 21-acetate | 251 |
| 3α-hydroxy-21-methoxy-3β-trifluoromethyl-5β-19-nor-pregnan-20-one | 252 |
| 3α-Hydroxy-5α-androstane | 252 |
| 3α-Hydroxy-17b-ethynyl-5α-androstane | 254 |
| 3α, 21-dihydroxy-3β-methyl-5α-pregnan-20-one 21-mesylate | 255 |
| 3α, 20-dihydroxy-3β, 20-dimethyl-5α-pregnane | 264 |
| 3α-hydroxy-3β-ethyl-5α-pregnan-20-one | 265 |
| 3α, 21-Dihydroxy-3β-trifluoromethyl-19-nor-5β-pregnan-20-one | 265⊛ |
| 3β-[(3',4'-Dimethoxyphenyl) ethynyl] -3α-hydroxy-5/3-pregnan-20-one | 283 |
| 3α, 20a-dihydroxy-5β-pregnane | 292 |
| 3β-(Buta-2,3-dienyl)-3α-hydroxy-5α-pregnan-20-one | 347 |
| 3α-hydroxy-17(Z)-methoxymethylene-19-nor-5α-androstane | 350 |
| 3α-hydroxy-3β-methyl-5α-19-nor-pregn-(Z)-17(20)-ene | 364 |
| 3α,21-Dihydroxy-3β-trifluoromethyl-5β-pregnan-20-one, 21-acetate | 370⊛ |
| 3α-hydroxy-19-nor-5α-pregnan-cis-17(20)-ene | 373 |
| 3α-hydroxy-3β-phenyl-5β-pregnan-20-one | 382 |
| 3β-(3'-Hydroxyphenyl)ethynyl-3α-hydroxy-5β-pregnan-20-one | 407 |
| 3β-cyanomethyl-3α-hydroxy-5α-pregnan-20-one | 439 |
| 3α-hydroxy-17b-hydroxymethyl-5α-androstane- | 455 |
| 3β-Ethynyl-3α-hydroxy-5β-19-nor-pregn-17(z)-ene | 460 |
| 3α-Hydroxy-3β-trifluoromethyl-5β-19-nor-pregn-17(20)-ene | 482 |

| | |
|---|---|
| All entries marked with ⊛ in the above table are embodiments within the scope of the claims. All other entries are for reference only. | |

The pharmaceutical compositions of this invention are prepared in conventional dosage unit forms by incorporating an active compound of the invention, or a mixture of such compounds, with a nontoxic pharmaceutical carrier in nontoxic amounts sufficient to produce the desired pharmacodynamic activity in a subject, animal or human. Preferably, the composition contains the active ingredient in an active, but nontoxic amount, selected from 5 mg to 250 mg of active ingredient per dosage unit. The medicaments of this invention prevent insomnia and produce sleep.

The pharmaceutical carrier employed may be, for example, solids, liquids, or time release substances (see, e.g., Remington's Pharmaceutical Sciences, 14th Edition, 1970). Representative solid carriers are lactose, terra alba, sucrose, talc, gelatin, agar, pectin, acacia, magnesium stearate, stearic acid, microcrystalline cellulose, polymer hydrogels, and the like. Typical liquid carriers are propylene glycol, aqueous solutions of β-cyclodextrins, syrup, peanut oil, olive oil and like emulsions. Similarly, the carrier or diluent may include any time-delay material well known to the art, such as glycerol monostearate or glycerol distearate alone or with wax, microcapsules, microspheres, liposomes, or hydrogels.

A wide variety of pharmaceutical forms can be employed. Thus, when using a solid carrier, the preparation can be plain milled, micronized, in oil, tableted, placed in a hard gelatin or enteric-coated capsule in micronized powder or pellet form, or in the form of a troche, lozenge, or suppository. When the compounds of the invention are administered in the form of suppositories for rectal administration, the compounds may be mixed in material such as cocoa butter and polyethylene glycols or other suitable non-irritating materials solid at room temperature, but liquid at rectal temperature. When using a liquid carrier, the preparation can be in the form of a liquid, such as an ampule, or an aqueous or nonaqueous liquid suspension. Liquid dosage forms will generally also contain pharmaceutically acceptable preservatives and the like. In addition, parental administration, nasal spray, sublingual and buccal administration, and timed release skin patches are also suitable pharmaceutical forms for topical administration of the compounds of the invention.

The formulations are to be administered orally. The inventors have discovered that specific formulations can significantly alter the activity of the neuroactive steroid, which tends to be insoluble in water and therefore poorly absorbed after oral administration. For example, it was thought that pregnanolone was orally inactive. L. Gyermek, "Pregnanolone: a Highly Potent, Naturally Occurring Hypnotic-Anesthetic Agent" Proc. Soc. Exp. Biol. Med. 125:1058-1062, 1967.

The inventors have discovered various formulations of pregnanolone which the body can absorb through the gastrointestinal tract in sufficient quantities to induce sleep after oral administration. The bioavailability of a drug is quantified by measuring serum blood levels before and after administration. This method of quantification is described by Robert H. Purdy et al. in "Radioimmunoassay of 3α-hydroxy-5α-pregnan-20-one in rat and human plasma" Steroids 55:290-296 (1990). Generally, after a blood sample is drawn, the serum is separated and then frozen. The serum is chromatographed to remove competing materials including endogenous steroids. Pregnanolone has a retention time of 19.0 minutes when it is chromatographed using HPLC techniques on an 8 cm Radial-Pak-5-µm silica column (Waters Associates) using the solvent system 0.2% ethanol (95%) in dichloromethane at a flow rate of 3 ml/min. Then, a radioimmunoassay is run to quantify the serum blood levels of the chromatographed material.

The following represents unclaimed matter.

To demonstrate that pregnanolone, when administered orally in certain formulations, is orally active to induce sleep in humans, the inventors conducted a trial using 8 healthy male volunteers between the ages of 18 and 35. The study was an unblinded, single dose crossover study in which subjects were sequestered prior to and immediately following drug administration.

Baseline medical history, laboratory values, drug and alcohol screens were done for each subject. Throughout the study the subjects remained in the clinical facility from Day-1 until 48 hours post drug administration. While in the clinic, the ingestion of caffeine, alcohol, or other drugs was prohibited to the subjects.

Following an overnight fast, a defined high fat breakfast was served to each subject and consumed within 15 minutes. Starting at 0900 GMT the subjects were administered pregnanolone according to the randomization defined in the protocol. Pregnanolone (500 mg; granulated powder) was administered orally in one of four formulations. Formulation 1 had the following ingredients: pregnanolone (micronized, obtained from AKZO, Arnheim, The Netherlands) (5%), polyvinylpovidone (polyvinylpyrrolidone or PDP) (Luviskol K-30, from BASF, Germany) (94%), and sodium lauryl sulfate (SLS) (from Nomeco, Copenhagen, Denmark) (1%). Formulation 2 contained preganolone (12.3%), and β-cyclodextrin (7 moles of H₂O per 1 mole of cyclodextrin; from Sigma, St. Louis) (87.7%). Formulation 3 contained pregnanolone (99%; nanosized), and sodium lauryl sulfate (1%). Formulation 4 contained pregnanolone (99%), and sodium lauryl sulfate (1%). The pregnanolone used in the study was uniformly micronized (available from AKZO) in all cases with the exception of Formulation 3 which was nanosized (submicron particle size). Access to water ad libitum was allowed except during the hour immediately following drug administration. Meals were standardized in time throughout the study.

Formulation 1 was prepared by dissolving all constituents in ethanol. The mixture was stirred and heated until most of the ethanol had evaporated. The product was dried to completion in vacuum for 24 hours and ground in a mortar.

Formulation 2 was prepared by shaking 1.23 g. of pregnanolone, 6.77 g. cyclodextrin in 50 ml. of water for hours at 37°C. The complex was filtered, dried under vacuum for 24 hours, and ground in a mortar.

Formulation 3 was prepared by dissolving the constituents in carbon dioxide at an elevated temperature and high pressure (up to 700 bar). The pressure is released and the particles precipitate, which produced some submicron particles.

Formulation 4 was prepared by dry blending the micronized pregnanolone with sodium lauryl sulphate, followed by sieving.

Serum samples for analysis of pregnanolone level were obtained prior to and immediately following drug administration and continued for 24 hours. Serum was separated, frozen, and stored until analyzed by radioimmunoassay.

Samples obtained during the study were analyzed for pregnanolone using radioimmunoassays and the results analyzed using appropriate non-compartmental pharmacokinetic parameters. Relative bioavailability assessments were made between formulations based on observed serum concentration of pregnanolone.

Evaluation of clinical response to drug administration was obtained through observations carried out by the clinical facility's staff and trained observers. Observations regarding onset of and apparent depth of sleep were recorded over the course of the four hour treatment period and later correlated with serum pregnanolone concentrations. Duration of sleep was not evaluated as subjects were frequently disturbed in the process of obtaining vital signs and blood samples.

Each individual received the four formulations separated by drug-free wash-out days during the three week trial. No serious or unexpected adverse effects were noted for any of the treatment groups. Body temperature was closely monitored and recorded prior to drug administration at 0.5, 1.0, 1.5, 2.5, 3, 4, 8, and 12 hours post drug administration. No decrement in body temperature was observed in any of the subjects during the course of the study with any of the formulations.

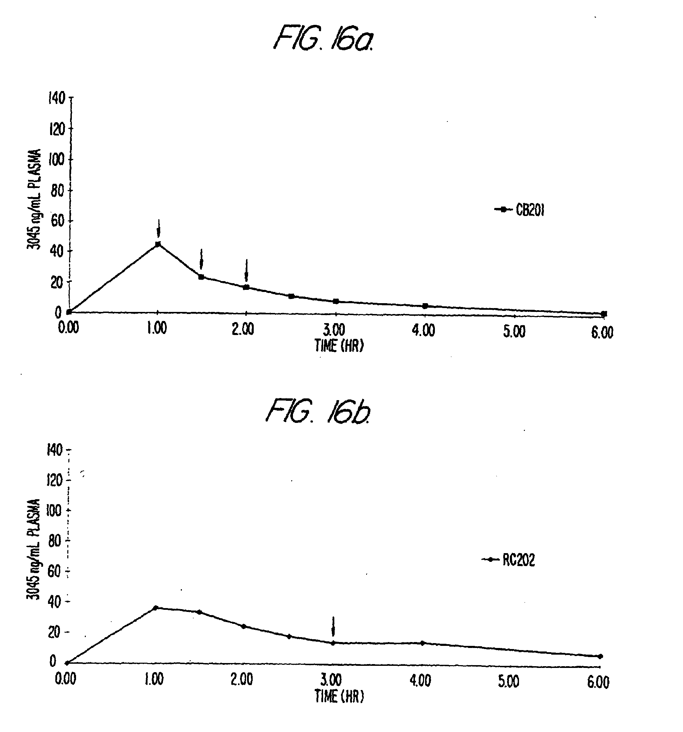

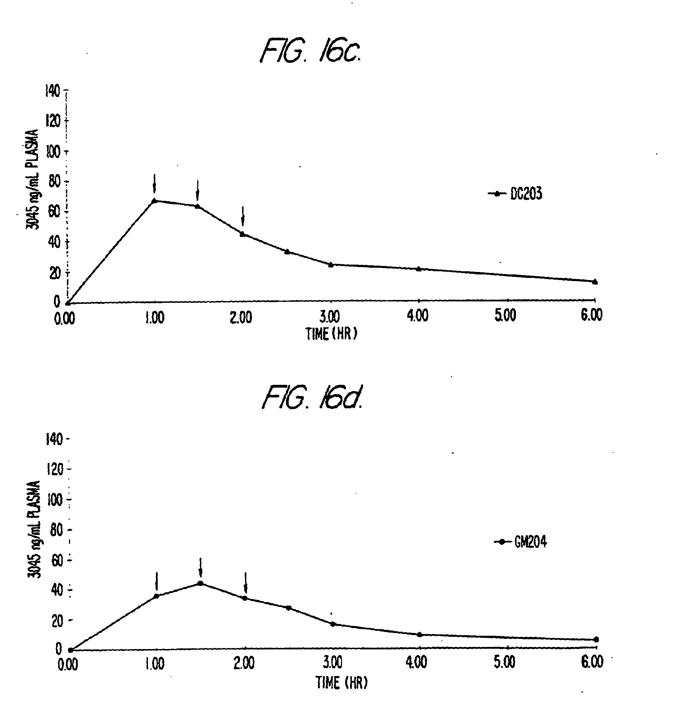

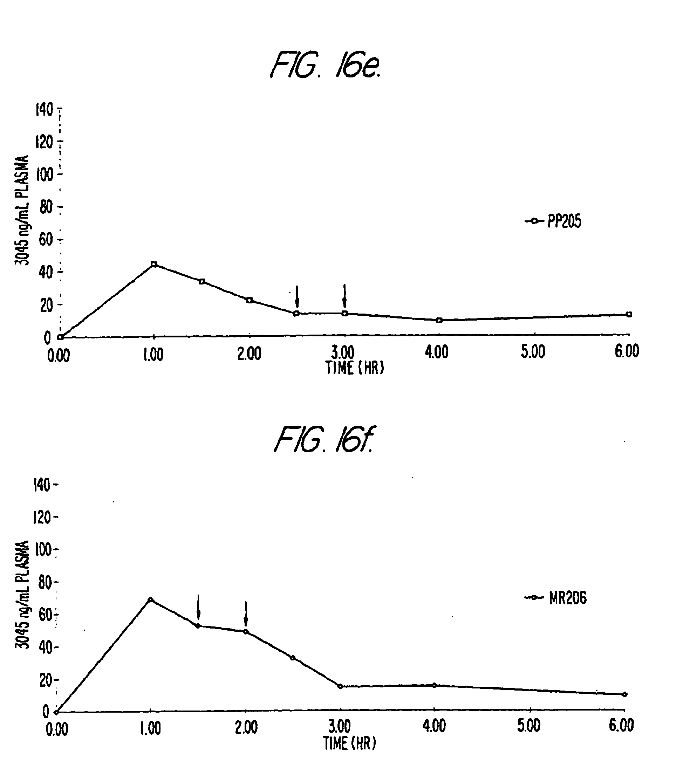

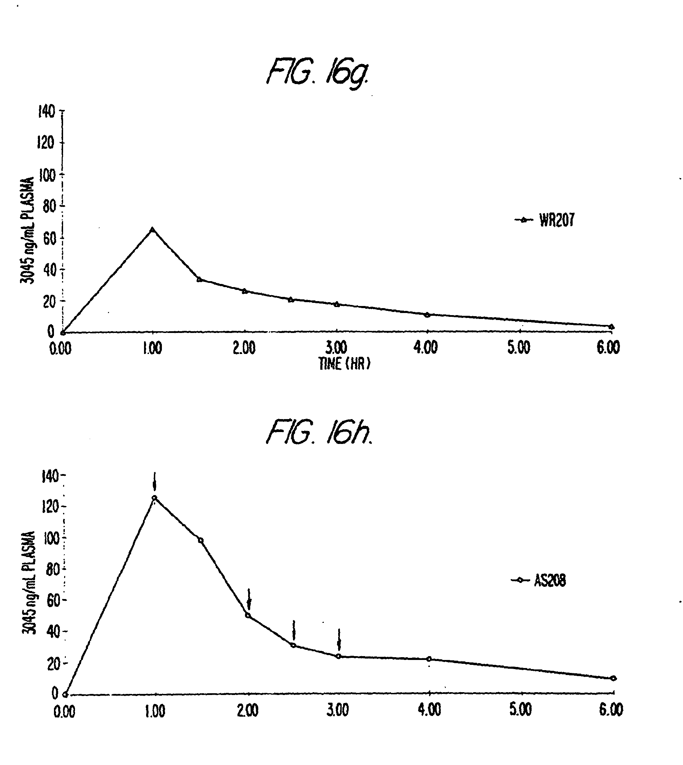

After approximately one hour signs of drowsiness and/or sleep was observed. Among the volunteers receiving pregnanolone in β-cyclodextrin (Formulation 2), seven of eight subjects were observed to sleep with time of onset ranging from <1 hour to 3 hours post drug administration. Duration of sleep was impossible to evaluate as subjects were awakened every 30 minutes for blood draws and vital signs. Subjective evaluations indicated depth of sleep ranging from light to difficult to arouse. Results are summarized in Figures 16A-16H where arrows mark points at which observations of sleep were recorded. Only one subject was observed to remain awake throughout the study period after receiving pregnanolone formulated in β-cyclodextrin. Figures 16A-16H also show the plasma blood levels for this formulation. Peak plasma levels for the 8 volunteers ranged from about 40 to 120 nanograms per ml. Table 2 shows that the mean peak concentration for this formulation was 61.7 nanograms per ml.

**Table 2**

| Relative Absorption of Four CCD 3045 Formulations | | |
|---|---|---|
| CCD 3045 Formulation | AUC (ng.h/mL) | Peak Concentration (ng/mL) |
| PVP Formulation | 136.2 (61.1)* | 60.9 (28.7)* |
| Beta CD Formulation | 136.4 (53.8) | 61.7 (28.5) |
| Nanosized Powder +1% SLS | 59.8 (23.8) | 22.0 (9.9) |
| Micronized Powder +1% SLS | 117.1 (36.6) | 59.4 (21.0) |
| *Mean (SD) | | |

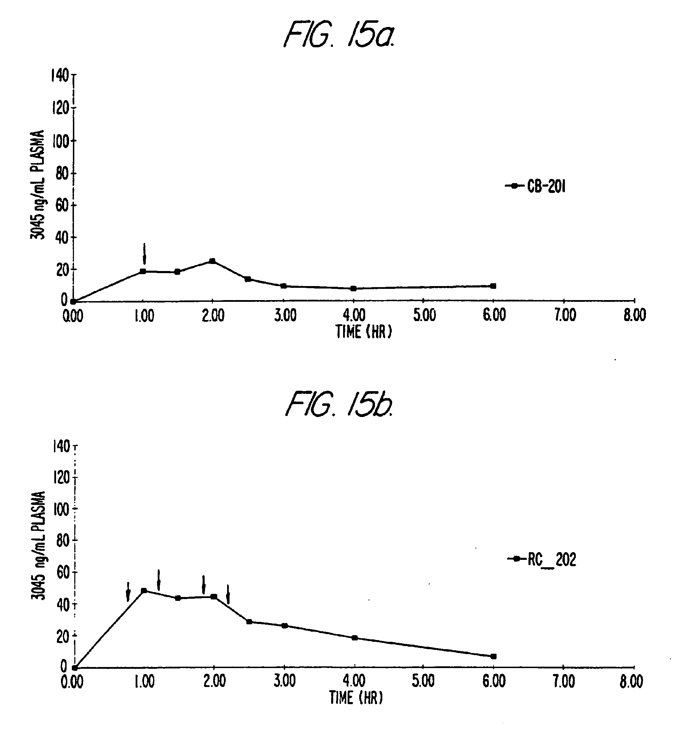

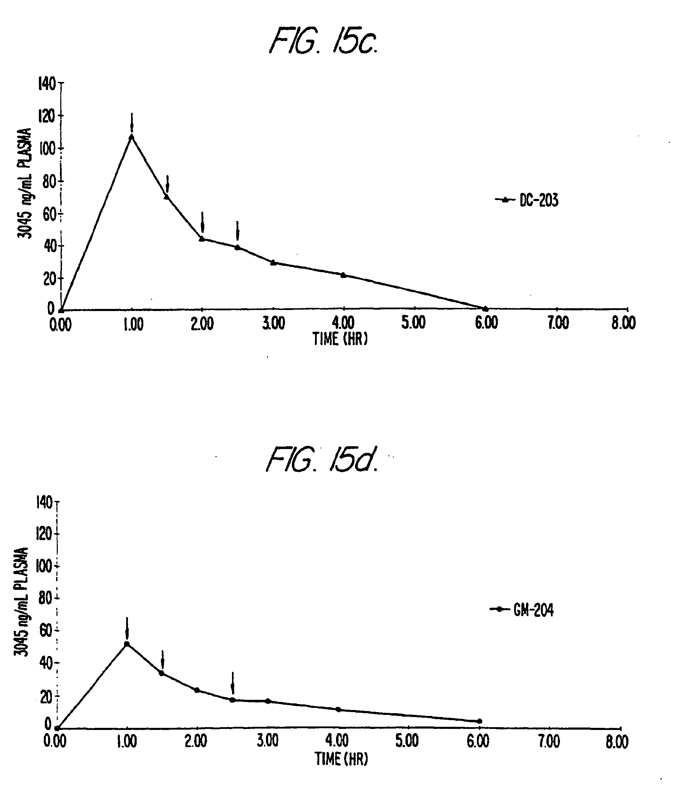

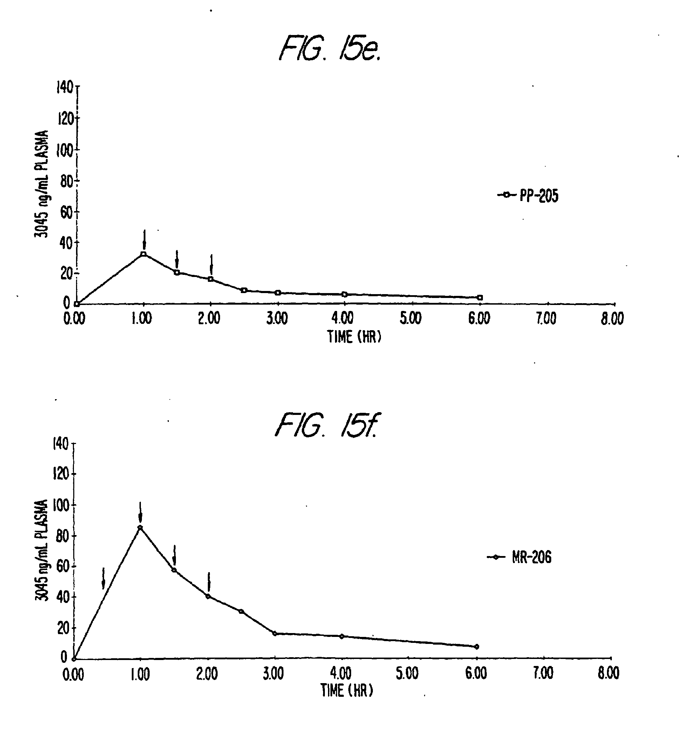

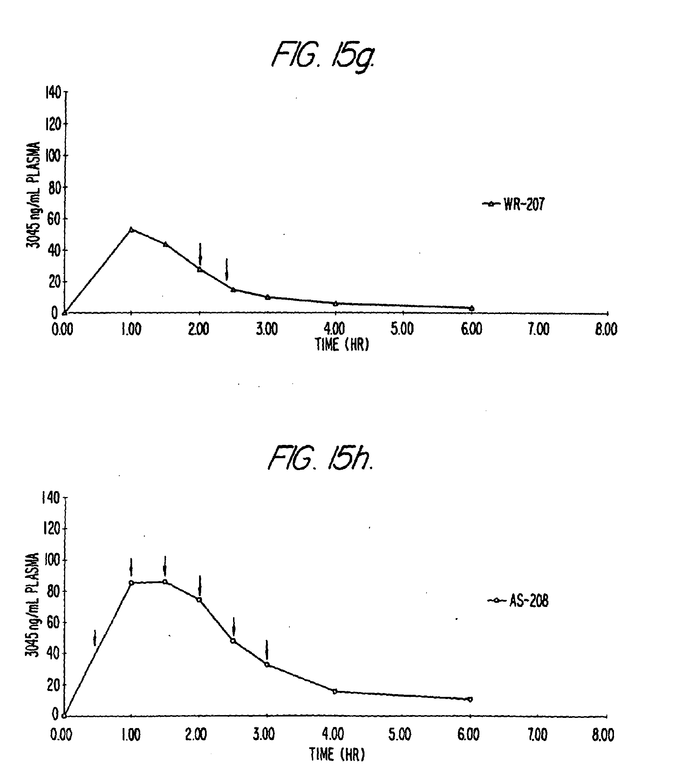

Similar findings were observed in subjects receiving pregnanolone formulated with polyvinylpovidone and sodium lauryl sulfate. All eight subjects were observed to exhibit signs of hypnosis at time points ranging from <1 hour to three hours. Figures 15A-15H show that the peak plasma levels for this formulation ranged from about 20 to 110 nanograms per ml. Table 2 shows that the mean peak concentration for the PVP formulation was 60.9 nanograms per ml.

Formulation 4 consisting of micronized pregnanolone with 1% sodium lauryl sulfate produced peak plasma levels ranging from about 40 to 110 nanograms per ml. Table 2 shows that the mean peak concentration for the micronized formulation was 59.4 nanograms per ml.

Formulation 3 produced peak plasma levels ranging from 10 to 35 nanograms per ml. Table 2 shows that the mean peak concentration for the nanosized formulation was 22.0 nanograms per ml.

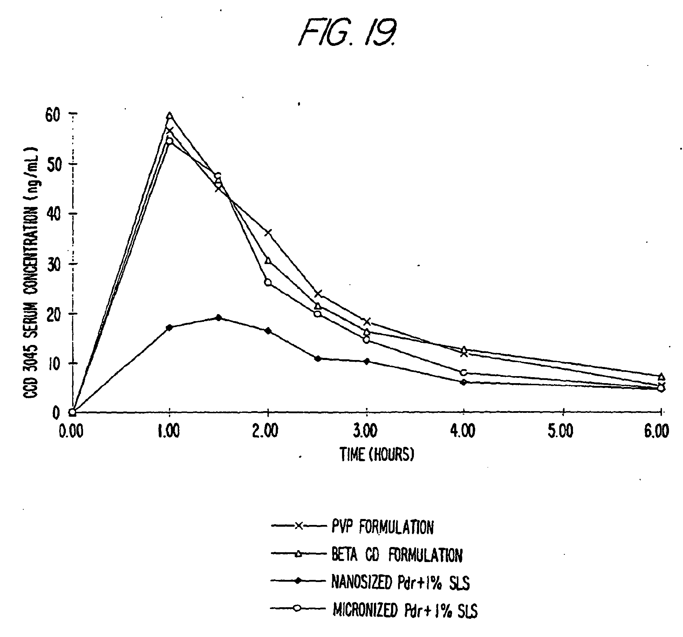

The assessments of relative absorption or bioavailability demonstrate that all but the nanosized formulation have comparable absorption both in terms of the rate and extent of absorption. The serum samples were analyzed for each subject over a 48 hour interval. Figure 19 illustrates the mean serum levels observed for the four different formulations. Three of the formulations, PVP, Beta CD, and the Micronized Powder + SLS all had comparable mean serum concentration-time profiles, while the nanosized pregnanolone formulation had much reduced levels, its peak approximately 35% of the others. In addition to examining the mean serum levels, relative assessment of the peak and extent of absorption can be determined by looking at the peak concentration and area under the serum concentration-time (AUC) profile. Table 1 illustrates those findings.

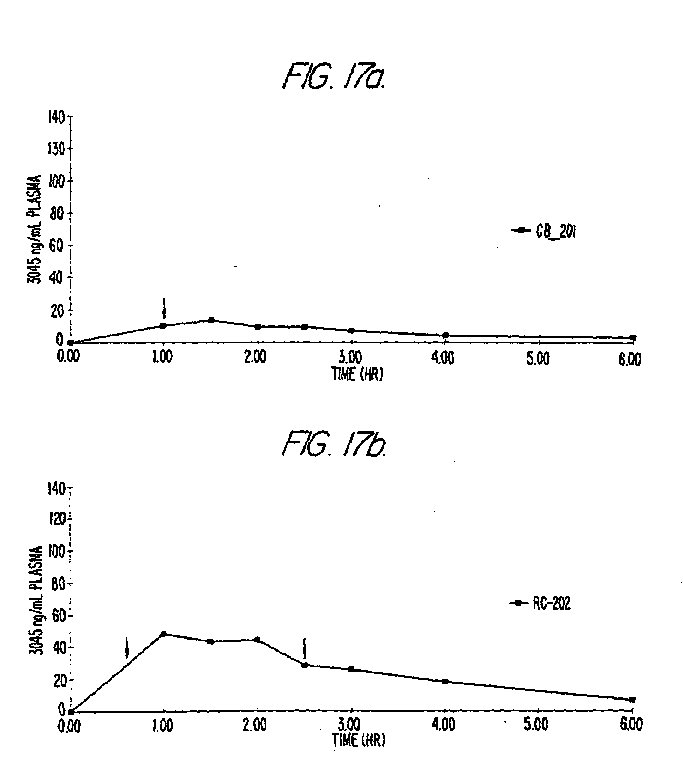

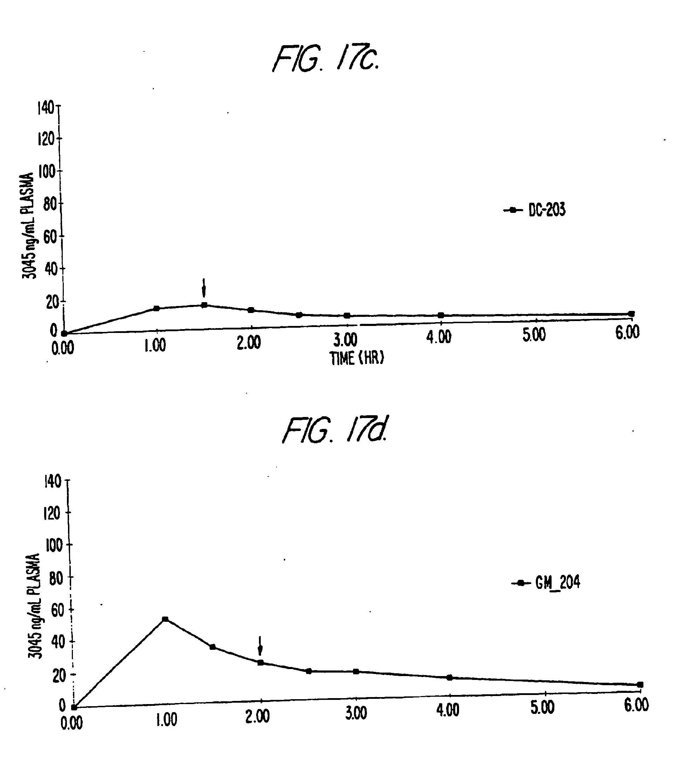

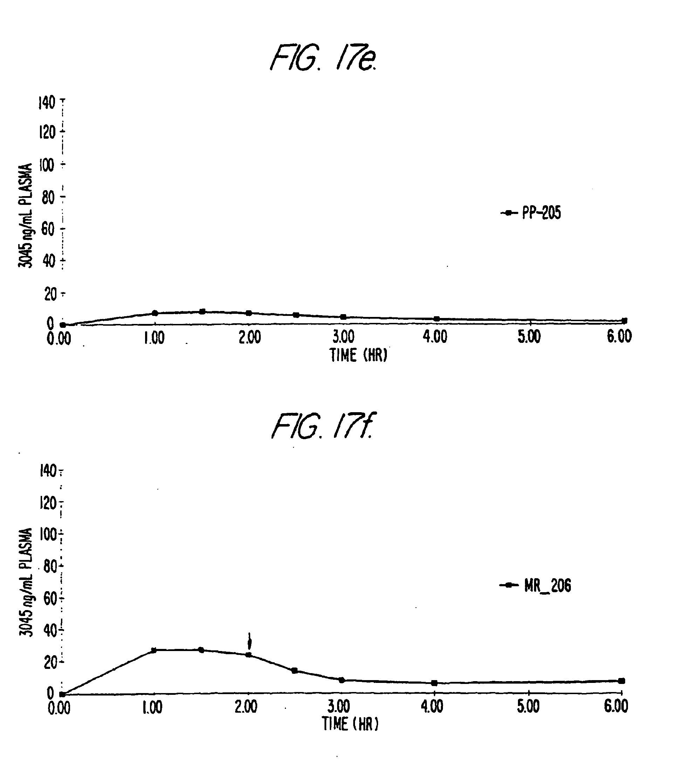

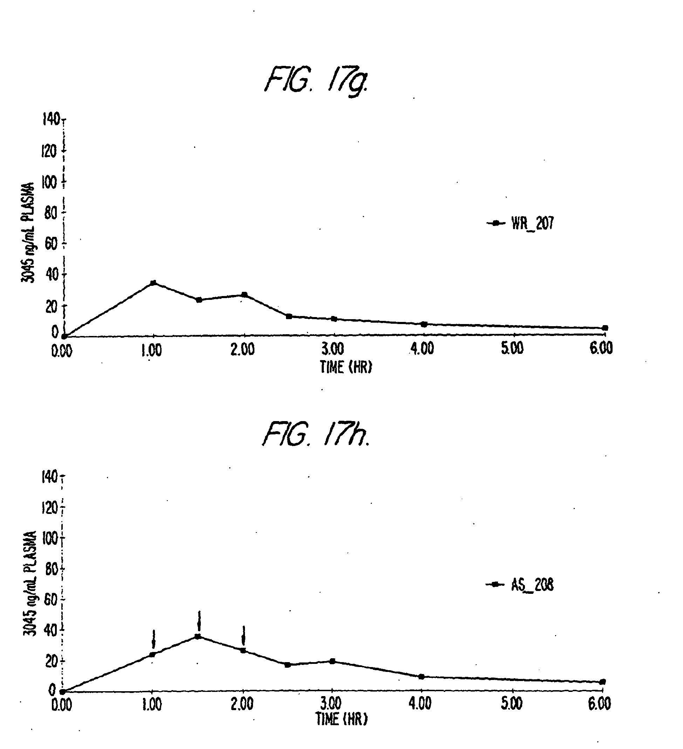

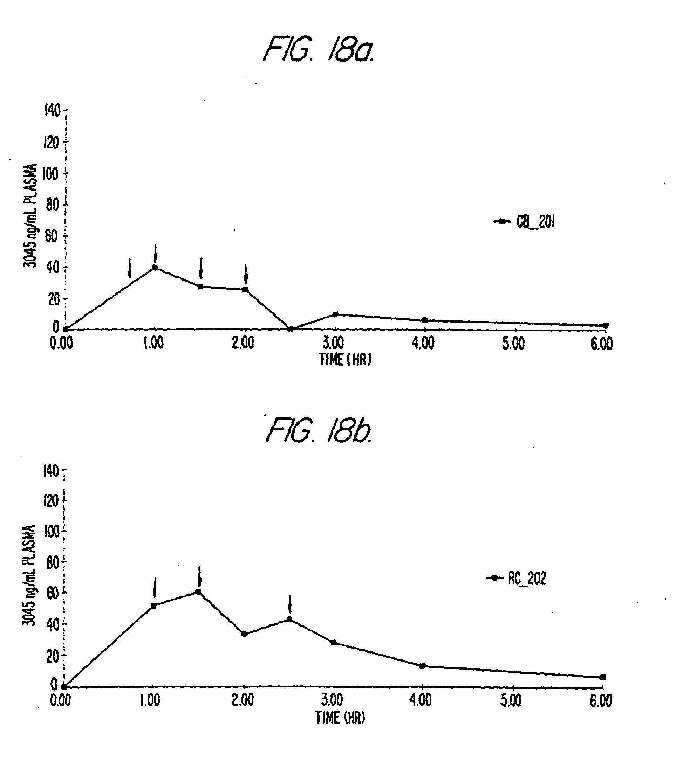

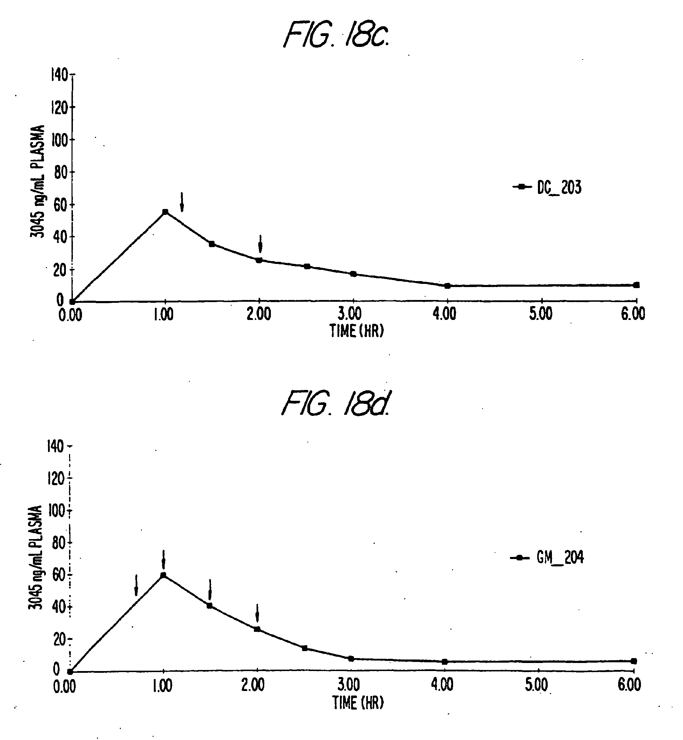

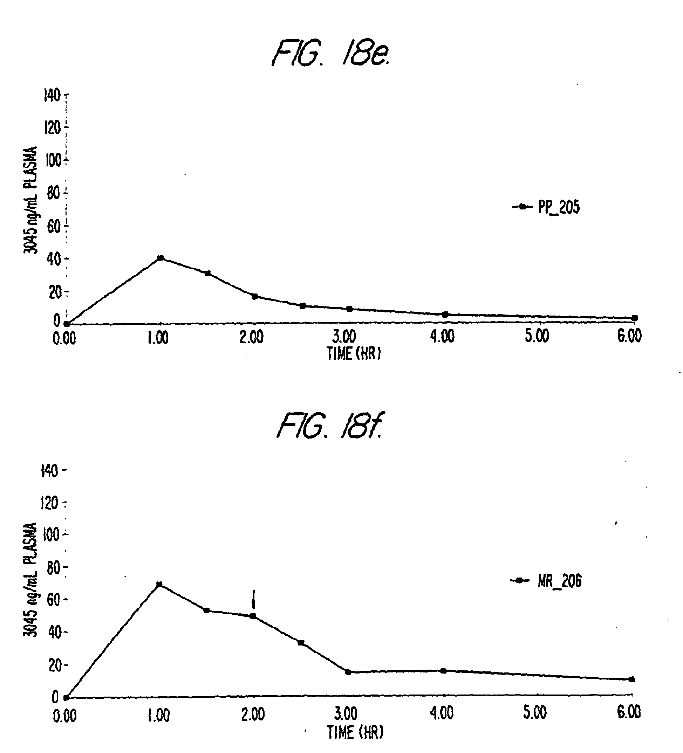

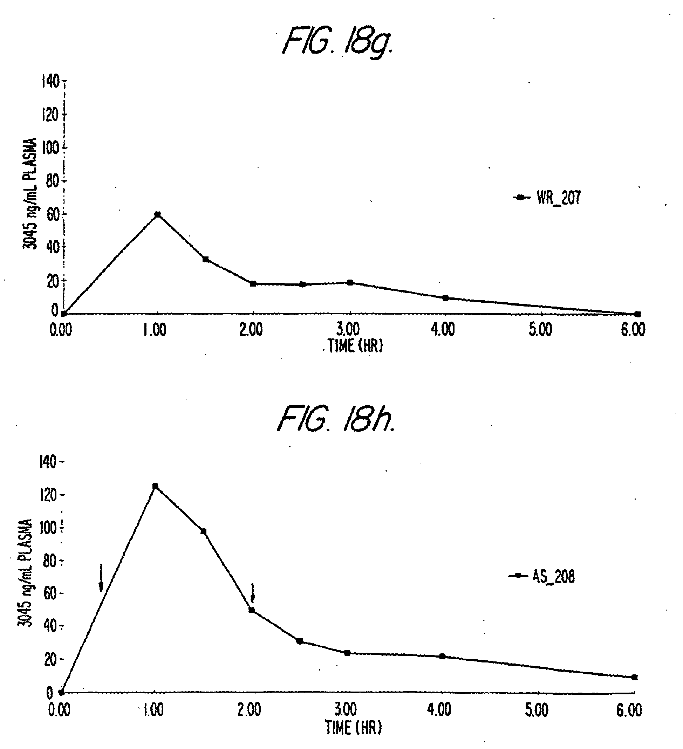

Figures 15A-H, 16A-H, 17A-H, and 18A-H are plots of the serum concentration versus time for individual subjects. At each point where sedative effects were observed, an arrow is placed at the point in time. As can be observed, higher serum concentrations, generally above 30 ng/ml, are related to observe sedative effects.

In another human study, plasma concentrations and sleep propensity were measured in 18 young, healthy, male volunteers for 2 hours after oral administration of single doses of pregnanolane. Two different formulations were tested. Formulation I used β-cyclodextrin as the excipient. Formulation II used sodium lauryl sulfate as the excipient. Both β-cyclodextrin and sodium lauryl sulfate are known pharmaceutical excipients and were chosen to enhance the absorption of pregnanolone without affecting the toxicological profile of the compound.

Formulation I was a β-cyclodextrin inclusion complex prepared by suspending 15.0g of pregnanolone and 82.57 g β-cyclodextrin in 610 mL distilled water in a 1000 mL Erlenmeyer flask. The suspension was stirred by magnetic bar stirrer for 36 hours at 37°C. The suspension was then filtered through a 0.22 µm filter (Millipore, type GV) on which the complex was collected. The resultant complex was dried in a drying cupboard at 50°C for 12 hours after which the mass was comminuted in a porcelain mortar, and the granulate was passed through a 300 µm sieve. The granulate was dried in vacuo over P₂O₅ at 50°C for 24 hours. This formulation was packaged in sachets containing 150 mg of pregnanolone.

Formulation II was a 99:1 physical mixture of pregnanolone and sodium lauryl sulphate prepared by mixing 49.50 g pregnanolone, which had been passed through a 125 µm sieve, and 0.50 g sodium lauryl sulfate in a mortar and pestle for 30 minutes. The mixture is then passed through a 300 µm sieve. This formulation was packaged as 250 mg pregnanolone capsules and 500 mg pregnanolone sachets.

Prospective subjects were examined 21 days prior to their first dose to determine medical histories and baseline clinical laboratory values. The subjects were told not to take any prescription or non-prescription drugs before or during the study. Each subject participated twice within a two-week period. Successive single dose treatments were separated by a one-week interval.

Test subjects fasted overnight and reported to the study center 3 hours prior to dose administration. A light snack was given 2 hours prior to the dose administration. A standard FDA-approved high-fat breakfast was given to the subjects 20 minutes prior to dosing and was finished 5 minutes prior to dosing. After breakfast, the subjects were given one of the two formulations in liquid yogurt. The contents of a sachet was added to 100 mL of liquid yogurt and stirred for 20-30 seconds. The subjects then drank the yogurt. An additional 30 mL of yogurt was mixed with the remnants in the cup and consumed assure full administration. After drinking this, the sides and bottom of the cup were scraped and fed to the subject. No liquids were allowed for the first hour after drug administration. Subjects had to remain sitting or standing for the first hour after ingestion.

Mean plasma concentrations for the β-cyclodextrin formulation reached a peak value of 82.08 ± 57.18 ng/mL at 1 hour post dosing. The mean plasma concentration was 79.33 **±** 49.71 ng/mL at 1.5 hours and was 74.21 ± 37.21 ng/mL at 2 hours.

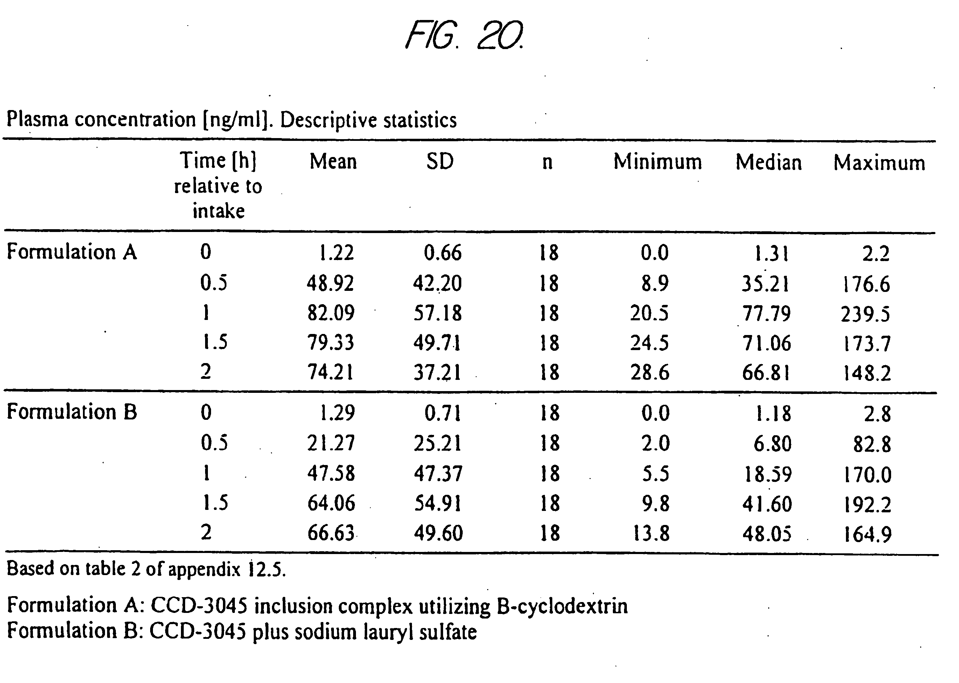

The sodium lauryl sulfate formulation reached its peak mean plasma concentration later. The mean value was 64.06 ± 54.91 ng/mL of 1.5 hours and was 66.63 ± 49.60 ng/mL at 2 hours post dosing. These statistics are shown in Figure 20.

Subjects were monitored by EEG, EOG, and EMG which were recorded for 10 minutes in a darkened room with closed eyes. The recordings were made for 5 minutes under reaction time conditions. Subjects were supposed to respond as quickly as possible to one of two different tones presented in random sequence and intervals by pressing a button with their left or right thumb. The subjects then were recorded for 5 minutes without interruption.

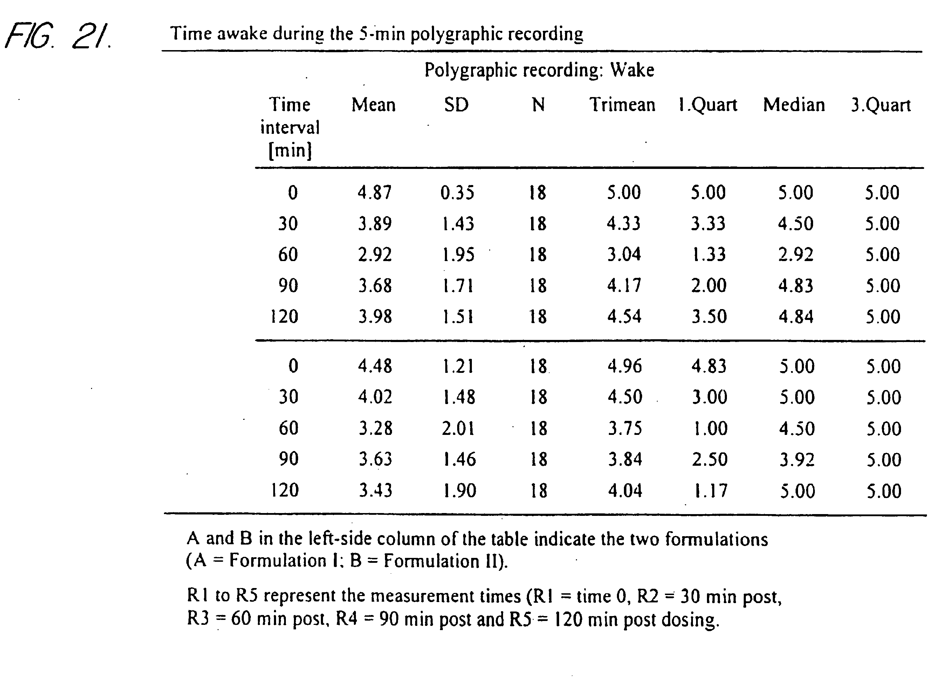

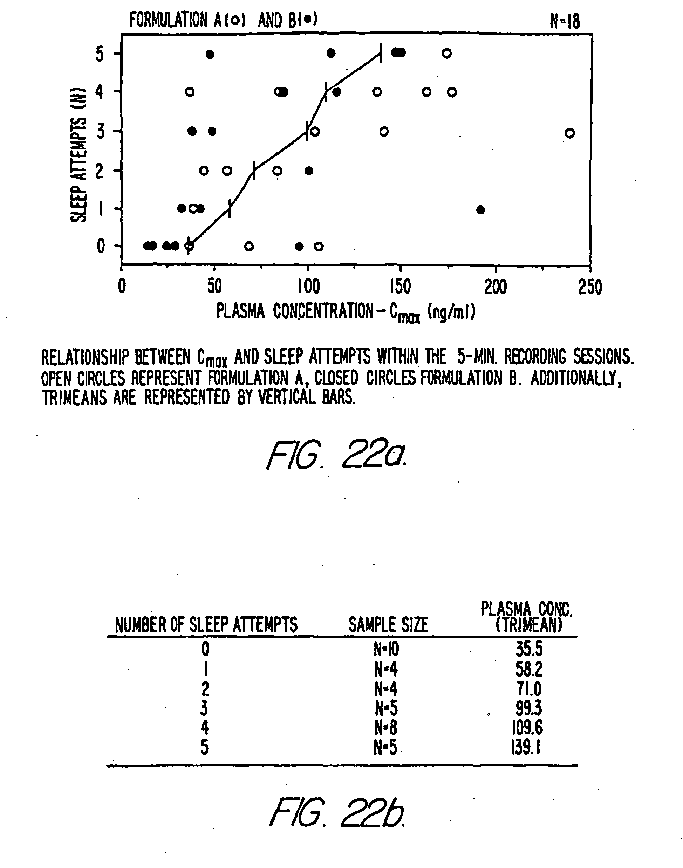

As shown in Figure 21 the time awake decreased post dosing. The number of sleep attempts was also determined. A sleep attempt was defined as a transition from wakefulness to any sleep stage within the 5 minute recording session. On each study day, a subject had five polygraphic recording sessions. Therefore, the maximum number of sleep attempts could vary from 0 to 5. A five indicates that sleep was observed even during the 5 minute pre-dose recording session. The number of sleep attempts was plotted against the maximum plasma concentration (C max) in Figure 22A. A low plasma concentration generally coincided with few sleep attempts while high plasma concentrations coincided with a high number of sleep attempts. Figure 22B shows the trimean plasma concentration levels for different numbers of sleep attempts.

The data from these human studies show: (1) that pregnanolone can be formulated in such a way as to produce suitable blood levels from oral administration; and (2) that the blood levels of pregnanolone found in the volunteers correlated to the observation of drowsiness and/or sleepiness.

To demonstrate the pharmacological properties of the compounds used in the present invention, the inventors had in vivo experiments run on rats in which the effects of pregnanolone (reference), 3α-hydroxy-3β-trifluoromethyl-5β-pregnan-20-one 21-phosphate disodium salt, 3β-ethynyl-3α-hydroxy-5β-pregnan-20-one (reference), 3α-hydroxy-3β-methyl-5α-pregnan-20-one (reference), 3α,21-dihydroxy-3β-trifluoromethyl-50-pregnan-20-one 21-hemisuccinate sodium salt, 3α-hydroxy-3β-ethynyl-5α-pregnan-20-one (reference), 3α,21-dihyroxy-3β-ethynyl-5β-pregnan-20-one 21-hemisuccinate (reference) 3α-hydroxy-3β-trifluoromethyl-19-nor-5β-pregnan-20-one, 3α-hydroxy-21-(pyrid-4-ylthio)-5β-pregnan-20-one (reference), 3α-hydroxy-3β-trifluoromethyl-5β-19-nor-pregnan-20-one 21-phosphate disodium salt on brain EEG, locomotor activity, and body temperature were measured. Rats are a suitable model for human sleep because all compounds that are hypnotics in man have hypnotic effects in rats and vice versa. Edgar, D.M. "Drug Discovery and Evaluation Using the Advanced Technology Sleep-Wake Bioassay Laboratory at Stanford University" p. 4, December 1992. In addition, the rat model for human sleep has been accepted by peer-review committees at NIH Id. at 5. Furthermore, rat sleep and human sleep are fundamentally the same. Id. at 4. For example, the proportion of time spent in NREM versus REM sleep is about 4:1 in both rats and humans. Id. at 5.

Rat preparation, monitoring, and analysis of the data was conducted as described below. Adult, male Wistar rats (275-350 g at the time of surgery, Charles River Laboratories) were anesthetized (Nembutal, 60mg/kg) and surgically prepared with a cranial implant that permitted intermittent EEG and EMG recording. Body temperature and locomotor activity were monitored via a miniature transmitter (Minimitter) surgically placed in the abdomen. The cranial implant consisted of stainless steel screws (2 frontal [+3.9 AP form bregma, ±2.0 ML] and two occipital [-6.4 AP, ±5.5 ML]) for EEG recording. Two Teflon-coated steel wires were positioned under the nuchal trapezoid muscles for EMG recording. All leads were soldered to a miniature connector prior to surgery, and chemically sterilized in Glutarex (3M Co.). The implant assembly was affixed to the skull with dental acrylic. A minimum of three weeks was allowed for recovery.

Each rat was permanently housed in its own individual recording cage located within separate, ventilated compartments of custom-designed stainless steel cabinets. Each Nalgene microisolator cage, which is an individual recording cage, was enhanced with a filter-top riser and swivel-commutator. Food and water were available ad libitum. A 24 hour light-dark cycle (12 hours on, 12 hours off) was maintained throughout the study using 4-watt fluorescent bulbs 5cm from the cage. Animals were undisturbed for three days both before and after treatments. In practice, animals were undisturbed during the weekend. Treatments were administered on Monday evening or Tuesday morning, and the animals were again left undisturbed until Friday, when cages were cleaned and the data copied off the computers.

The computers monitored the EEG, EMG, body temperature and non-specific locomotor activity (LMA) via telemetry, and drinking activity from 48 rats simultaneously. The computer classified the arousal state every 10 seconds using pattern-matching algorithms which included behavior-dependent contextual rules. Drinking and LMA were recorded every 10 seconds, while body temperature was recorded every minute.

As nocturnal creatures, rats are most active when the lights are off, and like humans, are most sensitive to hypnotic effects toward the end of their activity dominated period. Therefore, time of treatment is important. By administering the drug at CT-18, which is the peak of the rat's activity dominated period, the natural tendency for the rats to sleep at lights on (3:00 clock time or Circadian Time, CT-0) cannot mask the hypnotic activity of the drug. By administering the drug at CT-5 when the propensity for REM sleep is greatest, the drug's effect on REM sleep can be measured more accurately. The activity dominated period begins when the lights are turned off at CT-12 or 15:00 clock time on the Figures.

Compounds were suspended in sterile 0.25% methylcellulose, 20-50% hydroxy propyl cyclodextrin, or water depending on the physical properties of the steroid. These vehicles provide adequate bioavailability for all hypnotics tested to date. The drugs and controls were injected intraperitoneally or administered orally in a volume of 1 ml/kg or 10 mg/kg, respectively, under dim red illumination for the CT-18 studies or under regular light for the CT-5 studies.

A matched control group, N=20, was selected from a pool of 60 available control candidates. These matched control groups were constructed as follows. For each of the variables (NREM, REM, LMA, body temperature, and sleep bouts), a curve of hourly-averaged values was computed for each candidate for the 24 hours preceding injection. The 20 candidates whose curve best fit (least squares) the mean curve for the treatment group were selected. Therefore, a matched-pairs procedure was not used, but parallel treatment groups with closely comparable pre-treatment baseline data were used.

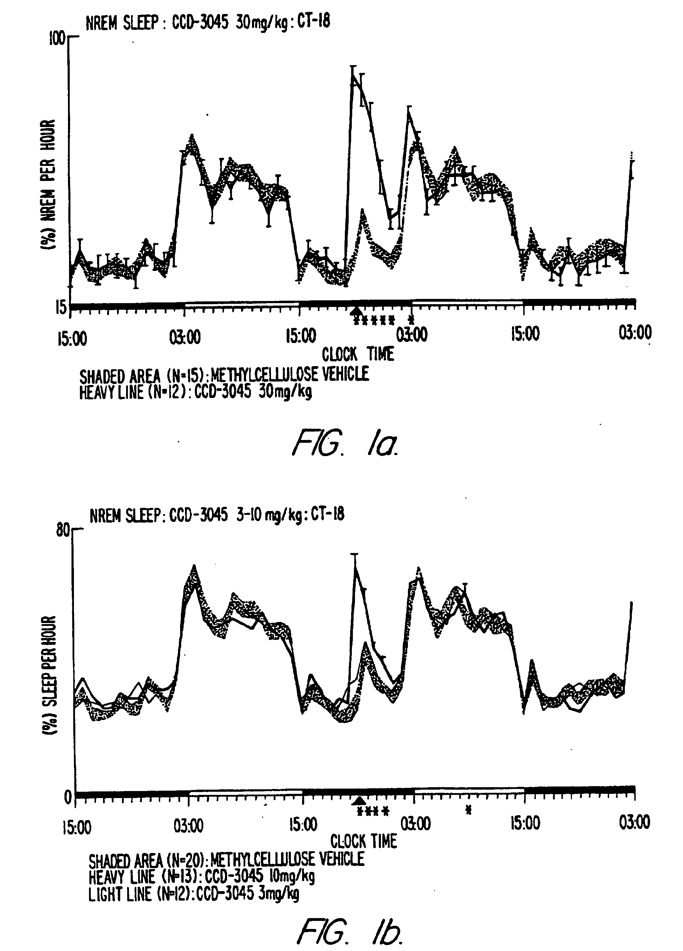

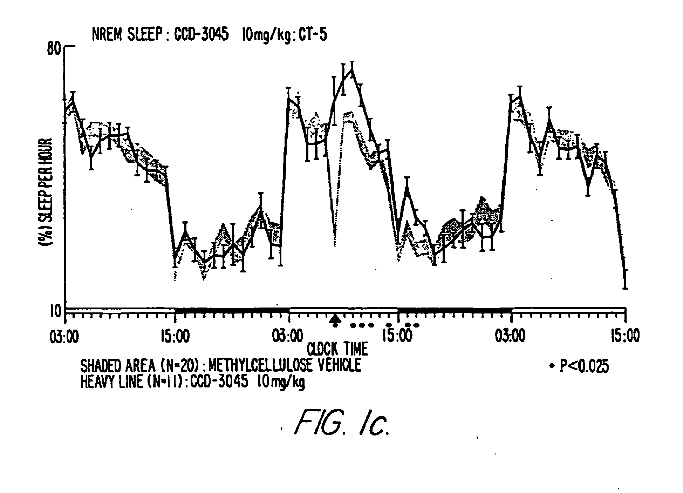

Figures 1A, 1B, and 1C demonstrate that pregnanolone administered at either CT-5 or CT-18, is effective in promoting NREM sleep independent of the time at which it was administered. At both times, there is rapid onset and high potency. At CT-5, the rat is more rested and it is more difficult to induce sleep. These results indicate that the neuroactive steroids of the invention are desired hypnotic agents when sleep must be induced earlier than usual, as may occur in shift work and jet lag. Furthermore, since a physician prescribing a hypnotic can never be certain of the patient's sleep debt, it is advantageous to have a hypnotic drug such as the present steroids with a predictable hypnotic action independent of the patient's sleep debt.

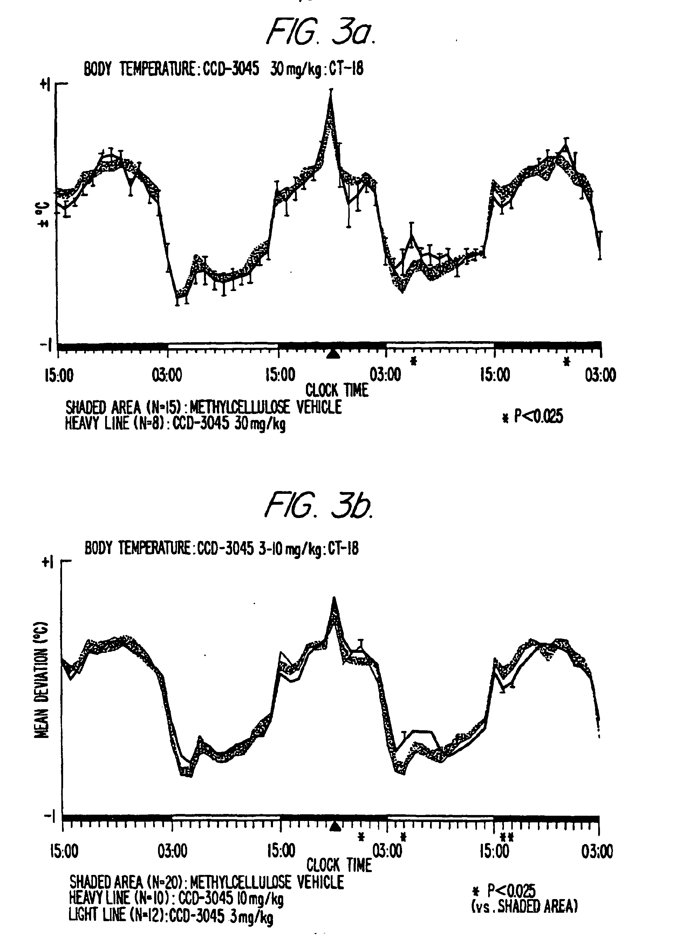

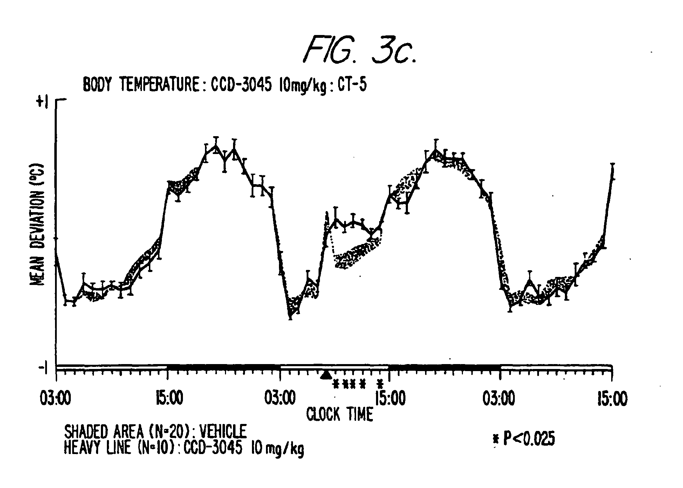

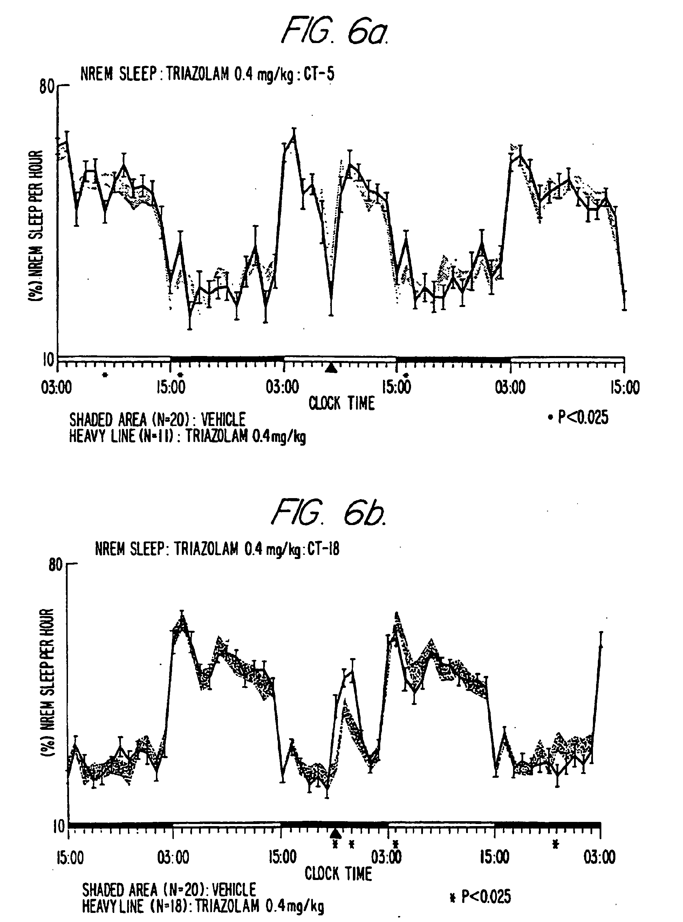

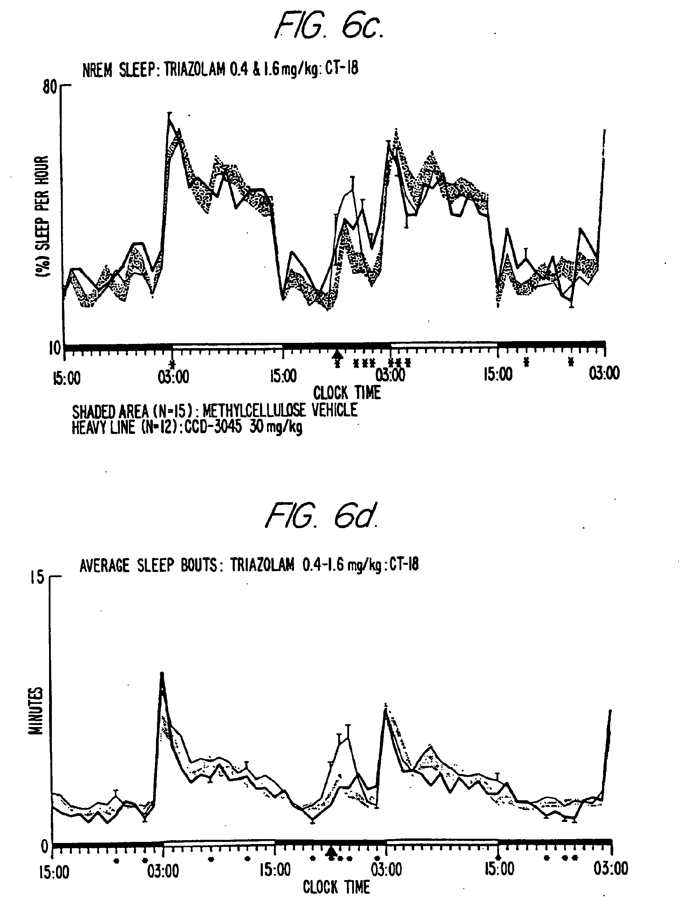

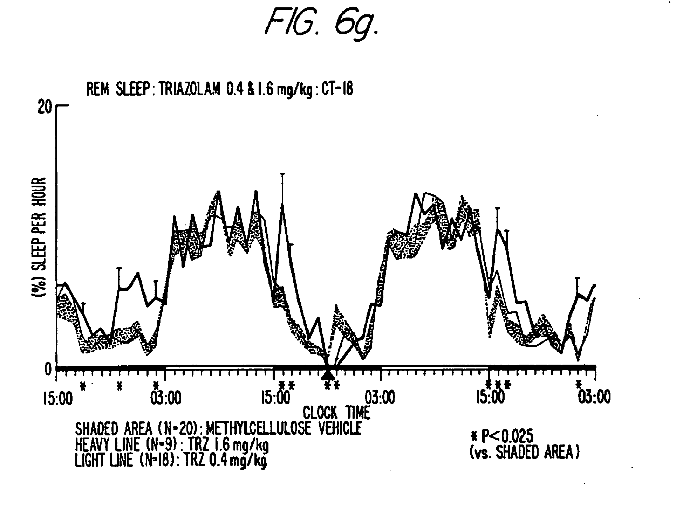

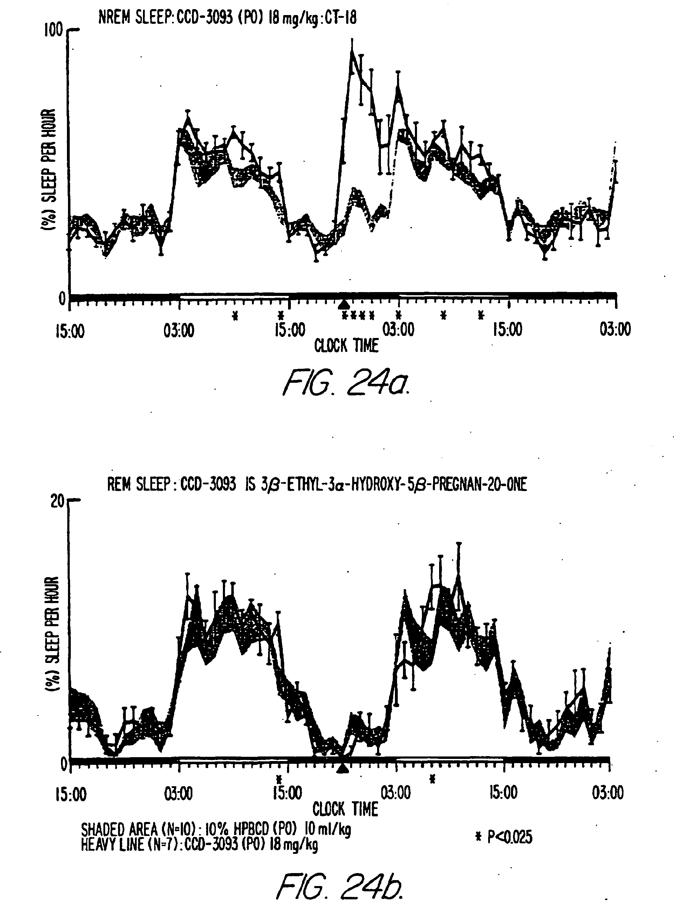

Figures 1A-C also indicate that pregnanolone lacks a rebound effect. Rebound effect is defined as the reduction of NREM sleep after the hypnotic action of the treatment has returned to control levels. Pregnanolone produced no rebound reduction of NREM sleep at CT-5 or CT-18 for any dose. As shown by FIG. 24A, 3β-ethynyl-3α-hydroxy-5β-pregnan-20-one also produced no rebound reduction of NREM sleep at CT-18. Triazolam showed a rebound reduction of NREM sleep when administered at CT-18. Figure 6B shows a star ("*") at the right hand side, indicating a statistically significant reduction in NREM sleep.

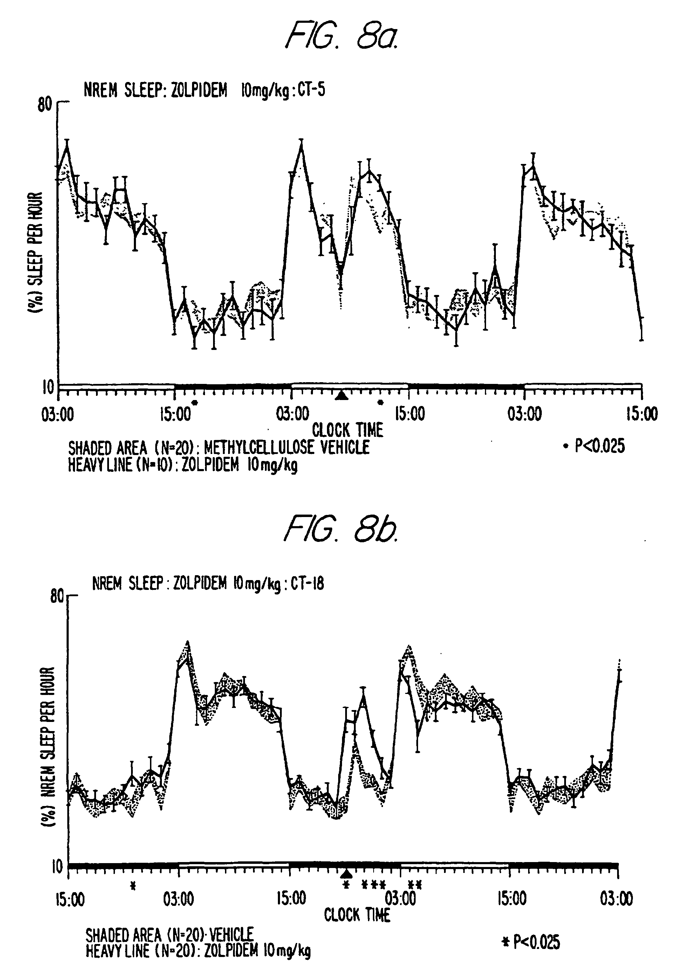

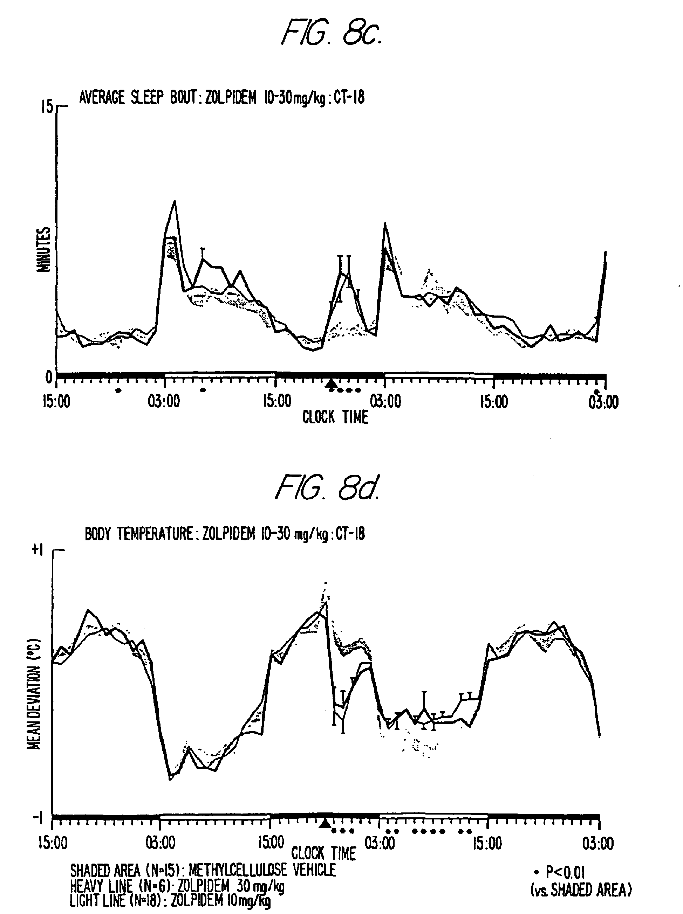

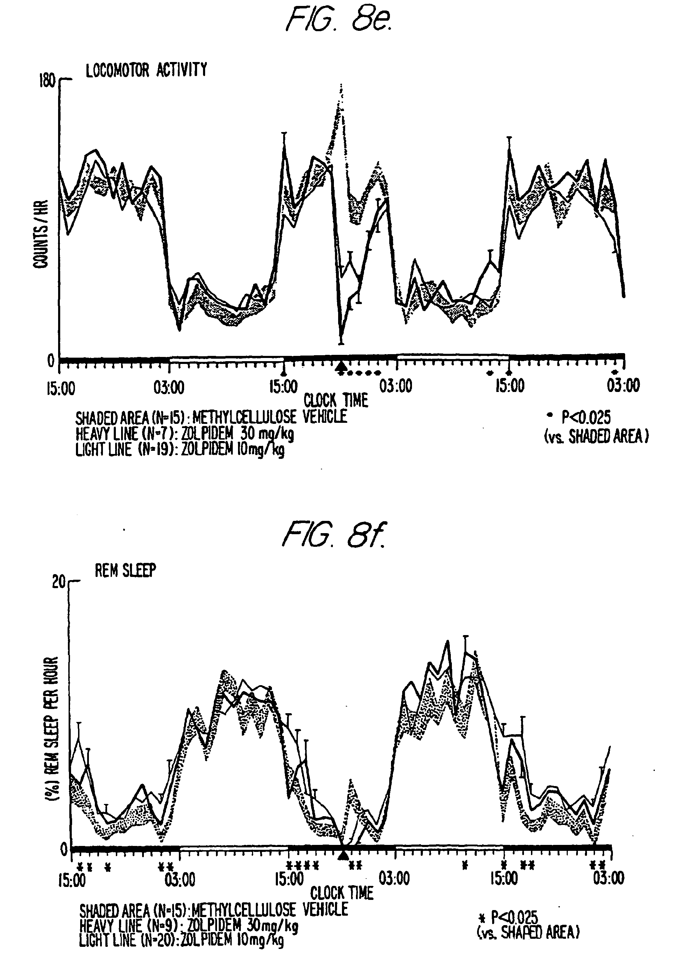

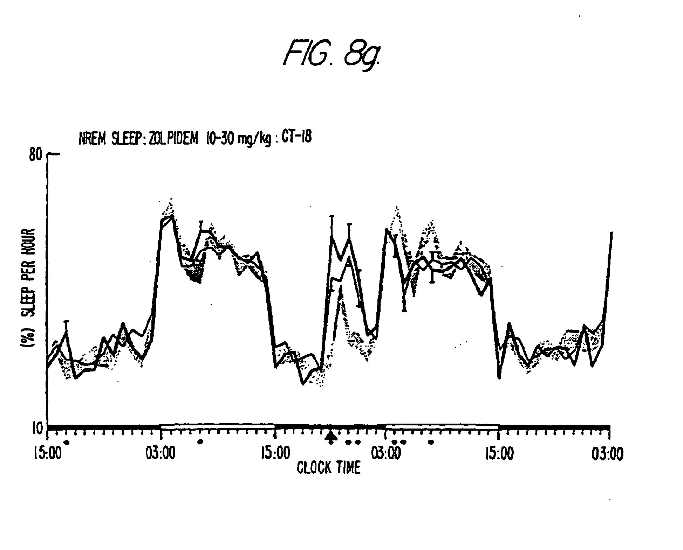

Pregnanolone is unique among hypnotic agents in being both potent at both CT-5 and CT-18, and not producing rebound wakefulness. While our studies indicate that Zolpidem did not produce rebound insomnia, it was also unable to effectively induce NREM sleep at CT-5, as shown in Figure 8A. This feature could possibly imply that pregnanolone or other neuroactive steroids which do not produce rebound insomnia would produce less tolerance, withdrawal, and rebound insomnia clinically compared to current BZ-receptor ligands, which are their most important limitations.

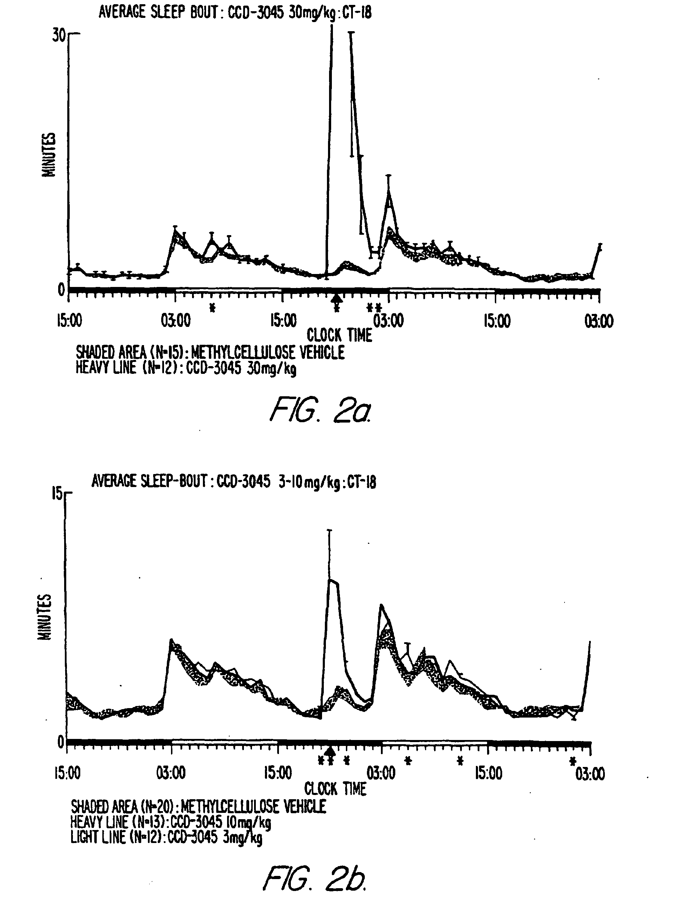

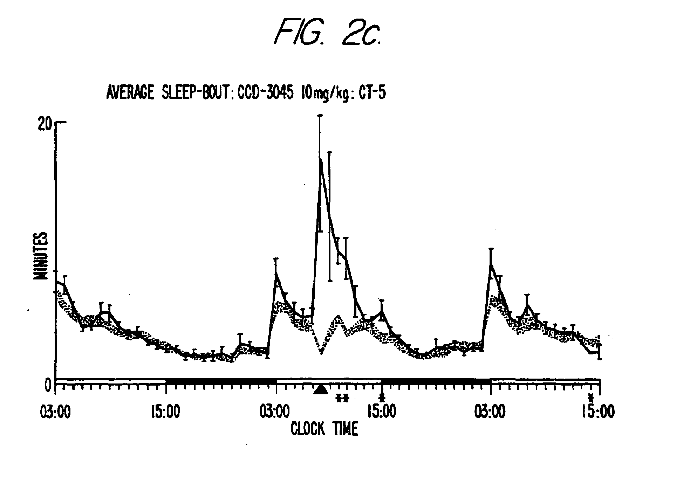

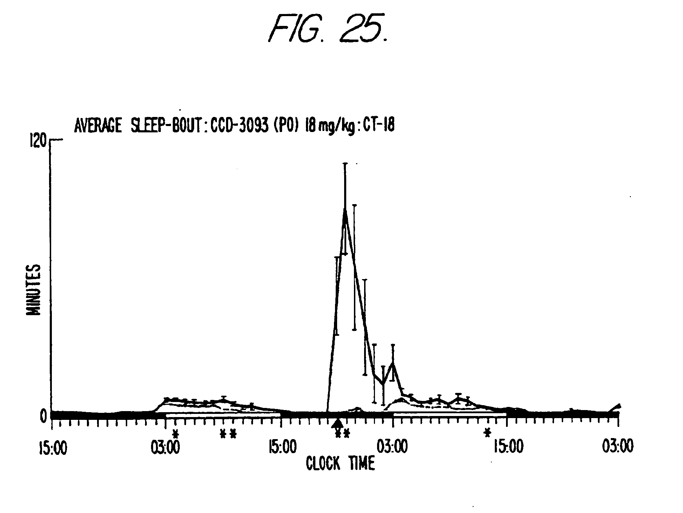

Figures 2A, 2B, 2C, and 25 demonstrates that administration of pregnanolone and the administration of 3β-ethynyl-3α-hydroxy-5β-pregnan-20-one causes a strong increase in the duration of uninterrupted NREM sleep or "sleep bout length". Sleep bout length is of interest because it may parallel the human tendency to awaken periodically during the night, which has been shown to be an important factor in determining the restorative value of sleep in humans. This strong consolidation of sleep has great potential in the treatment of age related insomnia.

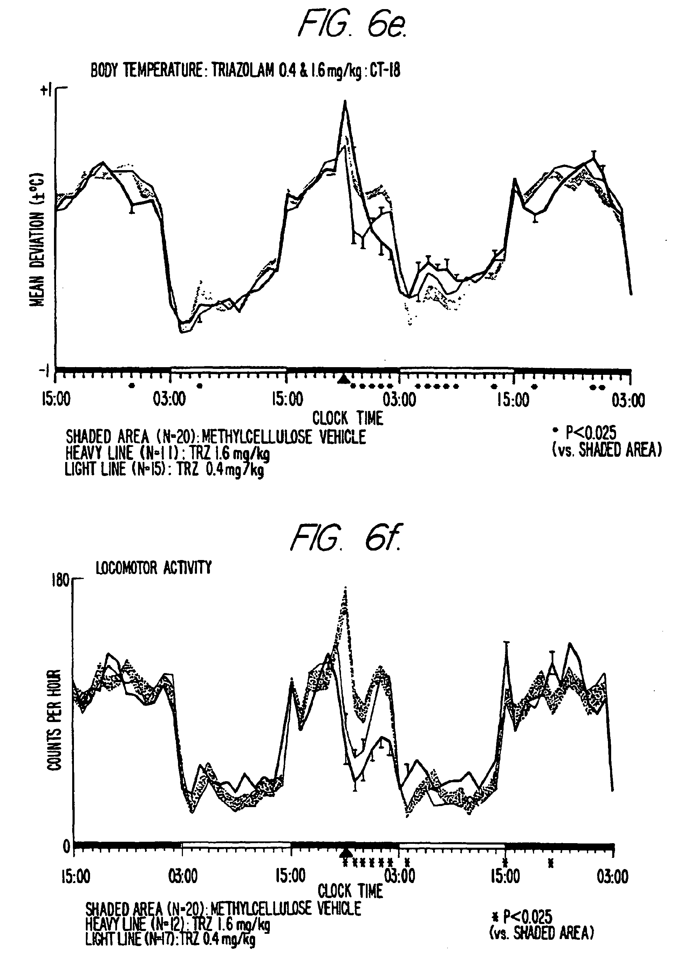

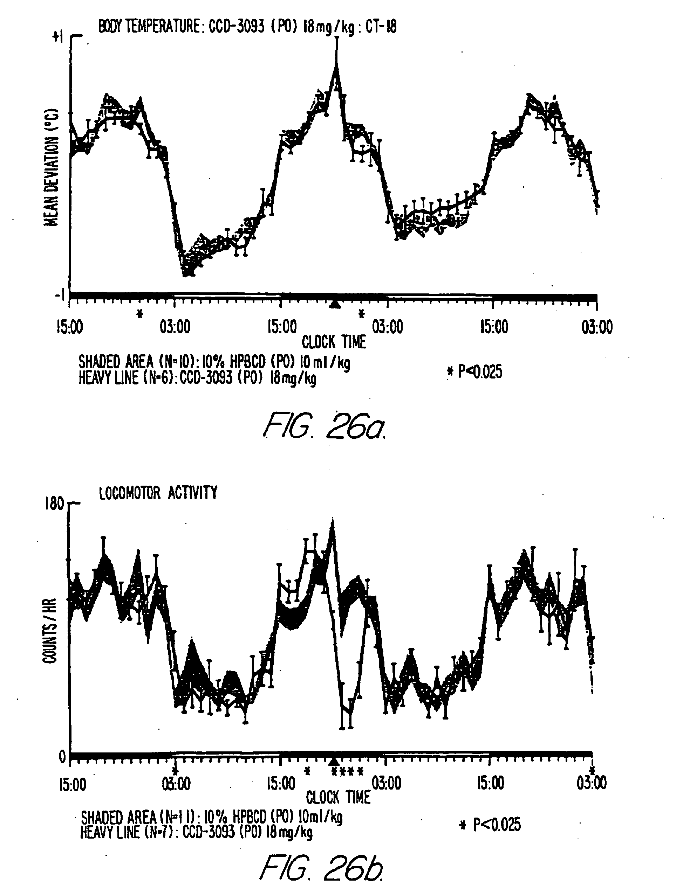

Figures 3A, 3B, and 3C indicate that pregnanolone tends not to affect body temperature, contrary to the teachings of Attallah Kappas et al. who found that intramuscular administration of pregnanolone caused a pyrogenic effect in man. "Fever-Producing Steroids of Endogenous Origin in Man" 105:68/701-68/708 A.M.A. Archives of Internal Medicine 1960. Note in the hours prior to injection, body temperature fluctuates in a near-sinusoidal rhythm. Handling and injection cause a brief increase in body temperature. Figure 26A also shows that 3β-ethynyl-3α-hydroxy-5β-pregnan-20-one has a minimal effect on body temperature. Triazolam and Zolpidem affect body temperature as revealed in Figures 6E and 8D, respectively. A lack of temperature effect suggests that the NREM inducing mechanism of pregnanolone and the neuroactive steroids of the invention does not produce undesirable side-effects on homeostatic control functions.

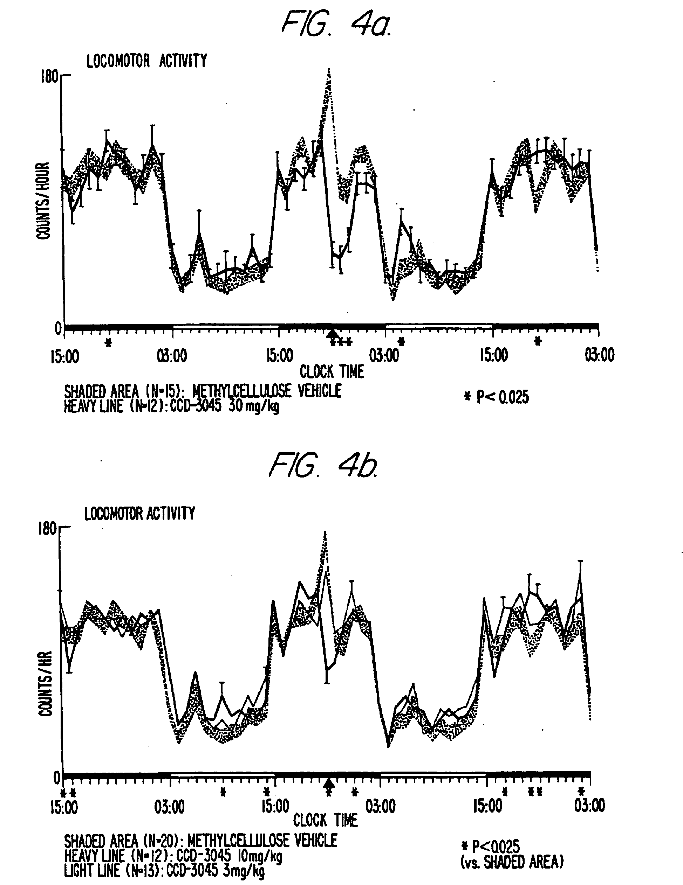

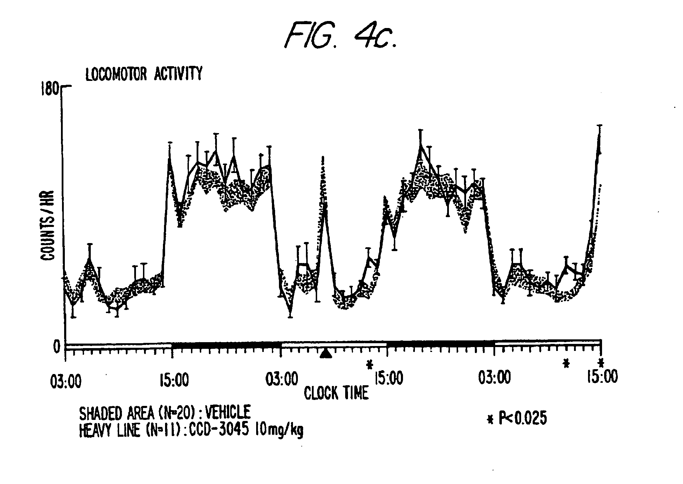

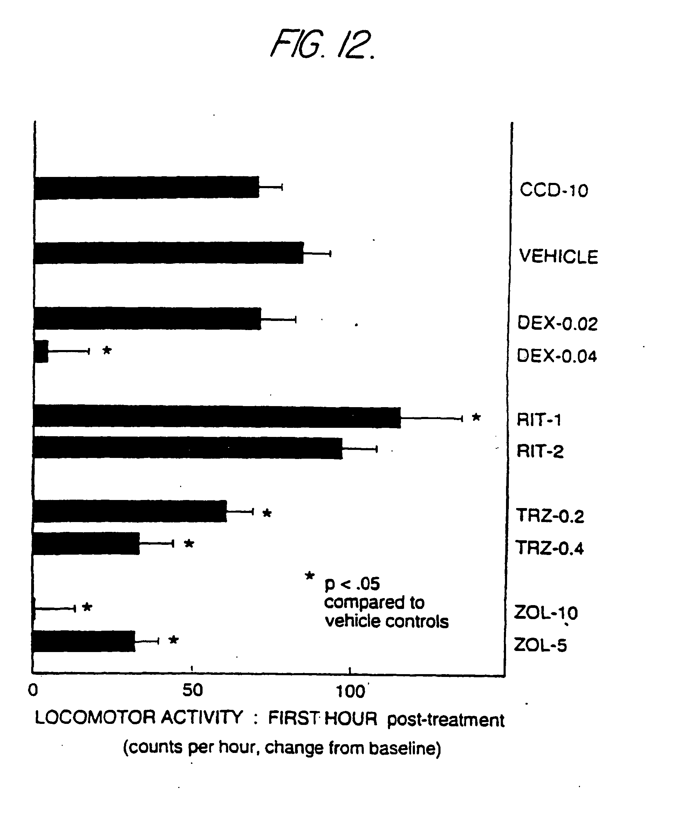

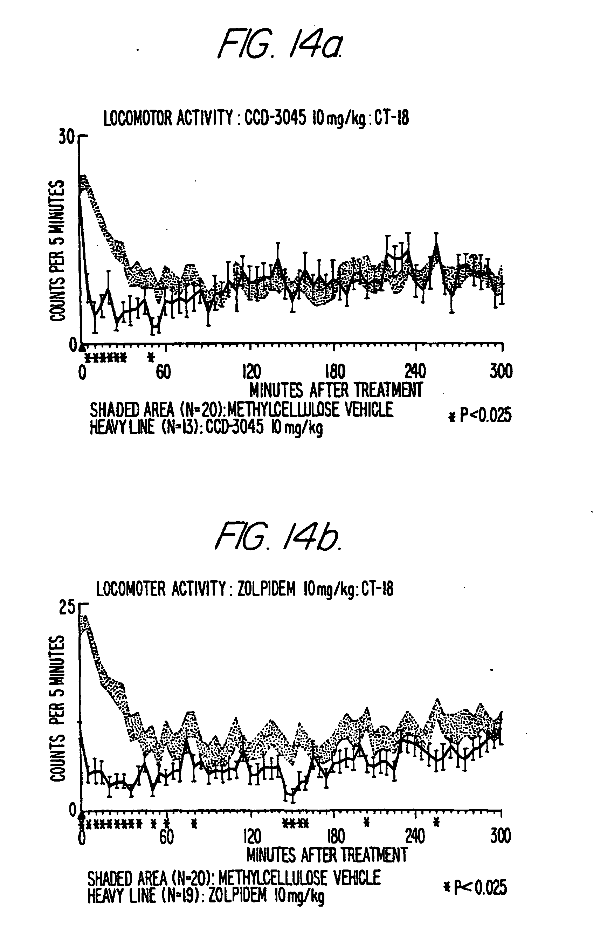

Pregnanolone produces a moderately smaller reduction in locomotor activity (LMA) over time at 30 mg/kg and no reduction at 10 mg/kg as compared to Triazolam and Zolpidem, as illustrated in Figures 4A-C, 6F and 8E. 3β-Ethynyl-3α-hydroxy-5β-pregnan-20-one also produced a moderately smaller reduction in LMA over time as illustrated in Figure 26B. Both Triazolam and Zolpidem reduced LMA below vehicle control levels when administered at CT-5 as indicated in the bar graph of Figure 12, even though Triazolam had no hypnotic effect at CT-5. Figures 14A and 14B demonstrate that during the effective period of pregnanolone and Zolpidem when the rats are asleep, they are not moving. However, Zolpidem continues to depress LMA even after the hypnotic action has worn off. Motor incoordination appears to be comparatively reduced, which is a valuable trait for hypnotics used with the elderly.

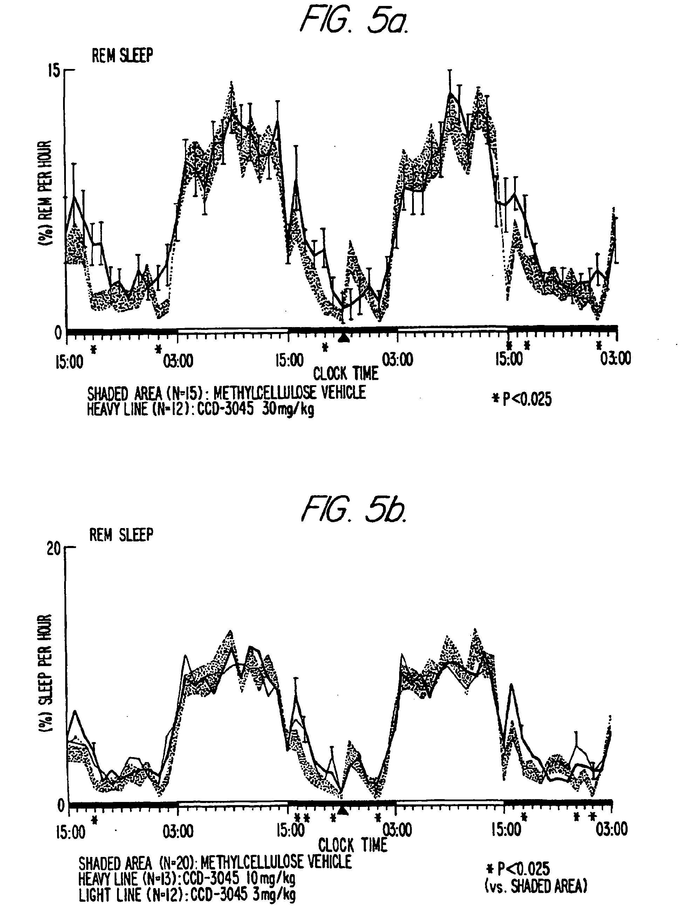

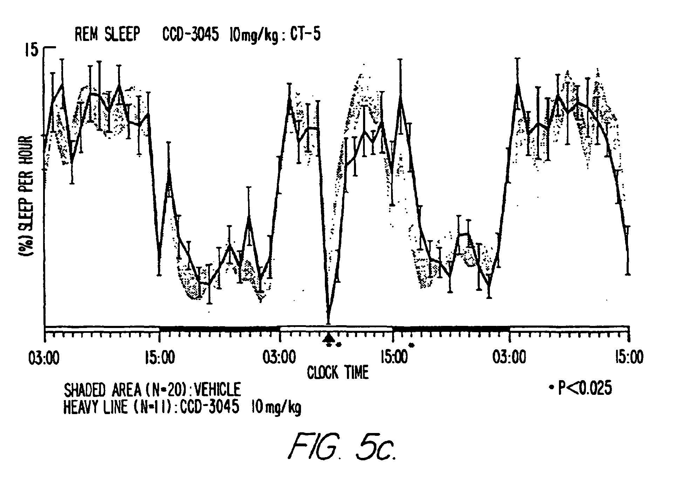

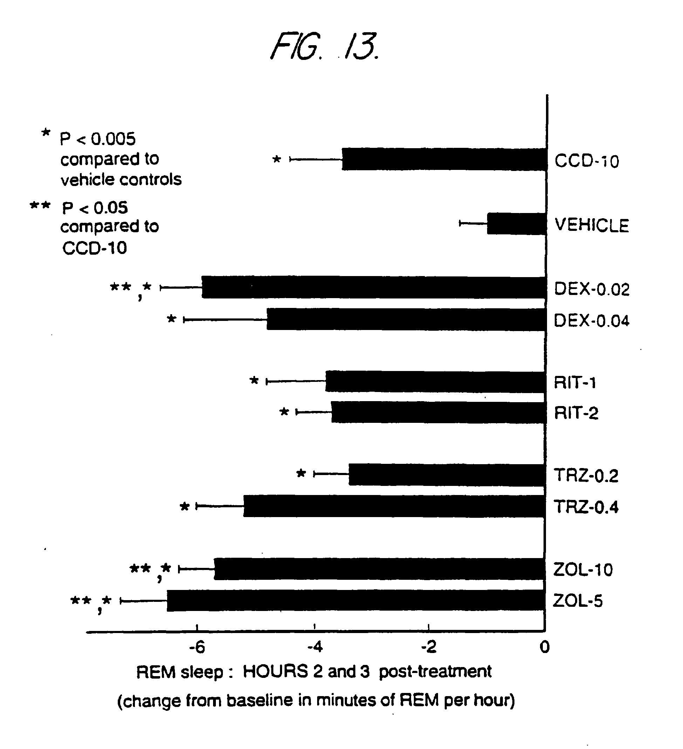

Figures 5A-5C and 24B indicate that pregnanolone and 3β-ethynyl-3α-hydroxy-5β-pregnan-20-one, respectively, lack an inhibitory effect on REM sleep compared to other hypnotics, such as the benzodiazepines, which reliably reduce REM sleep. Figures 6F and 8F show that Triazolam and Zolpidem, respectively, administered at CT-18 reduced REM sleep in the first few hours after administration. For example, the bar graph in Figure 13 demonstrates that Zolpidem interferes with REM sleep at a dose administered at CT-5 that induced significantly less NREM sleep than pregnanolone.

Pregnanolone, 3β-ethynyl-3α-hydroxy-5α-pregnan-20-one, and the other neuroactive steroids are unique in that they combine a potent NREM-promoting effect with slight REM-interference. Strong inhibition of REM sleep in humans, such as alcohol and barbiturates produce, has been associated with sleep disruption as the drug effect diminishes and REM rebound sets in. Although the biological function of REM sleep is unknown, the finding that the neuroactive steroids of the invention only slightly interfere with REM, while potently inducing sleep, is a favorable attribute.

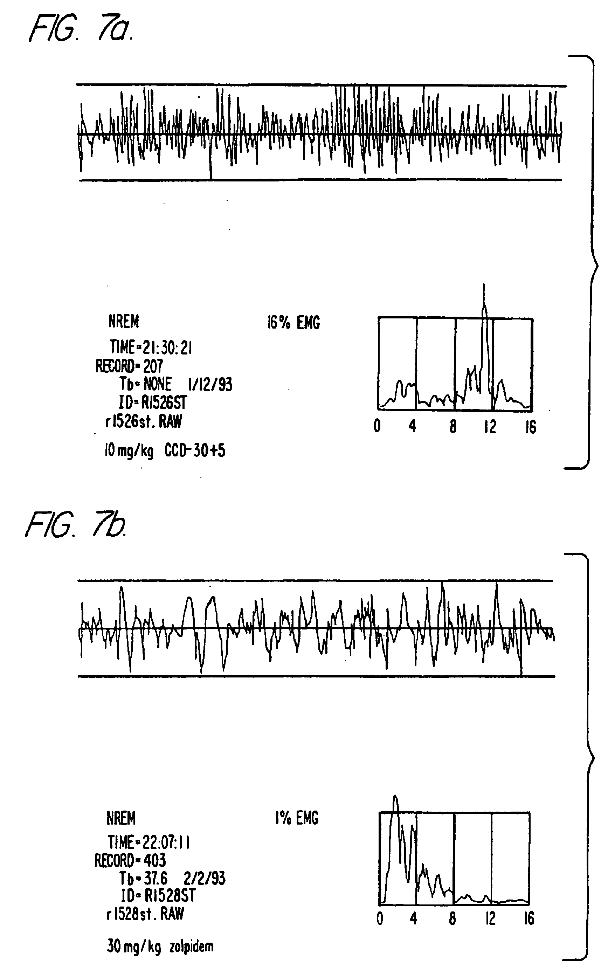

Pregnanolone produced a high-frequency, high - amplitude EEG pattern during NREM sleep. This EEG pattern is reproduced in Figures 7A-B. In contrast, deep normal NREM sleep generally has a low frequency, high amplitude EEG wave. Pregnanolone's effect on EEG spectral profiles was closely similar after both CT-5 and CT-18 treatments. This EEG pattern was distinct from the other hypnotics, as shown in Figures 7C-D.

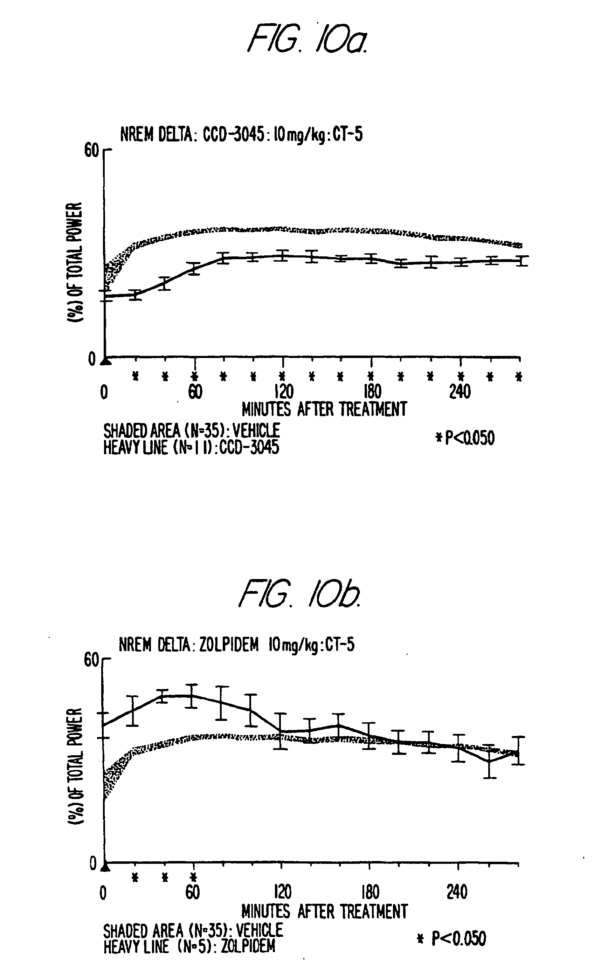

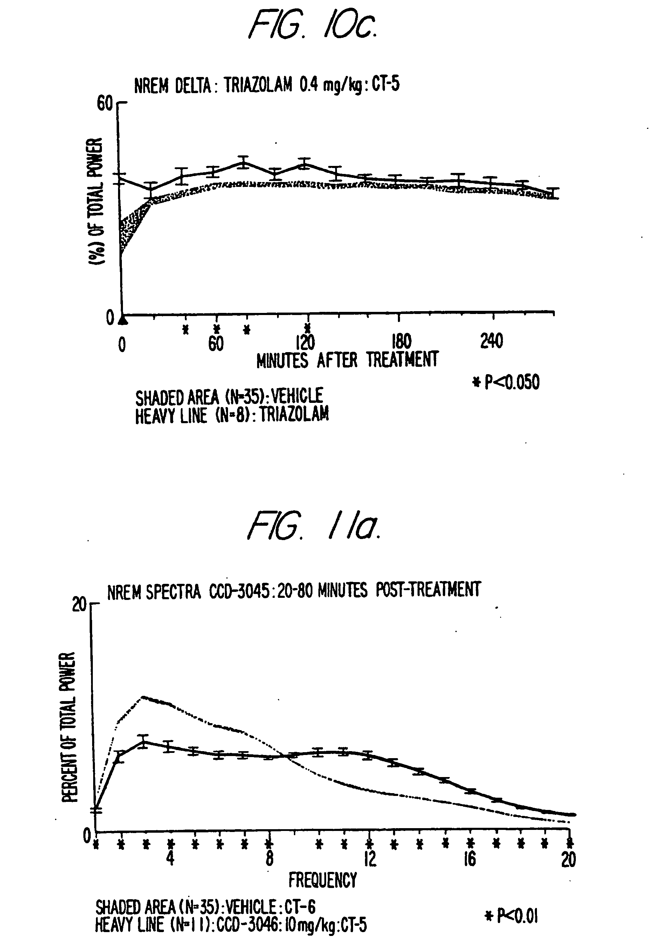

As illustrated in Figure 10A pregnanolone had characteristic effects on NREM EEG delta activity (power). The "depth" of NREM sleep may be quantified as the proportion of EEG activity that occurs at low (delta) frequencies of 0.1-4.0 Hz. For each 10 second period of EEG activity that was determined to be NREM sleep, the EEG delta power was measured and averaged across 20 minute intervals and then compared to vehicle controls. Figure 10A indicates that pregnanolone reduced the percentage of EEG power in the low delta range for at least 5 hours after administration. In contrast, Figures 10C and 10B show that Triazolam and Zolpidem, respectively, increased the percentage of EEG power in the delta range for at least 1 hour after treatment.

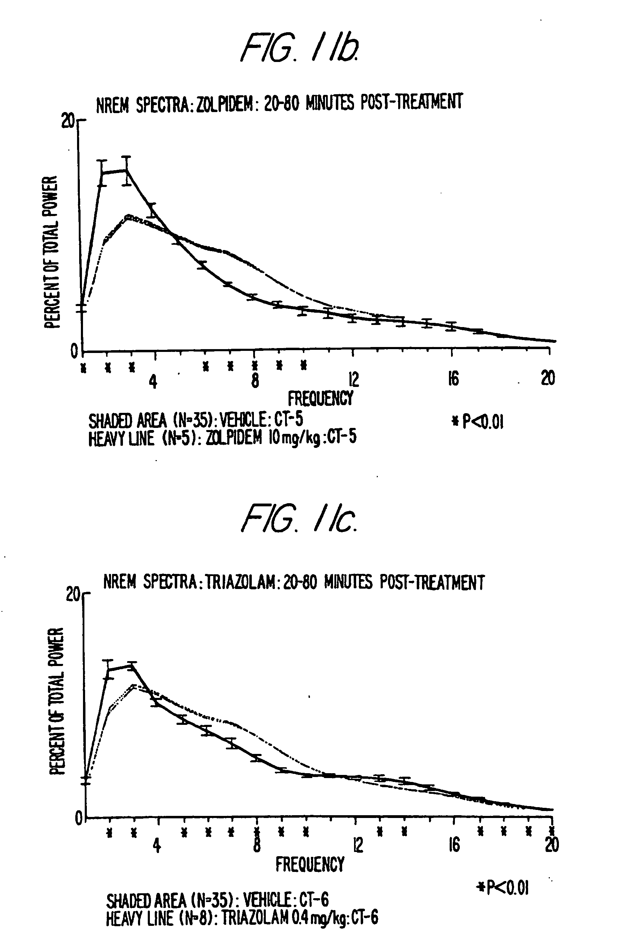

It was also found that pregnanolone produced a characteristic distribution of frequencies in the EEG during the period of peak drug effect. The purpose of determining a drug's "EEG spectral profile" is to determine which frequencies have increased or decreased in abundance following treatment. Figure 11A shows that pregnanolone shifted the power from the lower frequencies (0.1-8 Hz) to the higher frequencies (≥10 Hz). In contrast, Zolpidem and Triazolam increased the percentage of EEG power in the lower delta frequency range. See Figures 11B and 11C, respectively.

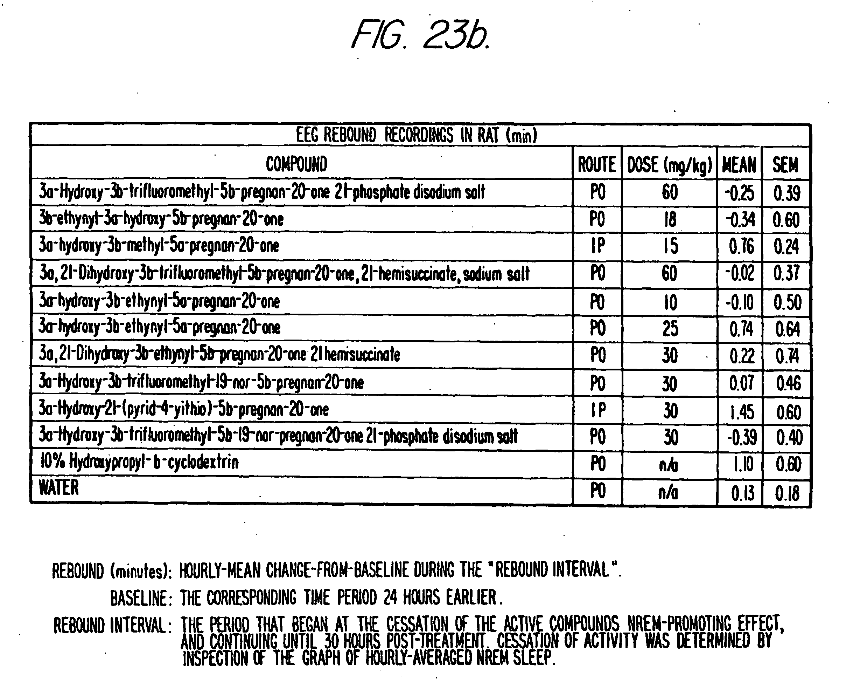

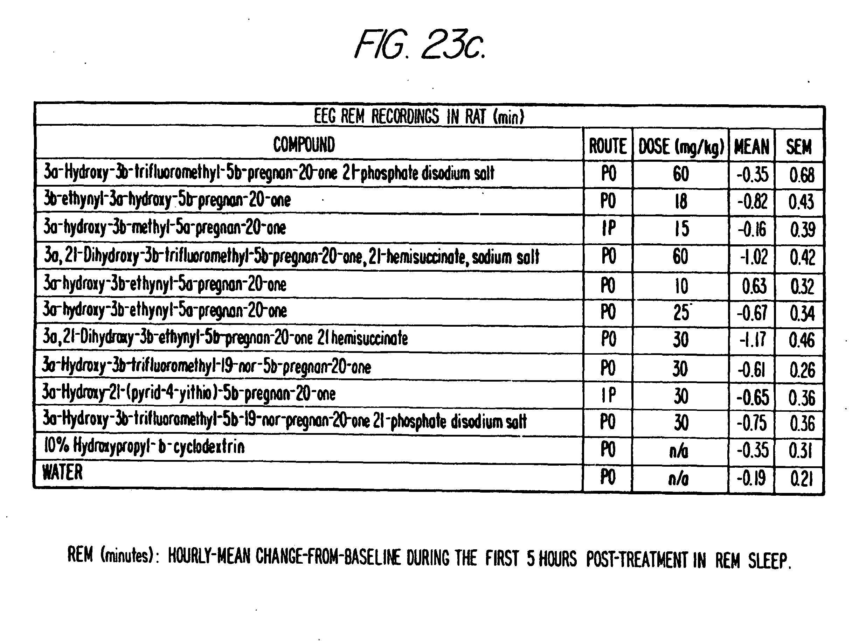

Using the same experimental design as used for pregnanolone, nine other neuroactive steroids with various structure modifications of the invention were tested on rats. All nine synthetic neuroactive steroids produced profiles similar to pregnanolone. The effects on REM, NREM, rebound insomnia, locomotor activity, body temperature and power spectrum were similar to those described above for pregnanolone and 3β-ethynyl-3α-hydroxy-5β-pregnan-20-one. The changes in REM, rebound insomnia, and NREM sleep in these experiments are summarized in Figures 23A-C, respectively. Like pregnanolone, all nine steroids showed only slight REM interference, while producing a potent NREM promoting effect. (Figs. 23A & C). Note also that these steroids produced little or no rebound effects. See Fig. 23B.

In summary, pregnanolone and the nine other synthetic neuroactive steroids strongly increased NREM sleep, but did not cause any temperature drop, only slightly interfered with REM sleep, did not significantly reduce locomotor activity after the hypnotic action had worn off, and was not followed by rebound wakefulness. By contrast, Zolpidem and Triazolam reduced body temperature and locomotor activity after the hypnotic effects had worn off. Zolpidem and Dexmedetomidine (an antihistamine which is a class of drugs known to cause drowsiness) strongly interfered with REM sleep. Dexmedetomidine produced strong rebound effects. Taken together, these results show that the neuroactivity steroids of this invention may have a more specific mode of hypnotic action than BZ-receptor ligands, thereby producing less impairment of other important functions (i.e., body temperature, and possibly motor coordination or motivation). Benzodiazepines, in contrast, impair LMA after the hypnotic effects wear off, a trait that is undesirable in a sleep inducing medication.

The medicament for the treatment of insomnia in accordance with this invention is to be administered to a subject in need of induced sleep in a nontoxic amount sufficient to produce sleep. The preferred dose is 5 mg to 250 mg of active compound.

## Claims

1. The use of a 3β-substituted, 3α-hydroxy-pregnan-20-one or 19-norpregnan-20-one compound having Formula II: 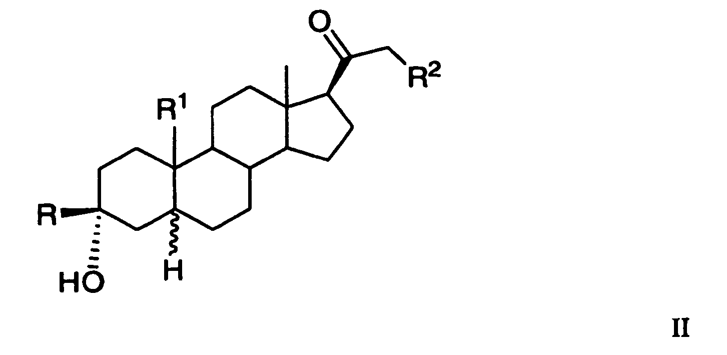 and sodium salts thereof;
wherein
R is C₁₈ trihaloalkyl
R¹ is hydrogen or a C₁₋₈ alkyl group; and
R² is one of hydrogen, hydroxy, pyrid-4-ylthio, hemisuccinoyloxy or phosphoryloxy, in the manufacture of medicament for the treatment of insomnia wherein the medicament is to be
administered orally.

2. The use as claimed in claim 1, wherein R is selected from the group consisting of trifluoromethyl and 2',2',2'-trifluoroethyl.

3. The use as claimed in claim 1, wherein R² is selected from the group consisting of hemisuccinoyloxy, phosphoryloxy and sodium salts thereof.

4. The use as claimed in claim 1, wherein the compound is 3α-hydroxy-3β-trifluoromethyl-5β-pregnan-20-one 21-phosphate disodium salt; 3a,21-dihydroxy-3β-trifluoromethyl-5β-pregnan-20-one 21-hemisuccinate sodium salt; 3α-hydroxy-3β-trifluoromethyl-19-nor-5β-pregnan-20-one;3α-hydroxy-3β-trifluoromethyl-5β-19-nor-pregnan-20-one 21-phosphate disodium salt;3a-hydroxy-3β-trifluoromethyl-5β-pregnan-20-one;
3α-hydroxy-3β-trifluoromethyl-19-nor-5α-pregnan-20-one;
3α,21-dihydroxy-3β-trifluoromethyl-5β-pregnan-20-one;
3α,21-dihydroxy-3β-trifluoromethyl-5β-pregnan-20-one, 21-hemisuccinate;
3α,21-dihydroxy-3β-trifluoromethyl-5β-pregnan-20-one, 21-phosphate disodium salt; or
3α,21-dihydroxy-3β-trifluoromethyl-19-nor-5β-pregnan-20-one.

5. The use as claimed in claim 4, wherein the compound is 3α-hydroxy-3β-trifluoromethyl-19-nor-5β-pregnan-20-one.

## Patentansprüche

1. Verwendung einer 3β-substituierten, 3α-Hydroxy-pregnan-20-on- oder 19-Norpregnan-20-on-Verbindung mit der Formel II: 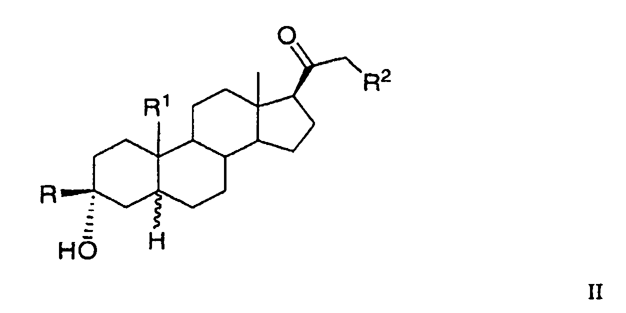 und Natriumsalzen davon;
worin
R für C₁₋₈-Trihalogenalkyl steht
R¹ für Wasserstoff oder eine C₁₋₈-Alkylgruppe steht; und
R² eines von Wasserstoff, Hydroxy, Pyrid-4-ylthio, Hemisuccinoyloxy oder Phosphoryloxy darstellt, in der Herstellung von Medikament für die Behandlung von Schlaflosigkeit, wobei das Medikament oral zu verabreichen ist.

2. Verwendung wie in Anspruch 1 beansprucht, wobei R ausgewählt ist aus der Gruppe bestehend aus Trifluormethyl und 2',2',2'-Trifluorethyl.

3. Verwendung wie in Anspruch 1 beansprucht, wobei R² ausgewählt ist aus der Gruppe bestehend aus Hemisuccinoyloxy, Phosphoryloxy und Natriumsalzen davon.

4. Verwendung wie in Anspruch 1 beansprucht, wobei die Verbindung 3α-Hydroxy-3β-trifluormethyl-5β-pregnan-20-on-21-phosphat-dinatriumsalz; 3α,21-Dihydroxy-3β-trifluormethyl-5β-pregnan-20-on-21-hemisuccinat-natriumsalz; 3α-Hydroxy-3β-trifluormethyl-19-nor-5β-pregnan-20-on; 3α-Hydroxy-3β-trifluormethyl-5β-19-nor-pregnan-20-on-21-phosphatdinatriumsalz; 3α-Hydroxy-3β-trifluormethyl-5β-pregnan-20-on; 3α-Hydroxy-3β-trifluormethyl-19-nor-5α-pregnan-20-on; 3α,21-Dihydroxy-3β-trifluormethyl-5β-pregnan-20-on; 3α,21-Dihydroxy-3β-trifluormethyl-5β-pregnan-20-on, 21-Hemisuccinat; 3α,21-Dihydroxy-3β-trifluormethyl-5β-pregnan-20-on, 21-Phosphatdinatriumsalz; oder 3α,21-Dihydroxy-3β-trifluormethyl-19-nor-5β-pregnan-20-on ist.

5. Verwendung wie in Anspruch 4 beansprucht, wobei die Verbindung 3α-Hydroxy-3β-trifluormethyl-19-nor-5β-pregnan-20-on ist.

## Revendications

1. Utilisation d'un composé 3α-hydroxyprégnane-20-one ou 19-norprégnane-20-one substitué en position 3α de formule II : 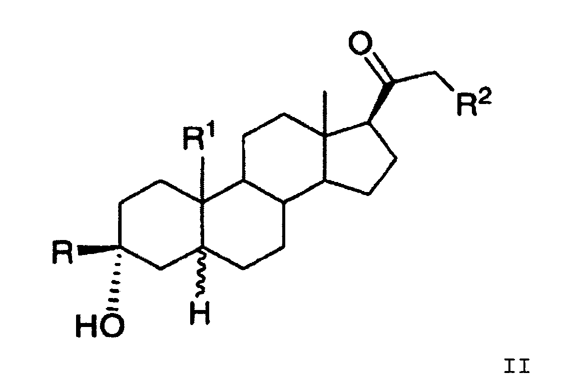 et des sels de sodium de ce dernier ;
dans laquelle :
R est un trihaloalkyle en C_{1 à 8}
R¹ est hydrogène ou un groupe alkyle en C_{1 à 8} ; et
R² est l'un de hydrogène, hydroxy, pyrid-4-ylthio, hémisuccinoyloxy ou phosphoryloxy, dans la fabrication d'un médicament pour le traitement de l'insomnie, dans laquelle le médicament est destiné à être administré par voie orale.

2. Utilisation selon la revendication 1, dans laquelle R est choisi parmi le groupe constitué du trifluorométhyle et du 2',2',2'-trifluoroéthyle.

3. Utilisation selon la revendication 1, dans laquelle R² est choisi parmi le groupe constitué de l'hémisuccinoyloxy, du phosphoryloxy et des sels de sodium de ces derniers.

4. Utilisation selon la revendication 1, dans laquelle le composé est le sel disodique de 3α-hydroxy-3β-trifluorométhyl-5β-prégnane-20-one 21-phosphate ; le sel de sodium de 3α,21-dihydroxy-3β-trifluorométhyl-5β-prégnane-20-one 21-hémisuccinate ; 3α-hydroxy-3β-trifluorométhyl-19-nor-5β-prégnane-20-one ; le sel disodique de 3α-hydroxy-3β-trifluorométhyl-5β-19-nor-prégnane-20-one 21-phosphate ; 3α-hydroxy-3β-trifluorométhyl-5β-prégnane-20-one ;
3α-hydroxy-3β-trifluorométhyl-19-nor-5β-prégnane-20-one ;
3α,21-dihydroxy-3β-trifluorométhyl-5β-prégnane-20-one ;
3α,21-dihydroxy-3β-trifluorométhyl-5β-prégnane-20-one, 21-hémisuccinate ;
le sel disodique de 3α,21-dihydroxy-3β-trifluorométhyl-5β-prégnane-20-one, 21-phosphate ; ou
3α,21-dihydroxy-3β-trifluorométhyl-19-nor-5β-prégnane-20-one.

5. Utilisation selon la revendication 4, dans laquelle le composé est 3α-hydroxy-3β-trifluorométhyl-19-nor-5β-prégnane-20-one.
